(19) Europäisches Patentamt
European Patent Office
Office européen des brevets

(11) **EP 3 619 712 B1**

(12) **EUROPEAN PATENT SPECIFICATION**

(45) Date of publication and mention
of the grant of the patent:
**14.02.2024 Bulletin 2024/07**

(21) Application number: **19742664.6**

(22) Date of filing: **09.07.2019**

(51) International Patent Classification (IPC):
**G16B 40/20** (2019.01)    **G16B 20/20** (2019.01)
**G06N 3/045** (2023.01)    **G06N 3/08** (2023.01)

(52) Cooperative Patent Classification (CPC):
**G16B 20/20; G06N 3/045; G06N 3/08; G16B 40/20;**
G16B 30/00; G16B 50/10

(86) International application number:
**PCT/US2019/041078**

(87) International publication number:
**WO 2020/014280 (16.01.2020 Gazette 2020/03)**

(54) **DEEP LEARNING-BASED FRAMEWORK FOR IDENTIFYING SEQUENCE PATTERNS THAT CAUSE SEQUENCE-SPECIFIC ERRORS**

AUF TIEFENLERNEN BASIERTES RAHMENWERK ZUM IDENTIFIZIEREN VON SEQUENZSPEZIFISCHE FEHLER VERURSACHENDEN SEQUENZMUSTERN

CADRE BASÉ SUR UN APPRENTISSAGE PROFOND POUR IDENTIFIER DES MOTIFS DE SÉQUENCE QUI ENTRAÎNENT DES ERREURS SPÉCIFIQUES À UNE SÉQUENCE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.07.2018 US 201862696699 P
16.08.2018 NL 2021473
08.07.2019 US 201916505100**

(43) Date of publication of application:
**11.03.2020 Bulletin 2020/11**

(73) Proprietor: **Illumina, Inc.
San Diego, CA 92122 (US)**

(72) Inventors:
• **KASHEFHAGHIGHI, Dorna**
**San Diego, california 92122 (US)**
• **KIA, Amirali**
**San Diego, california 92122 (US)**
• **FARH, Kai-How**
**San Diego, california 92122 (US)**

(74) Representative: **Robinson, David Edward
Ashdown
Marks & Clerk LLP
1 New York Street
Manchester M1 4HD (GB)**

(56) References cited:
• **Brendan D O 'fallon ET AL: "A support vector machine for identification of single-nucleotide polymorphisms from next-generation sequencing data", Bioinformatics, 24 April 2013 (2013-04-24), XP055205142, Retrieved from the Internet: URL:http://bioinformatics.oxfordjournals.o rg/content/early/2013/04/24/bioinformatics .btt1 72.full.pdf [retrieved on 2015-07-29]**
• **VIOLA RAVASIO ET AL: "GARFIELD-NGS: Genomic vARiants FIltering by dEep Learning moDels in NGS", BIOINFORMATICS., vol. 34, no. 17, 14 April 2018 (2018-04-14), pages 3038-3040, XP055585241, GB ISSN: 1367-4803, DOI: 10.1093/bioinformatics/bty303**
• **Ryan Poplin ET AL: "Creating a universal SNP and small indel variant caller with deep neural networks", bioRxiv, 20 March 2018 (2018-03-20), XP055585250, DOI: 10.1101/092890 Retrieved from the Internet: URL:https://www.biorxiv.org/content/biorxi v/early/2018/03/20/092890.full.pdf [retrieved on 2019-05-03]**

**Description**

REFERENCES

Strelka™ application by Illumina Inc. hosted at https://github.com/Illumina/strelka and described in the article T Saunders, Christopher & Wong, Wendy & Swamy, Sajani & Becq, Jennifer & J Murray, Lisa & Cheetham, Keira. (2012). Strelka: Accurate somatic small-variant calling from sequenced tumor-normal sample pairs. Bioinformatics (Oxford, England). 28. 1811-7;
Strelka2™ application by Illumina Inc. hosted at https://github.com/Illumina/strelka and described in the article Kim, S., Scheffler, K., Halpern, A.L., Bekritsky, M.A., Noh, E., Källberg, M., Chen, X., Beyter, D., Krusche, P., and Saunders, C.T. (2017);
A. van den Oord, S. Dieleman, H. Zen, K. Simonyan, O. Vinyals, A. Graves, N. Kalchbrenner, A. Senior, and K. Kavukcuoglu, "WAVENET: A GENERATIVE MODEL FOR RAW AUDIO," arXiv:1609.03499, 2016;
S. Ö. Arik, M. Chrzanowski, A. Coates, G. Diamos, A. Gibiansky, Y. Kang, X. Li, J. Miller, A. Ng, J. Raiman, S. Sengupta and M. Shoeybi, "DEEP VOICE: REAL-TIME NEURAL TEXT-TO-SPEECH," arXiv:1702.07825, 2017;
F. Yu and V. Koltun, "MULTI-SCALE CONTEXT AGGREGATION BY DILATED CONVOLUTIONS," arXiv:1511.07122, 2016;
K. He, X. Zhang, S. Ren, and J. Sun, "DEEP RESIDUAL LEARNING FOR IMAGE RECOGNITION," arXiv:1512.03385, 2015;
R.K. Srivastava, K. Greff, and J. Schmidhuber, "HIGHWAY NETWORKS," arXiv: 1505.00387, 2015;
G. Huang, Z. Liu, L. van der Maaten and K. Q. Weinberger, "DENSELY CONNECTED CONVOLUTIONAL NETWORKS," arXiv:1608.06993, 2017;
C. Szegedy, W. Liu, Y. Jia, P. Sermanet, S. Reed, D. Anguelov, D. Erhan, V. Vanhoucke, and A. Rabinovich, "GOING DEEPER WITH CONVOLUTIONS," arXiv: 1409.4842, 2014;
S. Ioffe and C. Szegedy, "BATCH NORMALIZATION: ACCELERATING DEEP NETWORK TRAINING BY REDUCING INTERNAL COVARIATE SHIFT," arXiv: 1502.03167, 2015;
Srivastava, Nitish, Hinton, Geoffrey, Krizhevsky, Alex, Sutskever, Ilya, and Salakhutdinov, Ruslan, "DROPOUT: A SIMPLE WAY TO PREVENT NEURAL NETWORKS FROM OVERFITTING," The Journal of Machine Learning Research, 15 (1):1929-1958, 2014;
J. M. Wolterink, T. Leiner, M. A. Viergever, and I. Isgum, "DILATED CONVOLUTIONAL NEURAL NETWORKS FOR CARDIOVASCULAR MR SEGMENTATION IN CONGENITAL HEART DISEASE," arXiv:1704.03669, 2017;
L. C. Piqueras, "AUTOREGRESSIVE MODEL BASED ON A DEEP CONVOLUTIONAL NEURAL NETWORK FOR AUDIO GENERATION," Tampere University of Technology, 2016;
J. Wu, "Introduction to Convolutional Neural Networks," Nanjing University, 2017;
I. J. Goodfellow, D. Warde-Farley, M. Mirza, A. Courville, and Y. Bengio, "CONVOLUTIONAL NETWORKS", Deep Learning, MIT Press, 2016;
J. Gu, Z. Wang, J. Kuen, L. Ma, A. Shahroudy, B. Shuai, T. Liu, X. Wang, and G. Wang, "RECENT ADVANCES IN CONVOLUTIONAL NEURAL NETWORKS," arXiv:1512.07108, 2017;
M. Lin, Q. Chen, and S. Yan, "Network in Network," in Proc. of ICLR, 2014;
L. Sifre, "Rigid-motion Scattering for Image Classification, Ph.D. thesis, 2014;
L. Sifre and S. Mallat, "Rotation, Scaling and Deformation Invariant Scattering for Texture Discrimination," in Proc. of CVPR, 2013;
F. Chollet, "Xception: Deep Learning with Depthwise Separable Convolutions," in Proc. of CVPR, 2017;
X. Zhang, X. Zhou, M. Lin, and J. Sun, "ShuffleNet: An Extremely Efficient Convolutional Neural Network for Mobile Devices," in arXiv: 1707.01083, 2017;
K. He, X. Zhang, S. Ren, and J. Sun, "Deep Residual Learning for Image Recognition," in Proc. of CVPR, 2016;
S. Xie, R. Girshick, P. Dollár, Z. Tu, and K. He, "Aggregated Residual Transformations for Deep Neural Networks," in Proc. of CVPR, 2017;
A. G. Howard, M. Zhu, B. Chen, D. Kalenichenko, W. Wang, T. Weyand, M. Andreetto, and H. Adam, "Mobilenets: Efficient Convolutional Neural Networks for Mobile Vision Applications," in arXiv:1704.04861, 2017;
M. Sandler, A. Howard, M. Zhu, A. Zhmoginov, and L. Chen, "MobileNetV2: Inverted Residuals and Linear Bottlenecks," in arxiv:1801.04381v3, 2018;
Z. Qin, Z. Zhang, X. Chen, and Y. Peng, "FD-MobileNet: Improved MobileNet with a Fast Downsampling Strategy," in arXiv: 1802.03750, 2018;
PCT International Patent Application No. PCT/US17/61554, titled "Validation Methods and Systems for Sequence Variant Calls", filed on November 14, 2017;

U.S. Provisional Patent Application No. 62/447,076, titled "Validation Methods and Systems for Sequence Variant Calls", filed on January 17, 2017;

U.S. Provisional Patent Application No. 62/422,841, titled "Methods and Systems to Improve Accuracy in Variant Calling", filed on November 16, 2016; and

N. ten DIJKE, "Convolutional Neural Networks for Regulatory Genomics," Master's Thesis, Universiteit Leiden Opleiding Informatica, 17 June 2017.

## FIELD OF THE TECHNOLOGY DISCLOSED

The technology disclosed relates to artificial intelligence type computers and digital data processing systems and corresponding data processing methods and products for emulation of intelligence (i.e., knowledge based systems, reasoning systems, and knowledge acquisition systems); and including systems for reasoning with uncertainty (e.g., fuzzy logic systems), adaptive systems, machine learning systems, and artificial neural networks. In particular, the technology disclosed relates to using deep neural networks such as convolutional neural networks (CNNs) and fully-connected neural networks (FCNNs) for analyzing data.

## BACKGROUND

The subject matter discussed in this section should not be assumed to be prior art merely as a result of its mention in this section. Similarly, a problem mentioned in this section or associated with the subject matter provided as background should not be assumed to have been previously recognized in the prior art. The subject matter in this section merely represents different approaches, which in and of themselves can also correspond to implementations of the claimed technology.

Next-generation sequencing has made large amounts of sequenced data available for variant filtering. Sequenced data are highly correlated and have complex interdependencies, which has hindered the application of traditional classifiers like support vector machine to the variant filtering task. Advanced classifiers that are capable of extracting high-level features from sequenced data are thus desired.

Deep neural networks are a type of artificial neural networks that use multiple nonlinear and complex transforming layers to successively model high-level features. Deep neural networks provide feedback via backpropagation which carries the difference between observed and predicted output to adjust parameters. Deep neural networks have evolved with the availability of large training datasets, the power of parallel and distributed computing, and sophisticated training algorithms. Deep neural networks have facilitated major advances in numerous domains such as computer vision, speech recognition, and natural language processing.

Convolutional neural networks (CNNs) and recurrent neural networks (RNNs) are components of deep neural networks. Convolutional neural networks have succeeded particularly in image recognition with an architecture that comprises convolution layers, nonlinear layers, and pooling layers. Recurrent neural networks are designed to utilize sequential information of input data with cyclic connections among building blocks like perceptrons, long short-term memory units, and gated recurrent units. In addition, many other emergent deep neural networks have been proposed for limited contexts, such as deep spatio-temporal neural networks, multidimensional recurrent neural networks, and convolutional auto-encoders.

The goal of training deep neural networks is optimization of the weight parameters in each layer, which gradually combines simpler features into complex features so that the most suitable hierarchical representations can be learned from data. A single cycle of the optimization process is organized as follows. First, given a training dataset, the forward pass sequentially computes the output in each layer and propagates the function signals forward through the network. In the final output layer, an objective loss function measures error between the inferenced outputs and the given labels. To minimize the training error, the backward pass uses the chain rule to backpropagate error signals and compute gradients with respect to all weights throughout the neural network. Finally, the weight parameters are updated using optimization algorithms based on stochastic gradient descent. Whereas batch gradient descent performs parameter updates for each complete dataset, stochastic gradient descent provides stochastic approximations by performing the updates for each small set of data examples. Several optimization algorithms stem from stochastic gradient descent. For example, the Adagrad and Adam training algorithms perform stochastic gradient descent while adaptively modifying learning rates based on update frequency and moments of the gradients for each parameter, respectively.

Another core element in the training of deep neural networks is regularization, which refers to strategies intended to avoid overfitting and thus achieve good generalization performance. For example, weight decay adds a penalty term to the objective loss function so that weight parameters converge to smaller absolute values. Dropout randomly removes hidden units from neural networks during training and can be considered an ensemble of possible subnetworks. To enhance the capabilities of dropout, a new activation function, maxout, and a variant of dropout for recurrent neural networks called rnnDrop have been proposed. Furthermore, batch normalization provides a new regularization method through normalization of scalar features for each activation within a mini-batch and learning each mean and variance as parameters.

Given that sequenced data are multi- and high-dimensional, deep neural networks have great promise for bioinformatics research because of their broad applicability and enhanced prediction power. Convolutional neural networks have been adapted to solve sequence-based problems in genomics such as motif discovery, pathogenic variant identification, and gene expression inference. A hallmark of convolutional neural networks is the use of convolution filters. Unlike traditional classification approaches that are based on elaborately-designed and manually-crafted features, convolution filters perform adaptive learning of features, analogous to a process of mapping raw input data to the informative representation of knowledge. In this sense, the convolution filters serve as a series of motif scanners, since a set of such filters is capable of recognizing relevant patterns in the input and updating themselves during the training procedure. Recurrent neural networks can capture long-range dependencies in sequential data of varying lengths, such as protein or DNA sequences.

Therefore, an opportunity arises to use a principled deep learning-based framework that associates sequence patterns with sequencing errors. Relevant prior art can be found in Brendan D. O'Fallon et al., "A support vector machine for identification of single-nucleotide polymorphisms from next-generation sequencing data", Bioinformatics, 24 April 2013.

## BRIEF DESCRIPTION OF THE DRAWINGS

In the drawings, like reference characters generally refer to like parts throughout the different views. Also, the drawings are not necessarily to scale, with an emphasis instead generally being placed upon illustrating the principles of the technology disclosed. In the following description, various implementations of the technology disclosed are described with reference to the following drawings, in which:

**FIG. 1** is a block diagram that shows various aspects of DeepPOLY, a deep learning-based framework for identifying sequence patterns that cause sequence-specific errors (SSEs). **FIG. 1** includes modules such as a variant filter, a simulator, and an analyzer. **FIG. 1** also includes databases that store overlaid samples, nucleotide sequences, and repeat patterns.

**FIG. 2** illustrates an example architecture of the variant filter. The variant filter has a hierarchical structure built on a convolutional neural network (CNN) and a fully-connected neural network (FCNN). DeepPOLY uses the variant filter to test known sequence patterns for their effect on variant filtering.

**FIG. 3** shows one implementation of the processing pipeline of the variant filter.

**FIG. 4A** shows true and false positive plots that graphically illustrate the variant filter's performance on held-out data.

**FIGs. 4B** and **4C** show pile-up images of aligned reads that validate the variant filter's accuracy.

**FIG. 5** shows one implementation of one-hot encoding used to encode the overlaid sample that has a called variant at a target position flanked by 20-50 bases on each side.

**FIG. 6** illustrates examples of overlaid samples produced by the input preparer by overlaying the repeat patterns on nucleotide sequences.

**FIG. 7A** uses a box-and-whisker plot to identify sequence-specific errors causation by repeat patterns to left of the variant nucleotide at the target position in the overlaid samples.

**FIG. 7B** uses a box-and-whisker plot to identify sequence-specific errors causation by repeat patterns to right of the variant nucleotide at the target position in the overlaid samples.

**FIG. 7C** uses a box-and-whisker plot to identify sequence-specific errors causation by repeat patterns including a variant nucleotide at the target position in the overlaid samples.

**FIG. 8A** uses a box-and-whisker plot to identify sequence-specific errors causation by repeat patterns of homopolymers of a single base "C" overlaid at varying offsets on nucleotide sequences.

**FIG. 8B** uses a box-and-whisker plot to identify sequence-specific errors causation by repeat patterns of homopolymers of a single base "G" overlaid at varying offsets on nucleotide sequences.

**FIG. 8C** uses a box-and-whisker plot to identify sequence-specific errors causation by repeat patterns of homopolymers of a single base "A" overlaid at varying offsets on nucleotide sequences.

**FIG. 8D** uses a box-and-whisker plot to identify sequence-specific errors causation by repeat patterns of homopolymers of a single base "T" overlaid at varying offsets on nucleotide sequences.

**FIG. 9** displays classification scores as a distribution for likelihood that a variant nucleotide is a true variant or a false variant when repeat patterns of homopolymers of a single base are placed one by one "before" and "after" a variant nucleotide of each of the four bases at a target position.

**FIGs. 10A** to **10C** display a representation of naturally occurring repeat patterns of copolymers in each of the sample nucleotide sequences that contribute to a false variant classification.

**FIG. 11** is a simplified block diagram of a computer system that can be used to implement the variant filter.

**FIG. 12** illustrates one implementation of how sequence-specific errors (SSEs) are correlated to repeat patterns based on false variant classifications.

## DETAILED DESCRIPTION

The following discussion is presented to enable any person skilled in the art to make and use the technology disclosed, and is provided in the context of a particular application and its requirements.

## Introduction

Sequence-specific errors (SSEs) are base calling errors caused by specific sequence patterns. For example, the sequence patterns 'GGC' and 'GGCNG' and their inverted repeats have been found to cause large amounts of miscalls. SSEs lead to assembly gaps and mapping artifacts. Also, since any miscall can be mistaken for a variant, SSEs result in false variant calls and are a major obstacle to accurate variant calling.

We disclose a deep learning-based framework, DeepPOLY, which identifies sequence patterns that cause SSEs. DeepPOLY trains a variant filter on large-scale variant data to learn causal dependencies between sequence patterns and false variant calls. The variant filter has a hierarchical structure built on deep neural networks that evaluate an input sequence at multiple spatial scales and perform variant filtering, i.e., predict whether a called variant in the input sequence is a true variant call or a false variant call. The large-scale variant data includes pedigree variants, of which inherited variants are used as training examples of true variant calls and de novo variants observed in only one child are used as training examples of false variant calls. In some implementations, at least some of the de novo variants observed in only one child are used as training examples of true variant calls.

During training, parameters of the deep neural networks are optimized to maximize filtering accuracy using a gradient descent approach. The resulting variant filter learns to associate false variant calls with sequence patterns in the input sequences.

DeepPOLY then implements a simulation that uses the variant filter to test known sequence patterns for their effect on variant filtering. The known sequence patterns are repeat patterns (or copolymers) that differ in base composition, pattern length, and repeat factor. The repeat patterns are tested at varying offsets from the called variants.

The premise of the simulation is as follows: when a pair of a repeat pattern under test and a called variant is fed to the variant filter as part of a simulated input sequence and the variant filter classifies the called variant as a false variant call, then the repeat pattern is considered to have caused the false variant call and identified as SSE-causing. Under this premise, DeepPOLY tests hundreds and thousands of repeat patterns to identify which ones are SSE-causing, with offset sensitivity.

DeepPOLY also discovers naturally occurring sequence patterns that cause SSEs by processing naturally occurring input sequences through the variant filter and analyzing parameter activations of the deep neural networks during the processing. Those sequence patterns are identified as SSE-causing for which the input neurons of the deep neural networks produce the highest parameter activations and the output neurons produce a false variant call classification.

DeepPOLY confirms previously known SSE-causing sequence patterns and reports new more specific ones.

DeepPOLY is agnostic of the underlying sequencing chemistry, sequencing platform, and sequencing polymerases and can produce comprehensive profiles of SSE-causing sequence patterns for different sequencing chemistries, sequencing platforms, and sequencing polymerases. These profiles can be used to improve the sequencing chemistries, build higher quality sequencing platforms, and create different sequencing polymerases. They can also be used to recalculate base call quality scores and to improve variant calling accuracy.

The variant filter has two deep neural networks: a convolutional neural network (CNN) followed by a fully-connected neural network (FCNN). A repeat pattern under test is overlaid on a nucleotide sequence to produce an overlaid sample. The overlaid sample has a called variant at a target position flanked by 20-50 bases on each side. We regard the overlaid sample as an image with multiple channels that numerically encode the four types of bases, A, C, G, and T. The overlaid sample, spanning the called variant, is one-hot encoded to conserve the position-specific information of each individual base in the overlaid sample.

The convolutional neural network receives the one-hot overlaid sample because it is capable of preserving the spatial locality relationships within the overlaid sample. The convolutional neural network processes the overlaid sample through multiple convolution layers and produces one or more intermediate convolved features. The convolution layers utilize convolution filters to detect sequence patterns within the overlaid sample. The convolution filters act as motif detectors that scan the overlaid sample for low-level motifs and produce signals of different strengths depending on the underlying sequence patterns. The convolution filters are automatically learned after training on hundreds and thousands of training examples of true and false variant calls.

The fully-connected neural network then processes the intermediate convolved features through multiple fully-connected layers. The densely connected neurons of the fully-connected layers detect high-level sequence patterns encoded in the convolved features. Finally, a classification layer of the fully-connected neural network outputs probabilities for the called variant being a true variant call or a false variant call.

In addition to using dropout, pairs of batch normalization and rectified linear unit nonlinearity are interspersed between the convolutional layers and the fully-connected layers to enhance learning rates and reduce overfitting.

## Terminology

As used herein, the following terms have the meanings indicated.

A base refers to a nucleotide base or nucleotide, A (adenine), C (cytosine), T (thymine), or G (guanine).

The term "chromosome" refers to the heredity-bearing gene carrier of a living cell, which is derived from chromatin strands comprising DNA and protein components (especially histones). The conventional internationally recognized individual human genome chromosome numbering system is employed herein.

The term "site" refers to a unique position (e.g., chromosome ID, chromosome position and orientation) on a reference genome. In some implementations, a site may be a residue, a sequence tag, or a segment's position on a sequence. The term "locus" may be used to refer to the specific location of a nucleic acid sequence or polymorphism on a reference chromosome.

The term "sample" herein refers to a sample, typically derived from a biological fluid, cell, tissue, organ, or organism containing a nucleic acid or a mixture of nucleic acids containing at least one nucleic acid sequence that is to be sequenced and/or phased. Such samples include, but are not limited to sputum/oral fluid, amniotic fluid, blood, a blood fraction, fine needle biopsy samples (e.g., surgical biopsy, fine needle biopsy, etc.), urine, peritoneal fluid, pleural fluid, tissue explant, organ culture and any other tissue or cell preparation, or fraction or derivative thereof or isolated therefrom. Although the sample is often taken from a human subject (e.g., patient), samples can be taken from any organism having chromosomes, including, but not limited to dogs, cats, horses, goats, sheep, cattle, pigs, etc. The sample may be used directly as obtained from the biological source or following a pretreatment to modify the character of the sample. For example, such pretreatment may include preparing plasma from blood, diluting viscous fluids and so forth. Methods of pretreatment may also involve, but are not limited to, filtration, precipitation, dilution, distillation, mixing, centrifugation, freezing, lyophilization, concentration, amplification, nucleic acid fragmentation, inactivation of interfering components, the addition of reagents, lysing, etc.

The term "sequence" includes or represents a strand of nucleotides coupled to each other. The nucleotides may be based on DNA or RNA. It should be understood that one sequence may include multiple sub-sequences. For example, a single sequence (e.g., of a PCR amplicon) may have 350 nucleotides. The sample read may include multiple sub-sequences within these 350 nucleotides. For instance, the sample read may include first and second flanking subsequences having, for example, 20-50 nucleotides. The first and second flanking sub-sequences may be located on either side of a repetitive segment having a corresponding sub-sequence (e.g., 40-100 nucleotides). Each of the flanking sub-sequences may include (or include portions of) a primer sub-sequence (e.g., 10-30 nucleotides). For ease of reading, the term "sub-sequence" will be referred to as "sequence," but it is understood that two sequences are not necessarily separate from each other on a common strand. To differentiate the various sequences described herein, the sequences may be given different labels (e.g., target sequence, primer sequence, flanking sequence, reference sequence, and the like). Other terms, such as "allele," may be given different labels to differentiate between like objects.

The term "paired-end sequencing" refers to sequencing methods that sequence both ends of a target fragment. Paired-end sequencing may facilitate detection of genomic rearrangements and repetitive segments, as well as gene fusions and novel transcripts. Methodology for paired-end sequencing are described in PCT publication WO07010252, PCT application Serial No. PCTGB2007/003798 and US patent application publication US 2009/0088327. In one example, a series of operations may be performed as follows; (a) generate clusters of nucleic acids; (b) linearize the nucleic acids; (c) hybridize a first sequencing primer and carry out repeated cycles of extension, scanning and deblocking, as set forth above; (d) "invert" the target nucleic acids on the flow cell surface by synthesizing a complimentary copy; (e) linearize the resynthesized strand; and (f) hybridize a second sequencing primer and carry out repeated cycles of extension, scanning and deblocking, as set forth above. The inversion operation can be carried out be delivering reagents as set forth above for a single cycle of bridge amplification.

The term "reference genome" or "reference sequence" refers to any particular known genome sequence, whether partial or complete, of any organism which may be used to reference identified sequences from a subject. For example, a reference genome used for human subjects as well as many other organisms is found at the National Center for Biotechnology Information at ncbi.nlm.nih.gov. A "genome" refers to the complete genetic information of an organism or virus, expressed in nucleic acid sequences. A genome includes both the genes and the noncoding sequences of the DNA. The reference sequence may be larger than the reads that are aligned to it. For example, it may be at least about 100 times larger, or at least about 1000 times larger, or at least about 10,000 times larger, or at least about 105 times larger, or at least about 106 times larger, or at least about 107 times larger. In one example, the reference genome sequence is that of a full length human genome. In another example, the reference genome sequence is limited to a specific human chromosome such as chromosome 13. In some implementations, a reference chromosome is a chromosome sequence from human genome version hg19. Such sequences may be referred to as chromosome reference sequences, although the term reference genome is intended to cover such sequences. Other examples of reference sequences include genomes of other species, as well as chromosomes, sub-chromosomal regions (such as strands), etc., of any species. In various implementations, the reference genome is a consensus sequence or other combination derived from multiple individuals. However, in certain applications, the reference sequence may be taken from a particular individual.

The term "read" refer to a collection of sequence data that describes a fragment of a nucleotide sample or reference. The term "read" may refer to a sample read and/or a reference read. Typically, though not necessarily, a read represents a short sequence of contiguous base pairs in the sample or reference. The read may be represented symbolically by the base pair sequence (in ATCG) of the sample or reference fragment. It may be stored in a memory device and processed as appropriate to determine whether the read matches a reference sequence or meets other criteria. A read may be obtained directly from a sequencing apparatus or indirectly from stored sequence information concerning the sample. In some cases, a read is a DNA sequence of sufficient length (e.g., at least about 25 bp) that can be used to identify a larger sequence or region, e.g., that can be aligned and specifically assigned to a chromosome or genomic region or gene.

Next-generation sequencing methods include, for example, sequencing by synthesis technology (Illumina), pyrosequencing (454), ion semiconductor technology (Ion Torrent sequencing), single-molecule real-time sequencing (Pacific Biosciences) and sequencing by ligation (SOLiD sequencing). Depending on the sequencing methods, the length of each read may vary from about 30 bp to more than 10,000 bp. For example, Illumina sequencing method using SOLiD sequencer generates nucleic acid reads of about 50 bp. For another example, Ion Torrent Sequencing generates nucleic acid reads of up to 400 bp and 454 pyrosequencing generates nucleic acid reads of about 700 bp. For yet another example, single-molecule real-time sequencing methods may generate reads of 10,000 bp to 15,000 bp. Therefore, in certain implementations, the nucleic acid sequence reads have a length of 30-100 bp, 50-200 bp, or 50-400 bp.

The terms "sample read", "sample sequence" or "sample fragment" refer to sequence data for a genomic sequence of interest from a sample. For example, the sample read comprises sequence data from a PCR amplicon having a forward and reverse primer sequence. The sequence data can be obtained from any select sequence methodology. The sample read can be, for example, from a sequencing-by-synthesis (SBS) reaction, a sequencing-by-ligation reaction, or any other suitable sequencing methodology for which it is desired to determine the length and/or identity of a repetitive element. The sample read can be a consensus (e.g., averaged or weighted) sequence derived from multiple sample reads. In certain implementations, providing a reference sequence comprises identifying a locus-of-interest based upon the primer sequence of the PCR amplicon.

The term "raw fragment" refers to sequence data for a portion of a genomic sequence of interest that at least partially overlaps a designated position or secondary position of interest within a sample read or sample fragment. Non-limiting examples of raw fragments include a duplex stitched fragment, a simplex stitched fragment, a duplex un-stitched fragment and a simplex un-stitched fragment. The term "raw" is used to indicate that the raw fragment includes sequence data having some relation to the sequence data in a sample read, regardless of whether the raw fragment exhibits a supporting variant that corresponds to and authenticates or confirms a potential variant in a sample read. The term "raw fragment" does not indicate that the fragment necessarily includes a supporting variant that validates a variant call in a sample read. For example, when a sample read is determined by a variant call application to exhibit a first variant, the variant call application may determine that one or more raw fragments lack a corresponding type of "supporting" variant that may otherwise be expected to occur given the variant in the sample read.

The terms "mapping", "aligned," "alignment," or "aligning" refer to the process of comparing a read or tag to a reference sequence and thereby determining whether the reference sequence contains the read sequence. If the reference sequence contains the read, the read may be mapped to the reference sequence or, in certain implementations, to a particular location in the reference sequence. In some cases, alignment simply tells whether or not a read is a member of a particular reference sequence (i.e., whether the read is present or absent in the reference sequence). For example, the alignment of a read to the reference sequence for human chromosome 13 will tell whether the read is present in the reference sequence for chromosome 13. A tool that provides this information may be called a set membership tester. In some cases, an alignment additionally indicates a location in the reference sequence where the read or tag maps to. For example, if the reference sequence is the whole human genome sequence, an alignment may indicate that a read is present on chromosome 13, and may further indicate that the read is on a particular strand and/or site of chromosome 13.

The term "indel" refers to the insertion and/or the deletion of bases in the DNA of an organism. A micro-indel represents an indel that results in a net change of 1 to 50 nucleotides. In coding regions of the genome, unless the length of an indel is a multiple of 3, it will produce a frameshift mutation. Indels can be contrasted with point mutations. An indel inserts and deletes nucleotides from a sequence, while a point mutation is a form of substitution that replaces one of the nucleotides without changing the overall number in the DNA. Indels can also be contrasted with a Tandem Base Mutation (TBM), which may be defined as substitution at adjacent nucleotides (primarily substitutions at two adjacent nucleotides, but substitutions at three adjacent nucleotides have been observed.

The term "variant" refers to a nucleic acid sequence that is different from a nucleic acid reference. Typical nucleic acid sequence variant includes without limitation single nucleotide polymorphism (SNP), short deletion and insertion polymorphisms (Indel), copy number variation (CNV), microsatellite markers or short tandem repeats and structural variation. Somatic variant calling is the effort to identify variants present at low frequency in the DNA sample. Somatic variant calling is of interest in the context of cancer treatment. Cancer is caused by an accumulation of mutations in DNA. A DNA sample from a tumor is generally heterogeneous, including some normal cells, some cells at an early stage of cancer progression (with fewer mutations), and some late-stage cells (with more mutations). Because of this heterogeneity, when sequencing a tumor (e.g., from an FFPE sample), somatic mutations will often appear at a low frequency. For example, a SNV might be seen in only 10% of the reads covering a given base. A variant that is to be classified as somatic or germline by the variant classifier is also referred to herein as the "variant under test".

The term "noise" refers to a mistaken variant call resulting from one or more errors in the sequencing process and/or in the variant call application.

The term "variant frequency" represents the relative frequency of an allele (variant of a gene) at a particular locus in a population, expressed as a fraction or percentage. For example, the fraction or percentage may be the fraction of all chromosomes in the population that carry that allele. By way of example, sample variant frequency represents the relative frequency of an allele/variant at a particular locus/position along a genomic sequence of interest over a "population" corresponding to the number of reads and/or samples obtained for the genomic sequence of interest from an individual. As another example, a baseline variant frequency represents the relative frequency of an allele/variant at a particular locus/position along one or more baseline genomic sequences where the "population" corresponding to the number of reads and/or samples obtained for the one or more baseline genomic sequences from a population of normal individuals.

The term "variant allele frequency (VAF)" refers to the percentage of sequenced reads observed matching the variant divided by the overall coverage at the target position. VAF is a measure of the proportion of sequenced reads carrying the variant.

The terms "position", "designated position", and "locus" refer to a location or coordinate of one or more nucleotides within a sequence of nucleotides. The terms "position", "designated position", and "locus" also refer to a location or coordinate of one or more base pairs in a sequence of nucleotides.

The term "haplotype" refers to a combination of alleles at adjacent sites on a chromosome that are inherited together. A haplotype may be one locus, several loci, or an entire chromosome depending on the number of recombination events that have occurred between a given set of loci, if any occurred.

The term "threshold" herein refers to a numeric or non-numeric value that is used as a cutoff to characterize a sample, a nucleic acid, or portion thereof (e.g., a read). A threshold may be varied based upon empirical analysis. The threshold may be compared to a measured or calculated value to determine whether the source giving rise to such value suggests should be classified in a particular manner. Threshold values can be identified empirically or analytically. The choice of a threshold is dependent on the level of confidence that the user wishes to have to make the classification. The threshold may be chosen for a particular purpose (e.g., to balance sensitivity and selectivity). As used herein, the term "threshold" indicates a point at which a course of analysis may be changed and/or a point at which an action may be triggered. A threshold is not required to be a predetermined number. Instead, the threshold may be, for instance, a function that is based on a plurality of factors. The threshold may be adaptive to the circumstances. Moreover, a threshold may indicate an upper limit, a lower limit, or a range between limits.

In some implementations, a metric or score that is based on sequencing data may be compared to the threshold. As used herein, the terms "metric" or "score" may include values or results that were determined from the sequencing data or may include functions that are based on the values or results that were determined from the sequencing data. Like a threshold, the metric or score may be adaptive to the circumstances. For instance, the metric or score may be a normalized value. As an example of a score or metric, one or more implementations may use count scores when analyzing the data. A count score may be based on number of sample reads. The sample reads may have undergone one or more filtering stages such that the sample reads have at least one common characteristic or quality. For example, each of the sample reads that are used to determine a count score may have been aligned with a reference sequence or may be assigned as a potential allele. The number of sample reads having a common characteristic may be counted to determine a read count. Count scores may be based on the read count. In some implementations, the count score may be a value that is equal to the read count. In other implementations, the count score may be based on the read count and other information. For example, a count score may be based on the read count for a particular allele of a genetic locus and a total number of reads for the genetic locus. In some implementations, the count score may be based on the read count and previously-obtained data for the genetic locus. In some implementations, the count scores may be normalized scores between predetermined values. The count score may also be a function of read counts from other loci of a sample or a function of read counts from other samples that were concurrently run with the sample-of-interest. For instance, the count score may be a function of the read count of a particular allele and the read counts of other loci in the sample and/or the read counts from other samples. As one example, the read counts from other loci and/or the read counts from other samples may be used to normalize the count score for the particular allele.

The terms "coverage" or "fragment coverage" refer to a count or other measure of a number of sample reads for the same fragment of a sequence. A read count may represent a count of the number of reads that cover a corresponding fragment. Alternatively, the coverage may be determined by multiplying the read count by a designated factor that is based on historical knowledge, knowledge of the sample, knowledge of the locus, etc.

The term "read depth" (conventionally a number followed by "$\times$") refers to the number of sequenced reads with overlapping alignment at the target position. This is often expressed as an average or percentage exceeding a cutoff over a set of intervals (such as exons, genes, or panels). For example, a clinical report might say that a panel average coverage is 1,105$\times$ with 98% of targeted bases covered >100x.

The terms "base call quality score" or "Q score" refer to a PHRED-scaled probability ranging from 0-20 inversely proportional to the probability that a single sequenced base is correct. For example, a T base call with Q of 20 is considered likely correct with a confidence P-value of 0.01. Any base call with Q<20 should be considered low quality, and any variant identified where a substantial proportion of sequenced reads supporting the variant are of low quality should be considered potentially false positive.

The terms "variant reads" or "variant read number" refer to the number of sequenced reads supporting the presence of the variant.

## DeepPOLY

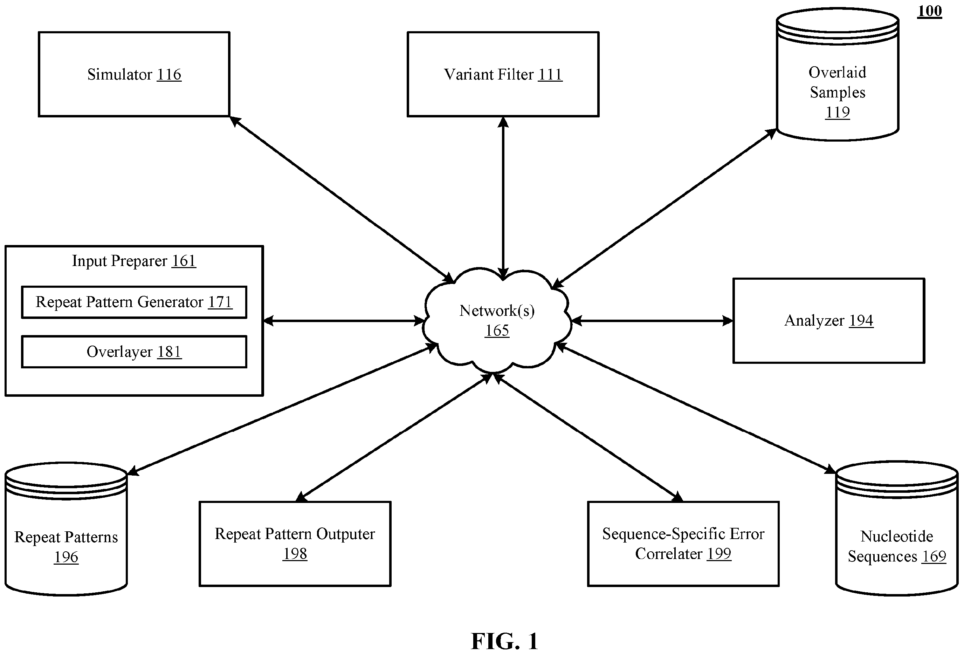

We describe DeepPOLY, a deep learning-based framework for identifying sequence patterns that cause sequence-specific errors (SSEs). The system and processes are described with reference to **FIG. 1.** Because **FIG. 1** is an architectural diagram, certain details are intentionally omitted to improve the clarity of the description. The discussion of **FIG. 1** is organized as follows. First, the modules of the figure are introduced, followed by their interconnections. Then, the use of the modules is described in greater detail.

**FIG. 1** includes the system **100.** The system **100** includes a variant filter **111** (also referred to herein as a variant filter subsystem), an input preparer **161** (also referred to herein as an input preparation subsystem), a simulator **116** (also referred to herein as a simulation subsystem), an analyzer **194** (also referred to herein as an analysis subsystem), a repeat patterns database **196,** a nucleotide sequences database **169,** an overlaid samples database **119,** and a repeat pattern outputer **198** (also referred to herein as a repeat pattern output subsystem).

The processing engines and databases of **FIG. 1,** designated as modules, can be implemented in hardware or software, and need not be divided up in precisely the same blocks as shown in **FIG. 1.** Some of the modules can also be implemented on different processors, computers, or servers, or spread among a number of different processors, computers, or servers. In addition, it will be appreciated that some of the modules can be combined, operated in parallel or in a different sequence than that shown in **FIG. 1** without affecting the functions achieved. The modules in **FIG. 1** can also be thought of as flowchart steps in a method. A module also need not necessarily have all its code disposed contiguously in memory; some parts of the code can be separated from other parts of the code with code from other modules or other functions disposed in between.

The interconnections of the modules of environment **100** are now described. The network(s) **114** couples the processing engines and the databases, all in communication with each other (indicated by solid double-arrowed lines). The actual communication path can be point-to-point over public and/or private networks. The communications can occur over a variety of networks, e.g., private networks, VPN, MPLS circuit, or Internet, and can use appropriate application programming interfaces (APIs) and data interchange formats, e.g., Representational State Transfer (REST), JavaScript Object Notation (JSON), Extensible Markup Language (XML), Simple Object Access Protocol (SOAP), Java Message Service (JMS), and/or Java Platform Module System. All of the communications can be encrypted. The communication is generally over a network such as the LAN (local area network), WAN (wide area network), telephone network (Public Switched Telephone Network (PSTN), Session Initiation Protocol (SIP), wireless network, point-to-point network, star network, token ring network, hub network, Internet, inclusive of the mobile Internet, via protocols such as EDGE, 3G, 4G LTE, Wi-Fi, and WiMAX. Additionally, a variety of authorization and authentication techniques, such as username/password, Open Authorization (OAuth), Kerberos, SecureID, digital certificates and more, can be used to secure the communications.

## Sequencing Process

Implementations set forth herein may be applicable to analyzing nucleic acid sequences to identify sequence variations. Implementations may be used to analyze potential variants/alleles of a genetic position/locus and determine a genotype of the genetic locus or, in other words, provide a genotype call for the locus. By way of example, nucleic acid sequences may be analyzed in accordance with the methods and systems described in US Patent Application Publication No. 2016/0085910 and US Patent Application Publication No. 2013/02961 75.

In one implementation, a sequencing process includes receiving a sample that includes or is suspected of including nucleic acids, such as DNA. The sample may be from a known or unknown source, such as an animal (e.g., human), plant, bacteria, or fungus. The sample may be taken directly from the source. For instance, blood or saliva may be taken directly from an individual. Alternatively, the sample may not be obtained directly from the source. Then, one or more processors direct the system to prepare the sample for sequencing. The preparation may include removing extraneous material and/or isolating certain material (e.g., DNA). The biological sample may be prepared to include features for a particular assay. For example, the biological sample may be prepared for sequencing-by-synthesis (SBS). In certain implementations, the preparing may include amplification of certain regions of a genome. For instance, the preparing may include amplifying predetermined genetic loci that are known to include STRs and/or SNPs. The genetic loci may be amplified using predetermined primer sequences.

Next, the one or more processors direct the system to sequence the sample. The sequencing may be performed through a variety of known sequencing protocols. In particular implementations, the sequencing includes SBS. In SBS, a plurality of fluorescently-labeled nucleotides are used to sequence a plurality of clusters of amplified DNA (possibly millions of clusters) present on the surface of an optical substrate (e.g., a surface that at least partially defines a channel in a flow cell). The flow cells may contain nucleic acid samples for sequencing where the flow cells are placed within the appropriate flow cell holders.

The nucleic acids can be prepared such that they comprise a known primer sequence that is adjacent to an unknown target sequence. To initiate the first SBS sequencing cycle, one or more differently labeled nucleotides, and DNA polymerase, etc., can be flowed into/through the flow cell by a fluid flow subsystem. Either a single type of nucleotide can be added at a time, or the nucleotides used in the sequencing procedure can be specially designed to possess a reversible termination property, thus allowing each cycle of the sequencing reaction to occur simultaneously in the presence of several types of labeled nucleotides (e.g., A, C, T, G). The nucleotides can include detectable label moieties such as fluorophores. Where the four nucleotides are mixed together, the polymerase is able to select the correct base to incorporate and each sequence is extended by a single base. Non-incorporated nucleotides can be washed away by flowing a wash solution through the flow cell. One or more lasers may excite the nucleic acids and induce fluorescence. The fluorescence emitted from the nucleic acids is based upon the fluorophores of the incorporated base, and different fluorophores may emit different wavelengths of emission light. A deblocking reagent can be added to the flow cell to remove reversible terminator groups from the DNA strands that were extended and detected. The deblocking reagent can then be washed away by flowing a wash solution through the flow cell. The flow cell is then ready for a further cycle of sequencing starting with introduction of a labeled nucleotide as set forth above. The fluidic and detection operations can be repeated several times to complete a sequencing run. Example sequencing methods are described, for example, in Bentley et al., Nature 456:53-59 (2008), International Publication No. WO 04/018497; U.S. Pat. No. 7,057,026; International Publication No. WO 91/06678; International Publication No. WO 07/123744; U.S. Pat. No. 7,329,492; U.S. Patent No. 7,211,414; U.S. Patent No. 7,315,019; U.S. Patent No. 7,405,281, and U.S. Patent Application Publication No. 2008/01 08082.

In some implementations, nucleic acids can be attached to a surface and amplified prior to or during sequencing. For example, amplification can be carried out using bridge amplification to form nucleic acid clusters on a surface. Useful bridge amplification methods are described, for example, in U.S. Patent No. 5,641,658; U.S. Patent Application Publication No. 2002/0055100; U.S. Patent No. 7,115,400; U.S. Patent Application Publication No. 2004/0096853; U.S. Patent Application Publication No. 2004/0002090; U.S. Patent Application Publication No. 2007/0128624; and U.S. Patent Application Publication No. 2008/0009420. Another useful method for amplifying nucleic acids on a surface is rolling circle amplification (RCA), for example, as described in Lizardi et al., Nat. Genet. 19:225-232 (1998) and U.S. Patent Application Publication No. 2007/0099208 A1.

One example SBS protocol exploits modified nucleotides having removable 3' blocks, for example, as described in International Publication No. WO 04/018497, U.S. Patent Application Publication No. 2007/0166705A1, and U.S. Patent No. 7,057,026. For example, repeated cycles of SBS reagents can be delivered to a flow cell having target nucleic acids attached thereto, for example, as a result of the bridge amplification protocol. The nucleic acid clusters can be converted to single stranded form using a linearization solution. The linearization solution can contain, for example, a restriction endonuclease capable of cleaving one strand of each cluster. Other methods of cleavage can be used as an alternative to restriction enzymes or nicking enzymes, including inter alia chemical cleavage (e.g., cleavage of a diol linkage with periodate), cleavage of abasic sites by cleavage with endonuclease (for example 'USER', as supplied by NEB, Ipswich, Mass., USA, part number M5505S), by exposure to heat or alkali, cleavage of ribonucleotides incorporated into amplification products otherwise comprised of deoxyribonucleotides, photochemical cleavage or cleavage of a peptide linker. After the linearization operation a sequencing primer can be delivered to the flow cell under conditions for hybridization of the sequencing primer to the target nucleic acids that are to be sequenced.

A flow cell can then be contacted with an SBS extension reagent having modified nucleotides with removable 3' blocks and fluorescent labels under conditions to extend a primer hybridized to each target nucleic acid by a single nucleotide addition. Only a single nucleotide is added to each primer because once the modified nucleotide has been incorporated into the growing polynucleotide chain complementary to the region of the template being sequenced there is no free 3'-OH group available to direct further sequence extension and therefore the polymerase cannot add further nucleotides. The SBS extension reagent can be removed and replaced with scan reagent containing components that protect the sample under excitation with radiation. Example components for scan reagent are described in U.S. Patent Application Publication No. 2008/0280773 A1 and U.S. Patent Application No. 13/018,255. The extended nucleic acids can then be fluorescently detected in the presence of scan reagent. Once the fluorescence has been detected, the 3' block may be removed using a deblock reagent that is appropriate to the blocking group used. Example deblock reagents that are useful for respective blocking groups are described in WO004018497, US 2007/0166705A1 and U.S. Patent No. 7,057,026. The deblock reagent can be washed away leaving target nucleic acids hybridized to extended primers having 3'-OH groups that are now competent for addition of a further nucleotide. Accordingly the cycles of adding extension reagent, scan reagent, and deblock reagent, with optional washes between one or more of the operations, can be repeated until a desired sequence is obtained. The above cycles can be carried out using a single extension reagent delivery operation per cycle when each of the modified nucleotides has a different label attached thereto, known to correspond to the particular base. The different labels facilitate discrimination between the nucleotides added during each incorporation operation. Alternatively, each cycle can include separate operations of extension reagent delivery followed by separate operations of scan reagent delivery and detection, in which case two or more of the nucleotides can have the same label and can be distinguished based on the known order of delivery.

Although the sequencing operation has been discussed above with respect to a particular SBS protocol, it will be understood that other protocols for sequencing any of a variety of other molecular analyses can be carried out as desired.

Then, the one or more processors of the system receive the sequencing data for subsequent analysis. The sequencing data may be formatted in various manners, such as in a .BAM file. The sequencing data may include, for example, a number of sample reads. The sequencing data may include a plurality of sample reads that have corresponding sample sequences of the nucleotides. Although only one sample read is discussed, it should be understood that the sequencing data may include, for example, hundreds, thousands, hundreds of thousands, or millions of sample reads. Different sample reads may have different numbers of nucleotides. For example, a sample read may range between 10 nucleotides to about 500 nucleotides or more. The sample reads may span the entire genome of the source(s). As one example, the sample reads are directed toward predetermined genetic loci, such as those genetic loci having suspected STRs or suspected SNPs.

Each sample read may include a sequence of nucleotides, which may be referred to as a sample sequence, sample fragment or a target sequence. The sample sequence may include, for example, primer sequences, flanking sequences, and a target sequence. The number of nucleotides within the sample sequence may include 30, 40, 50, 60, 70, 80, 90, 100 or more. In some implementations, one or more the sample reads (or sample sequences) includes at least 150 nucleotides, 200 nucleotides, 300 nucleotides, 400 nucleotides, 500 nucleotides, or more. In some implementations, the sample reads may include more than 1000 nucleotides, 2000 nucleotides, or more. The sample reads (or the sample sequences) may include primer sequences at one or both ends.

Next, the one or more processors analyze the sequencing data to obtain potential variant call(s) and a sample variant frequency of the sample variant call(s). The operation may also be referred to as a variant call application or variant caller. Thus, the variant caller identifies or detects variants and the variant classifier classifies the detected variants as somatic or germline. Alternative variant callers may be utilized in accordance with implementations herein, wherein different variant callers may be used based on the type of sequencing operation being performed, based on features of the sample that are of interest and the like. One non-limiting example of a variant call application, such as the Pisces™ application by Illumina Inc. (San Diego, CA) hosted at https://github.com/Illumina/Pisces and described in the article Dunn, Tamsen & Berry, Gwenn & Emig-Agius, Dorothea & Jiang, Yu & Iyer, Anita & Udar, Nitin & Strömberg, Michael. (2017). Pisces: An Accurate and Versatile Single Sample Somatic and Germline Variant Caller. 595-595. 10.1145/3107411.3108203.

Such a variant call application can comprise four sequentially executed modules:

(1) Pisces Read Stitcher: Reduces noise by stitching paired reads in a BAM (read one and read two of the same molecule) into consensus reads. The output is a stitched BAM.
(2) Pisces Variant Caller: Calls small SNVs, insertions and deletions. Pisces includes a variant-collapsing algorithm to coalesce variants broken up by read boundaries, basic filtering algorithms, and a simple Poisson-based variant confidence-scoring algorithm. The output is a VCF.
(3) Pisces Variant Quality Recalibrator (VQR): In the event that the variant calls overwhelmingly follow a pattern associated with thermal damage or FFPE deamination, the VQR step will downgrade the variant Q score of the suspect variant calls. The output is an adjusted VCF.

(4) Pisces Variant Phaser (Scylla): Uses a read-backed greedy clustering method to assemble small variants into complex alleles from clonal subpopulations. This allows for the more accurate determination of functional consequence by downstream tools. The output is an adjusted VCF.

Additionally or alternatively, the operation may utilize the variant call application Strelka™ application by Illumina Inc. hosted at https://github.com/Illumina/strelka and described in the article T Saunders, Christopher & Wong, Wendy & Swamy, Sajani & Becq, Jennifer & J Murray, Lisa & Cheetham, Keira. (2012). Strelka: Accurate somatic small-variant calling from sequenced tumor-normal sample pairs. Bioinformatics (Oxford, England). 28. 1811-7. 10.1093/bioinformatics/bts271. Furthermore, additionally or alternatively, the operation may utilize the variant call application Strelka2™ application by Illumina Inc. hosted at https://github.com/Illumina/strelka and described in the article Kim, S., Scheffler, K., Halpern, A.L., Bekritsky, M.A., Noh, E., Källberg, M., Chen, X., Beyter, D., Krusche, P., and Saunders, C.T. (2017). Strelka2: Fast and accurate variant calling for clinical sequencing applications. Moreover, additionally or alternatively, the operation may utilize a variant annotation/call tool, such as the Nirvana™ application by Illumina Inc. hosted at https://github.com/Illumina/Nirvana/wiki and described in the article Stromberg, Michael & Roy, Rajat & Lajugie, Julien & Jiang, Yu & Li, Haochen & Margulies, Elliott. (2017). Nirvana: Clinical Grade Variant Annotator. 596-596. 10.1145/3107411.31 08204.

Such a variant annotation/call tool can apply different algorithmic techniques such as those disclosed in Nirvana:

a. Identifying all overlapping transcripts with Interval Array: For functional annotation, we can identify all transcripts overlapping a variant and an interval tree can be used. However, since a set of intervals can be static, we were able to further optimize it to an Interval Array. An interval tree returns all overlapping transcripts in O(min(n,k lg n)) time, where n is the number of intervals in the tree and k is the number of overlapping intervals. In practice, since k is really small compared to n for most variants, the effective runtime on interval tree would be O(k lg n) . We improved to O(lg n + k) by creating an interval array where all intervals are stored in a sorted array so that we only need to find the first overlapping interval and then enumerate through the remaining (k-1).

b. CNVs/SVs (Yu): annotations for Copy Number Variation and Structural Variants can be provided. Similar to the annotation of small variants, transcripts overlapping with the SV and also previously reported structural variants can be annotated in online databases. Unlike the small variants, not all overlapping transcripts need be annotated, since too many transcripts will be overlapped with a large SVs. Instead, all overlapping transcripts can be annotated that belong to a partial overlapping gene. Specifically, for these transcripts, the impacted introns, exons and the consequences caused by the structural variants can be reported. An option to allow output all overlapping transcripts is available, but the basic information for these transcripts can be reported, such as gene symbol, flag whether it is canonical overlap or partial overlapped with the transcripts. For each SV/CNV, it is also of interest to know if these variants have been studied and their frequencies in different populations. Hence, we reported overlapping SVs in external databases, such as 1000 genomes, DGV and ClinGen. To avoid using an arbitrary cutoff to determine which SV is overlapped, instead all overlapping transcripts can be used and the reciprocal overlap can be calculated, i.e. the overlapping length divided by the minimum of the length of these two SVs.

c. Reporting supplementary annotations : Supplementary annotations are of two types: small and structural variants (SVs). SVs can be modeled as intervals and use the interval array discussed above to identify overlapping SVs. Small variants are modeled as points and matched by position and (optionally) allele. As such, they are searched using a binary-search-like algorithm. Since the supplementary annotation database can be quite large, a much smaller index is created to map chromosome positions to file locations where the supplementary annotation resides. The index is a sorted array of objects (made up of chromosome position and file location) that can be binary searched using position. To keep the index size small, multiple positions (up to a certain max count) are compressed to one object that stores the values for the first position and only deltas for subsequent positions. Since we use Binary search, the runtime is O(lg n), where n is the number of items in the database.

d. VEP cache files

e. Transcript Database : The Transcript Cache (cache) and Supplementary database (SAdb) files are serialized dump of data objects such as transcripts and supplementary annotations. We use Ensembl VEP cache as our data source for cache. To create the cache, all transcripts are inserted in an interval array and the final state of the array is stored in the cache files. Thus, during annotation, we only need to load a pre-computed interval array and perform searches on it. Since the cache is loaded up in memory and searching is very fast (described above), finding overlapping transcripts is extremely quick in Nirvana (profiled to less than 1% of total runtime?).

f. Supplementary Database : The data sources for SAdb are listed under supplementary material. The SAdb for small variants is produced by a k -way merge of all data sources such that each object in the database (identified by reference name and position) holds all relevant supplementary annotations. Issues encountered during parsing data source files have been documented in detail in Nirvana's home page. To limit memory usage, only the SA index is loaded up in memory. This index allows a quick lookup of the file location for a supplementary annotation. However, since the data has to be fetched from disk, adding supplementary annotation has been identified as Nirvana's largest bottleneck (profiled at ~30% of total runtime.)

g. Consequence and Sequence Ontology : Nirvana's functional annotation (when provided) follows the Sequence Ontology (SO) (http://www.sequenceontology.org/) guidelines. On occasions, we had the opportunity to identify issues in the current SO and collaborate with the SO team to improve the state of annotation.

Such a variant annotation tool can include pre-processing. For example, Nirvana included a large number of annotations from External data sources, like ExAC, EVS, 1000 Genomes project, dbSNP, ClinVar, Cosmic, DGV and ClinGen. To make full use of these databases, we have to sanitize the information from them. We implemented different strategy to deal with different conflicts that exist from different data sources. For example, in case of multiple dbSNP entries for the same position and alternate allele, we join all ids into a comma separated list of ids; if there are multiple entries with different CAF values for the same allele, we use the first CAF value. For conflicting ExAC and EVS entries, we consider the number of sample counts and the entry with higher sample count is used. In 1000 Genome Projects, we removed the allele frequency of the conflicting allele. Another issue is inaccurate information. We mainly extracted the allele frequencies information from 1000 Genome Projects, however, we noticed that for GRCh38, the allele frequency reported in the info field did not exclude samples with genotype not available, leading to deflated frequencies for variants which are not available for all samples. To guarantee the accuracy of our annotation, we use all of the individual level genotype to compute the true allele frequencies. As we know, the same variants can have different representations based on different alignments. To make sure we can accurately report the information for already identified variants, we have to preprocess the variants from different resources to make them have consistent representation. For all external data sources, we trimmed alleles to remove duplicated nucleotides in both reference allele and alternative allele. For ClinVar, we directly parsed the xml file we performed a five-prime alignment for all variants, which is often used in vcf file. Different databases can contain the same set of information. To avoid unnecessary duplicates, we removed some duplicated information. For example, we removed variants in DGV which has data source as 1000 genome projects, since we already reported these variants in 1000 genomes with more detailed information.

In accordance with at least some implementations, the variant call application provides calls for low frequency variants, germline calling and the like. As non-limiting example, the variant call application may run on tumor-only samples and/or tumor-normal paired samples. The variant call application may search for single nucleotide variations (SNV), multiple nucleotide variations (MNV), indels and the like. The variant call application identifies variants, while filtering for mismatches due to sequencing or sample preparation errors. For each variant, the variant caller identifies the reference sequence, a position of the variant, and the potential variant sequence(s) (e.g., A to C SNV, or AG to A deletion). The variant call application identifies the sample sequence (or sample fragment), a reference sequence/fragment, and a variant call as an indication that a variant is present. The variant call application may identify raw fragments, and output a designation of the raw fragments, a count of the number of raw fragments that verify the potential variant call, the position within the raw fragment at which a supporting variant occurred and other relevant information. Non-limiting examples of raw fragments include a duplex stitched fragment, a simplex stitched fragment, a duplex un-stitched fragment and a simplex un- stitched fragment.

The variant call application may output the calls in various formats, such as in a .VCF or .GVCF file. By way of example only, the variant call application may be included in a MiSeqReporter pipeline (e.g., when implemented on the MiSeq® sequencer instrument). Optionally, the application may be implemented with various workflows. The analysis may include a single protocol or a combination of protocols that analyze the sample reads in a designated manner to obtain desired information.

Then, the one or more processors perform a validation operation in connection with the potential variant call. The validation operation may be based on a quality score, and/or a hierarchy of tiered tests, as explained hereafter. When the validation operation authenticates or verifies that the potential variant call, the validation operation passes the variant call information (from the variant call application) to the sample report generator. Alternatively, when the validation operation invalidates or disqualifies the potential variant call, the validation operation passes a corresponding indication (e.g., a negative indicator, a no call indicator, an in-valid call indicator) to the sample report generator. The validation operation also may pass a confidence score related to a degree of confidence that the variant call is correct or the in-valid call designation is correct.

Next, the one or more processors generate and store a sample report. The sample report may include, for example, information regarding a plurality of genetic loci with respect to the sample. For example, for each genetic locus of a predetermined set of genetic loci, the sample report may at least one of provide a genotype call; indicate that a genotype call cannot be made; provide a confidence score on a certainty of the genotype call; or indicate potential problems with an assay regarding one or more genetic loci. The sample report may also indicate a gender of an individual that provided a sample and/or indicate that the sample include multiple sources. As used herein, a "sample report" may include digital data (e.g., a data file) of a genetic locus or predetermined set of genetic locus and/or a printed report of the genetic locus or the set of genetic loci. Thus, generating or providing may include creating a data file and/or printing the sample report, or displaying the sample report.

The sample report may indicate that a variant call was determined, but was not validated. When a variant call is determined invalid, the sample report may indicate additional information regarding the basis for the determination to not validate the variant call. For example, the additional information in the report may include a description of the raw fragments and an extent (e.g., a count) to which the raw fragments support or contradicted the variant call. Additionally or alternatively, the additional information in the report may include the quality score obtained in accordance with implementations described herein.

## Variant Call Application

Implementations disclosed herein include analyzing sequencing data to identify potential variant calls. Variant calling may be performed upon stored data for a previously performed sequencing operation. Additionally or alternatively, it may be performed in real time while a sequencing operation is being performed. Each of the sample reads is assigned to corresponding genetic loci. The sample reads may be assigned to corresponding genetic loci based on the sequence of the nucleotides of the sample read or, in other words, the order of nucleotides within the sample read (e.g., A, C, G, T). Based on this analysis, the sample read may be designated as including a possible variant/allele of a particular genetic locus. The sample read may be collected (or aggregated or binned) with other sample reads that have been designated as including possible variants/alleles of the genetic locus. The assigning operation may also be referred to as a calling operation in which the sample read is identified as being possibly associated with a particular genetic position/locus. The sample reads may be analyzed to locate one or more identifying sequences (e.g., primer sequences) of nucleotides that differentiate the sample read from other sample reads. More specifically, the identifying sequence(s) may identify the sample read from other sample reads as being associated with a particular genetic locus.

The assigning operation may include analyzing the series of n nucleotides of the identifying sequence to determine if the series of n nucleotides of the identifying sequence effectively matches with one or more of the select sequences. In particular implementations, the assigning operation may include analyzing the first n nucleotides of the sample sequence to determine if the first n nucleotides of the sample sequence effectively matches with one or more of the select sequences. The number n may have a variety of values, which may be programmed into the protocol or entered by a user. For example, the number n may be defined as the number of nucleotides of the shortest select sequence within the database. The number n may be a predetermined number. The predetermined number may be, for example, 10, 11, 12, 13, 14, 15, 16, 17, 18, 19, 20, 21, 22, 23, 24, 25, 26, 27, 28, 29, or 30 nucleotides. However, fewer or more nucleotides may be used in other implementations. The number n may also be selected by an individual, such as a user of the system. The number n may be based on one or more conditions. For instance, the number n may be defined as the number of nucleotides of the shortest primer sequence within the database or a designated number, whichever is the smaller number. In some implementations, a minimum value for n may be used, such as 15, such that any primer sequence that is less than 15 nucleotides may be designated as an exception.

In some cases, the series of n nucleotides of an identifying sequence may not precisely match the nucleotides of the select sequence. Nonetheless, the identifying sequence may effectively match the select sequence if the identifying sequence is nearly identical to the select sequence. For example, the sample read may be called for a genetic locus if the series of n nucleotides (e.g., the first n nucleotides) of the identifying sequence match a select sequence with no more than a designated number of mismatches (e.g., 3) and/or a designated number of shifts (e.g., 2). Rules may be established such that each mismatch or shift may count as a difference between the sample read and the primer sequence. If the number of differences is less than a designated number, then the sample read may be called for the corresponding genetic locus (i.e., assigned to the corresponding genetic locus). In some implementations, a matching score may be determined that is based on the number of differences between the identifying sequence of the sample read and the select sequence associated with a genetic locus. If the matching score passes a designated matching threshold, then the genetic locus that corresponds to the select sequence may be designated as a potential locus for the sample read. In some implementations, subsequent analysis may be performed to determine whether the sample read is called for the genetic locus.

If the sample read effectively matches one of the select sequences in the database (i.e., exactly matches or nearly matches as described above), then the sample read is assigned or designated to the genetic locus that correlates to the select sequence. This may be referred to as locus calling or provisional-locus calling, wherein the sample read is called for the genetic locus that correlates to the select sequence. However, as discussed above, a sample read may be called for more than one genetic locus. In such implementations, further analysis may be performed to call or assign the sample read for only one of the potential genetic loci. In some implementations, the sample read that is compared to the database of reference sequences is the first read from paired- end sequencing. When performing paired-end sequencing, a second read (representing a raw fragment) is obtained that correlates to the sample read. After assigning, the subsequent analysis that is performed with the assigned reads may be based on the type of genetic locus that has been called for the assigned read.

Next, the sample reads are analyzed to identify potential variant calls. Among other things, the results of the analysis identify the potential variant call, a sample variant frequency, a reference sequence and a position within the genomic sequence of interest at which the variant occurred. For example, if a genetic locus is known for including SNPs, then the assigned reads that have been called for the genetic locus may undergo analysis to identify the SNPs of the assigned reads. If the genetic locus is known for including polymorphic repetitive DNA elements, then the assigned reads may be analyzed to identify or characterize the polymorphic repetitive DNA elements within the sample reads. In some implementations, if an assigned read effectively matches with an STR locus and an SNP locus, a warning or flag may be assigned to the sample read. The sample read may be designated as both an STR locus and an SNP locus. The analyzing may include aligning the assigned reads in accordance with an alignment protocol to determine sequences and/or lengths of the assigned reads. The alignment protocol may include the method described in International Patent Application No. PCT/US2013/030867 (Publication No. WO 2014/142831), filed on March 15, 2013.

Then, the one or more processors analyze raw fragments to determine whether supporting variants exist at corresponding positions within the raw fragments. Various types of raw fragments may be identified. For example, the variant caller may identify a type of raw fragment that exhibits a variant that validates the original variant call. For example, the type of raw fragment may represent a duplex stitched fragment, a simplex stitched fragment, a duplex un-stitched fragment or a simplex un-stitched fragment. Optionally other raw fragments may be identified instead of or in addition to the foregoing examples. In connection with identifying each type of raw fragment, the variant caller also identifies the position, within the raw fragment, at which the supporting variant occurred, as well as a count of the number of raw fragments that exhibited the supporting variant. For example, the variant caller may output an indication that 10 reads of raw fragments were identified to represent duplex stitched fragments having a supporting variant at a particular position X. The variant caller may also output indication that five reads of raw fragments were identified to represent simplex un-stitched fragments having a supporting variant at a particular position Y. The variant caller may also output a number of raw fragments that corresponded to reference sequences and thus did not include a supporting variant that would otherwise provide evidence validating the potential variant call at the genomic sequence of interest.

Next, a count is maintained of the raw fragments that include supporting variants, as well as the position at which the supporting variant occurred. Additionally or alternatively, a count may be maintained of the raw fragments that did not include supporting variants at the position of interest (relative to the position of the potential variant call in the sample read or sample fragment). Additionally or alternatively, a count may be maintained of raw fragments that correspond to a reference sequence and do not authenticate or confirm the potential variant call. The information determined is output to the variant call validation application, including a count and type of the raw fragments that support the potential variant call, positions of the supporting variance in the raw fragments, a count of the raw fragments that do not support the potential variant call and the like.

When a potential variant call is identified, the process outputs an indicating of the potential variant call, the variant sequence, the variant position and a reference sequence associated therewith. The variant call is designated to represent a "potential" variant as errors may cause the call process to identify a false variant. In accordance with implementations herein, the potential variant call is analyzed to reduce and eliminate false variants or false positives. Additionally or alternatively, the process analyzes one or more raw fragments associated with a sample read and outputs a corresponding variant call associated with the raw fragments.

**Variant Filter**

Variant filter **111** includes a convolutional neural network (CNN) and a fully-connected neural network (FCNN). The input to the variant filter **111** are overlaid samples of nucleotide sequences from the overlaid samples database **119.** The nucleotide sequences from the nucleotide sequences database **169** are overlaid with repeat patterns from the repeat patterns database **196** to generate overlaid samples. An overlayer **181** overlays repeat patterns on nucleotide sequences from the database **169** to produce overlaid samples that are stored in the overlaid samples database **119.** The simulator **116** feeds combinations of repeat patterns overlaid on at least 100 nucleotide sequences in at least 100 overlaid samples to the variant filter for analysis. When overlaid samples with repeat pattern under test are given as input the variant filter **111,** the variant filter **111** outputs classification scores for likelihood that the variant nucleotide in each of the overlaid samples is a true variant or a false variant. Finally, the analyzer **194** causes display of the classification scores as a distribution for each of the repeat factors to support evaluation of sequence-specific error causation by the repeat patterns.

**Repeat Patterns**

A repeat pattern generator **171** generates repeat patterns "rp" using homopolymer or copolymer patterns of length "n" with distinct repeat factors "m". The homopolymer repeat patterns comprise a single base (A, C, G, or T) while copolymer repeat patterns comprise more than one bases. A "repeat pattern" is generated by applying a "repeat factor (m)" to a "pattern". The relationship between a pattern of length (n), a repeat factor (m) and a repeat pattern (rp) is represented by equation (1) as:

$$\text{pattern} * m = rp \qquad (1)$$

Table 1, presents examples of homopolymer repeat patterns. The length of homopolymer patterns is one i.e., "n = 1".

| n = Pattern Length | Pattern | m = Repeat Factor | Repeat Pattern (rp) |
|---|---|---|---|
| 1 | A | 5 | AAAAA (5 As) |
| 1 | A | 9 | AAAAAAAAA (9 As) |
| 1 | A | 13 | AAAAAAAAAAAAA (13 As) |
| 1 | A | 17 | AAAAAAAAAAAAAAAAA (17 As) |
| 1 | A | 21 | AAAAAAAAAAAAAAAAAAAAA (21 As) |
| 1 | A | 25 | AAAAAAAAAAAAAAAAAAAAAAAAA (25 As) |
| 1 | C | 5 | CCCCC (5 Cs) |
| 1 | C | 9 | CCCCCCCCC (9 Cs) |
| 1 | C | 13 | CCCCCCCCCCCCC (13 Cs) |
| 1 | C | 17 | CCCCCCCCCCCCCCCCC (17 Cs) |
| 1 | C | 21 | CCCCCCCCCCCCCCCCCCCCC (21 Cs) |
| 1 | C | 25 | CCCCCCCCCCCCCCCCCCCCCCCCC (25 Cs) |
| 1 | T | 5 | TTTTT (5 Cs) |
| 1 | T | 9 | TTTTTTTTT (9 Ts) |
| 1 | T | 13 | TTTTTTTTTTTTT (13 Ts) |
| 1 | T | 17 | TTTTTTTTTTTTTTTTT (17 Ts) |
| 1 | T | 21 | TTTTTTTTTTTTTTTTTTTTT (21 Ts) |
| 1 | T | 25 | TTTTTTTTTTTTTTTTTTTTTTTTT (25 Ts) |
| 1 | G | 5 | TTTTT (5 Cs) |
| 1 | G | 9 | TTTTTTTTT (9 Ts) |
| 1 | G | 13 | TTTTTTTTTTTTT (13 Ts) |
| 1 | G | 17 | TTTTTTTTTTTTTTTTT (17 Ts) |
| 1 | G | 21 | TTTTTTTTTTTTTTTTTTTTT (21 Ts) |
| 1 | G | 25 | TTTTTTTTTTTTTTTTTTTTTTTTT (25 Ts) |

A table 2, presents example repeat patterns of copolymers. The length of copolymer patterns is greater than one i.e., "n>1".

| n = Pattern Length | Pattern | m = Repeat Factor | Repeat Pattern (rp) |
|---|---|---|---|
| 2 | AC | 1 | AC (1 AC) |
| 2 | AC | 3 | ACACAC (3 ACs) |
| 2 | AC | 5 | ACACACACAC (5 ACs) |
| 2 | AC | 7 | ACACACACACACAC (7 ACs) |
| 2 | AC | 9 | ACACACACACACACACAC (9 ACs) |
| 2 | AC | 11 | ACACACACACACACACACACAC (11 ACs) |
| 2 | TA | 1 | TA (1 TA) |
| 2 | TA | 3 | TATATA (3 TAs) |
| 2 | TA | 5 | TATATATATA (5 TAs) |
| 2 | TA | 7 | TATATATATATA(7TAs) |
| 2 | TA | 9 | TATATATATATATATA (9 TAs) |

(continued)

| n = Pattern Length | Pattern | m = Repeat Factor | Repeat Pattern (rp) |
| --- | --- | --- | --- |
| 2 | TA | 11 | TATATATATATATATATATA (11 TAs) |
| 3 | AAT | 1 | AAT (1 AAT) |
| 3 | AAT | 2 | AATAAT (2 AATs) |
| 3 | AAT | 3 | AATAATAAT (3 AATs) |
| 3 | AAT | 4 | AATAATAATAAT (4 AATs) |
| 3 | AAT | 5 | AATAATAATAATAAT (5 AATs) |
| 3 | AAT | 6 | AATAATAATAATAATAAT (6 AATs) |
| 4 | CTAT | 1 | CTAT (1 CTAT) |
| 4 | CTAT | 2 | CTATCTAT (2 CTATs) |
| 4 | CTAT | 3 | CTATCTATCTAT (3 CTATs) |
| 4 | CTAT | 4 | CTATCTATCTATCTAT (4 CTATs) |
| 4 | CTAT | 5 | CTATCTATCTATCTATCTAT (5 CTATs) |
| 4 | CTAT | 6 | CTATCTATCTATCTATCTATCTAT (5 CTATs) |

## Variant Filter

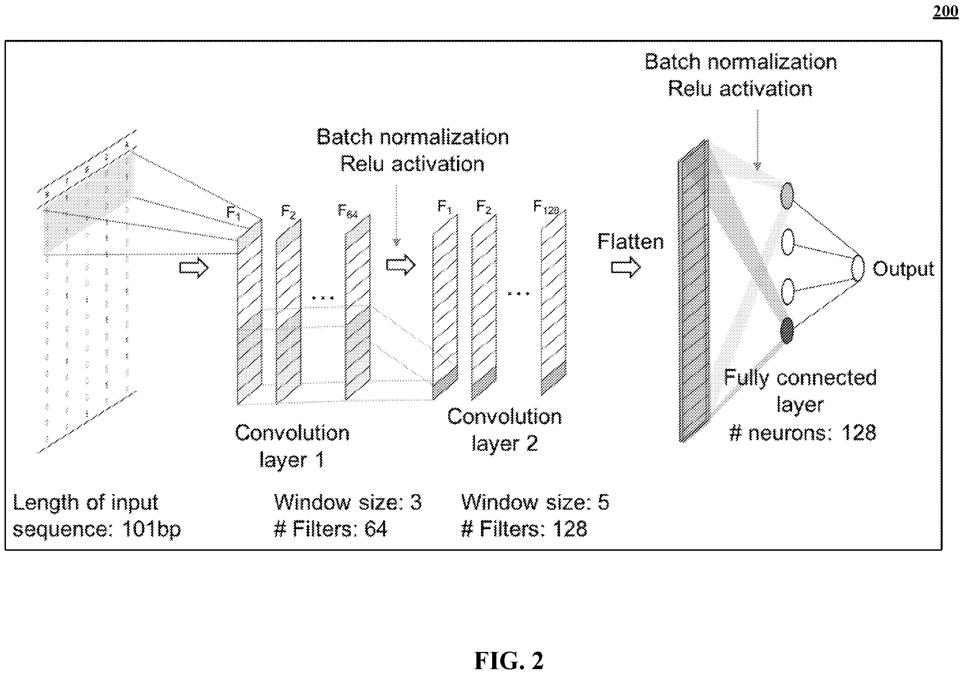

**FIG. 2** illustrates an example architecture **200** of the variant filter **111.** The variant filter **111** has a hierarchical structure built on a convolutional neural network (CNN) and a fully-connected neural network (FCNN). DeepPOLY uses the variant filter **111** to test known sequence patterns for their effect on variant filtering. The input to variant filter **111** comprises nucleotide sequences of length 101 having a variant nucleotide at the center and flanked on the left and the right by 50 nucleotides. It is understood that nucleotide sequences of different lengths can be used as inputs to the variant filter **111.**

The convolutional neural network comprises convolution layers which perform the convolution operation between the input values and convolution filters (matrix of weights) that are learned over many gradient update iterations during the training.

Let $(m, n)$ be the filter size and $W$ be the matrix of weights, then a convolution layer performs a convolution of the $W$ with the input $X$ by calculating the dot product $W \cdot x + b$, where $x$ is an instance of $X$ and $b$ is the bias. The step size by which the convolution filters slide across the input is called the stride, and the filter area $(m \times n)$ is called the receptive field. A same convolution filter is applied across different positions of the input, which reduces the number of weights learned. It also allows location invariant learning, i.e., if an important pattern exists in the input, the convolution filters learn it no matter where it is in the sequence. Additional details about convolutional neural network can be found in I. J. Goodfellow, D. Warde-Farley, M. Mirza, A. Courville, and Y. Bengio, "CONVOLUTIONAL NETWORKS," Deep Learning, MIT Press, 2016; J. Wu, "INTRODUCTION TO CONVOLUTIONAL NEURAL NETWORKS," Nanjing University, 2017; and N. ten DIJKE, "Convolutional Neural Networks for Regulatory Genomics," Master's Thesis, Universiteit Leiden Opleiding Informatica, 17 June 2017. The convolutional neural network architecture illustrated in **FIG. 2** has two convolution layers. The first convolution layer processes the input using 64 filters of size 3 each. The output of the first convolution layer is passed through a batch normalization layer.

Distribution of each layer of the convolutional neural network changes during training and it varies from one layer to another. This reduces the convergence speed of the optimization algorithm. Batch normalization (Ioffe and Szegedy 2015) is a technique to overcome this problem. Denoting the input of a batch normalization layer with $x$ and its output using $z$, batch normalization applies the following transformation on $x$:

$$z = \frac{x - \mu}{\sqrt{\sigma^2 + \varepsilon}}\gamma + \beta$$

Batch normalization applies mean-variance normalization on the input $x$ using $\mu$ and $\sigma$ and linearly scales and shifts it using $\gamma$ and $\beta$. The normalization parameters $\mu$ and $\sigma$ are computed for the current layer over the training set using a method called exponential moving average. In other words, they are not trainable parameters. In contrast, $\gamma$ and $\beta$ are trainable parameters. The values for $\mu$ and $\sigma$ calculated above during training are used in forward pass during production. A rectified linear unit (ReLU) nonlinearity function is applied to the output of batch normalization layer to produce a normalized output. Other examples of nonlinearity functions include sigmoid, hyperbolic tangent (tanh), and leaky ReLU.

A second convolution layer operates 128 filters of size 5 on the normalized output. The example CNN shown in **FIG. 2,** includes a flattening layer which flattens the output from the second convolution layer to a one dimensional array which is passed through a second set of batch normalization and ReLU activations layers. The normalized output from the second convolution layer is fed to the fully-connected neural network (FCNN). The fully-connected neural network comprises fully-connected layers - each neuron receives input from all the previous layer's neurons and sends its output to every neuron in the next layer. This contrasts with how convolutional layers work where the neurons send their output to only some of the neurons in the next layer. The neurons of the fully-connected layers are optimized over many gradient update iterations during the training. Additional details about the fully-connected neural network can be found in I. J. Goodfellow, D. Warde-Farley, M. Mirza, A. Courville, and Y. Bengio, "CONVOLUTIONAL NETWORKS," Deep Learning, MIT Press, 2016; J. Wu, "INTRODUCTION TO CONVOLUTIONAL NEURAL NETWORKS," Nanjing University, 2017; and N. ten DIJKE, "Convolutional Neural Networks for Regulatory Genomics," Master's Thesis, Universiteit Leiden Opleiding Informatica, 17 June 2017. A classification layer (e.g., softmax layer) following the full-connected layers produces classification scores for likelihood that each candidate variant at the target nucleotide position is a true variant or a false variant. The classification layer can be a softmax layer or a sigmoid layer. The number of classes and their type can be modified, depending on the implementation.

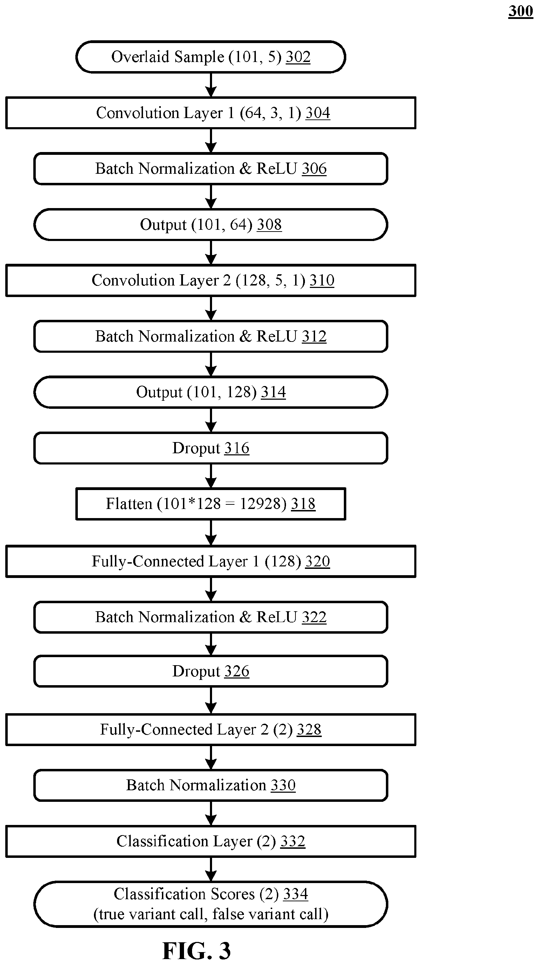

**FIG. 3** shows one implementation of the processing pipeline **300** of the variant filter **111.** In the illustrated implementation, the convolution neural network (CNN) has two convolution layers and the fully-connected neural network (FCNN) has two fully-connected layers. In other implementations, the variant filter **111,** and its convolution neural network and fully-connected neural network, can have additional, fewer, or different parameters and hyperparameters. Some examples of parameters are number of convolution layers, number of batch normalization and ReLU layers, number of fully-connected layers, number of convolution filters in respective convolution layers, number of neurons in respective fully-connected layers, number of outputs produced by the final classification layer, and residual connectivity. Some examples of hyperparameters are window size of the convolution filters, stride length of the convolution filters, padding, and dilation. In the discussion below, the term "layer" refers to an algorithm implemented in code as a software logic or module. Some examples of layers can be found in Keras™ documentation available at https://keras.io/layers/about-keras-layers/, the complete subject matter of which is expressly incorporated herein by reference in its entirety.

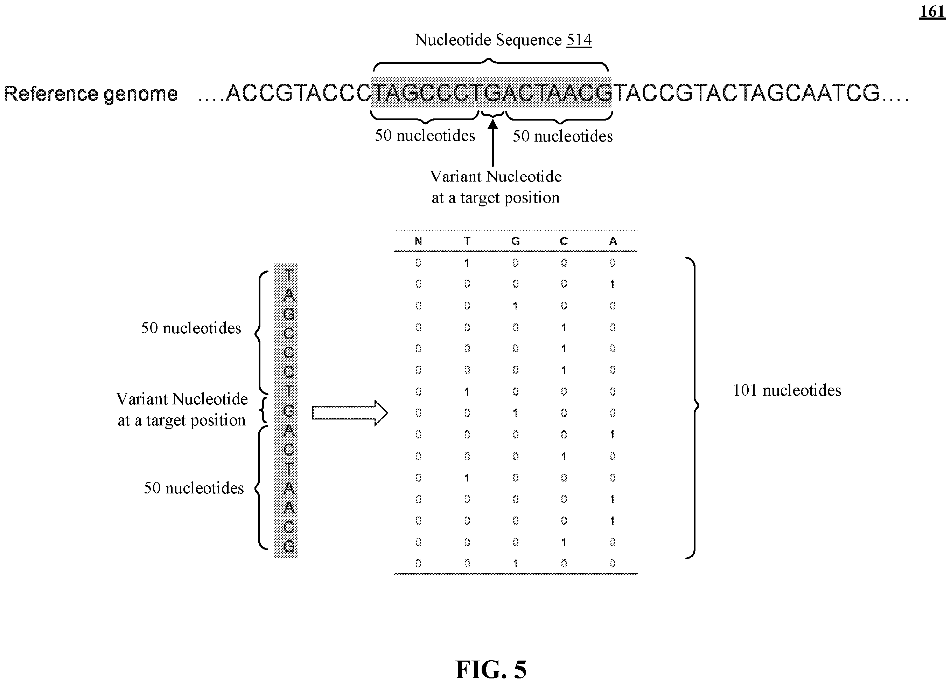

A one-hot encoded input sequence **302** is fed to a first convolution layer **304** of the convolutional neural network (CNN). The dimensionality of the input sequence **302** is 101, 5, where 101 represents the 101 nucleotides in the input sequence **302** with a particular variant at a center target position flanked by 50 nucleotides on each side, and 5 represents the 5 channels A, T, C, G, N used to encode the input sequence **302.** The preparation of input sequences **302** is described with reference to **FIG. 5.**

The first convolution layer **304** has 64 filters, each of which convolves over the input sequence **302** with a window size of 3 and stride length of 1. The convolution is followed by batch normalization and ReLU nonlinearity layers **306.** What results is an output (feature map) **308** of dimensionality 101, 64. Output **308** can be regarded as the first intermediate convolved feature.

Output **308** is fed as input to a second convolution layer **310** of the convolutional neural network. The second convolution layer **310** has 128 filters, each of which convolves over the output **308** with a window size of 5 and stride length of 1. The convolution is followed by batch normalization and ReLU nonlinearity layers **312.** What results is an output (feature map) **314** of dimensionality 101, 128. Output **314** can be regarded as the second intermediate convolved feature and also the final output of the convolutional neural network.

Dropout is an effective technique to prevent a neural network from overfitting. It works by randomly dropping a fraction of neurons from the network in each iteration of the training. This means that output and gradients of selected neurons are set to zero so they do not have any impact on forward and backward passes. In **FIG. 3,** dropout is performed at dropout layer **316** using a probability of 0.5.

After processing the output through the dropout layer, the output is flattened by a flattening layer **318** to allow downstream processing by the fully-connected neural network. Flattening includes vectorizing the output **314** to have either one row or one column. That is, by way of example, converting the output **314** of dimensionality 101, 128 into a flattened vector of dimensionality 1, 12928 (1 row and 101x128 = 12928 columns).

The flattened output of dimensionality 1, 12928 from flattening layer **318** is then fed as input to the fully-connected neural network (FCNN). The fully-connected neural network has two fully-connected layers **320** and **328.** The first fully-connected layer **320** has 128 neurons, which are fully connected to 2 neurons in the second fully-connected layer **328.** The first fully-connected layer **320** is followed by a batch normalization, ReLU non-linearity and dropout layers **322,** and **326.** The second fully-connected layer **328** is followed by a batch normalization layer **330.** The classification layer **332** (e.g., softmax) has 2 neurons which output the 2 classification scores or probabilities **334** for the particular variant being a true variant or a false variant.

**Performance of the Variant Caller on Held-Out Data**

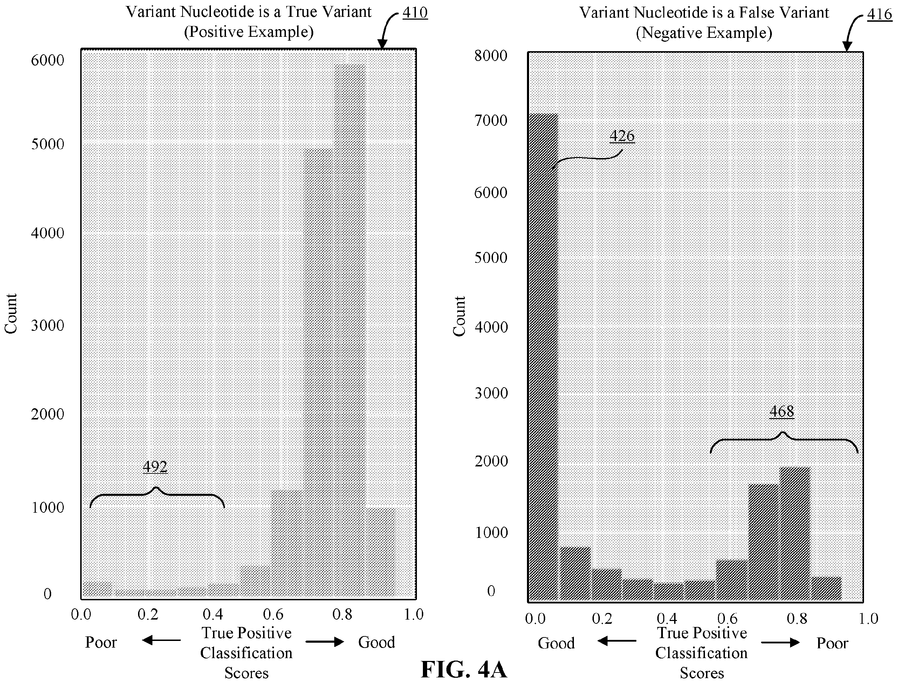

**FIG. 4A** shows true and false positive plots that graphically illustrate the variant filter's performance on held-out data. There are 28,000 validation examples in the held-out data set, with about 14,000 validation examples of true variants (positive examples) and 14,000 validation examples of false variants (negative examples). The two plots **410** and **416** show performance of the variant filter **111** when 28,000 validation examples are fed as input during the validation stage. The graphs **410** and **416** plot the classification scores along x-axis indicating the confidence of the trained model in predicting the true variants and the false variants as true positive. Thus, the trained model is expected to produce high classification scores for the true variants and low classification scores for the false variants. The height of the vertical bars indicate the count of validation examples with respective classification scores along the x-axis.

Plot **416** shows that the variant filter **111** classified more than 7,000 validation examples of false variants as "low confidence true positives" (i.e., classification score < 0.5 (e.g., **426**)), confirming that the model successfully learned to classify negative examples as false variants. The variant filter **111** classified some validation examples of false variants as "high confidence true positives" (e.g., **468**). This occurred because, in the training data and/or in the held-out data, some de novo variants observed in only one child were mislabeled as false variants when they were actually true variants.

Plot **410** shows that the variant filter **111** classified more than 11,000 validation examples of true variants as "high confidence true positives" (i.e., classification score > 0.5), confirming that the model successfully learned to classify positive examples as true variants.

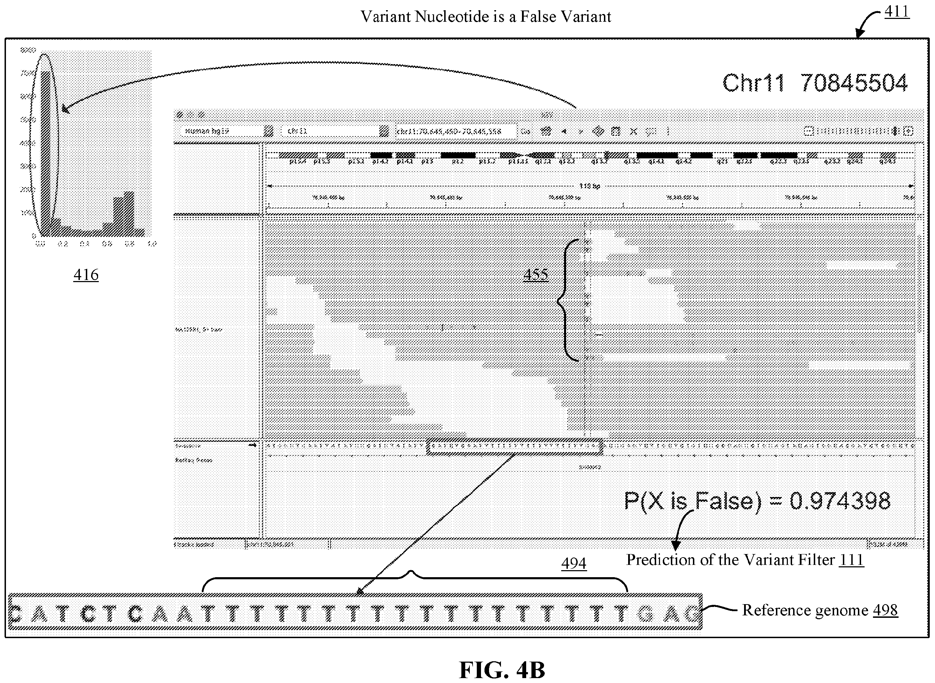

In **FIG. 4B,** the classification results of the variant filter **111** are compared against analysis derived from a pile-up image that aligns reads produced by a sequencer to a reference sequence **498**. The reference sequence **498** comprises a homopolymer repeat pattern of length18 of a single base "T" as shown by label **494** in **FIG. 4B.** The pile-up image shows that at least seven reads (indicated by reference label **455**) reported a "T" base at the position of a "G" nucleotide with respect to the reference genome **498**. Therefore, there are two possible resulting calls for calling the base at this position in the sequence: "G" or "T". The ground truth from the "platinum genomes pedigree" shows that none of the parents and grandparents have a variant nucleotide at this position in their respective reference sequences. Therefore, "T" base call is determined as "false positive" that occurred due to a sequencing error. In addition, the pile-up image shows that the "Ts" appear only at the end of read 1, which further confirms that the variant is false.

The performance of the variant filter **111** is consistent with the above analysis because the variant filter **111** classified the nucleotide at this position as a false variant with a high confidence, as illustrated in **FIG. 4B** by "P(X is False) = 0.974398".

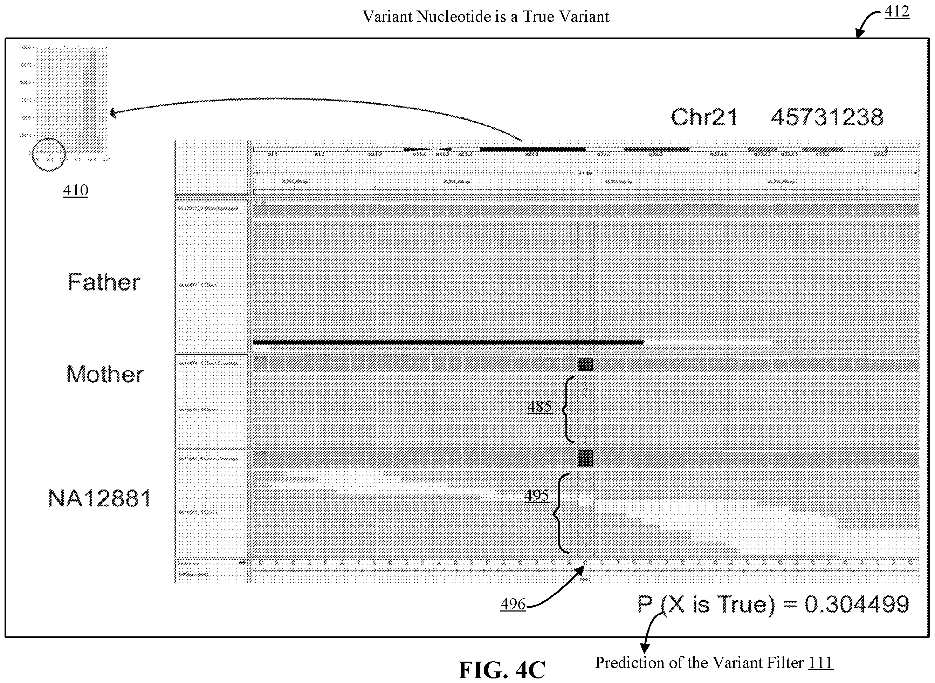

**FIG. 4C** shows pile-up image **412** of sequencing reads for an example that contains a true variant. The sequencing reads for the child (labelled as "NA12881") has at least three "T" nucleotides identified by a label **495**. The reference sequence has a "C" nucleotide at that position as identified by a label **496**. However, the mother's sequencing reads indicate at least seven "T" nucleotides at the same position. Therefore, this is an instance of an example having a true variant as shown by the plot **410** on the top left corner. The variant filter **111** classified this example as a true positive with a low confidence score ("P(X is True) = 0.304499"). That is, the variant filter **111** classified the target nucleotide as a false variant (or weakly classified as a true variant) because of presence of a repeat pattern of copolymer "AC" before the target nucleotide's position. The trained sequence considers repeat pattern as a potential sequence-specific error (SSE) and therefore, classified the variant "T" with a low confidence score.

**FIG. 5** shows an example input preparation by the input preparer **161** using one-hot encoding to encode the overlaid nucleotide sequences having a variant nucleotide at a target position for input to the variant filter **111.** A nucleotide sequence **514** comprising of at least 50 nucleotides on both sides (left and right) of a variant nucleotide at a target position is used for preparing the input. Note that the nucleotide sequence **514** is a portion of the reference genome. In one-hot encoding, each base pair in a sequence is encoded with a binary vector of four bits, with one of the bits being hot (i.e., 1) while other being 0. For instance, T = (1, 0, 0, 0), G = (0, 1, 0, 0), C = (0, 0, 1, 0), and A = (0, 0, 0, 1). In some implementations, an unknown nucleotide is encoded as N = (0, 0, 0, 0). The figure shows an example nucleotide sequence of 101 nucleotides represented using one-hot encoded.

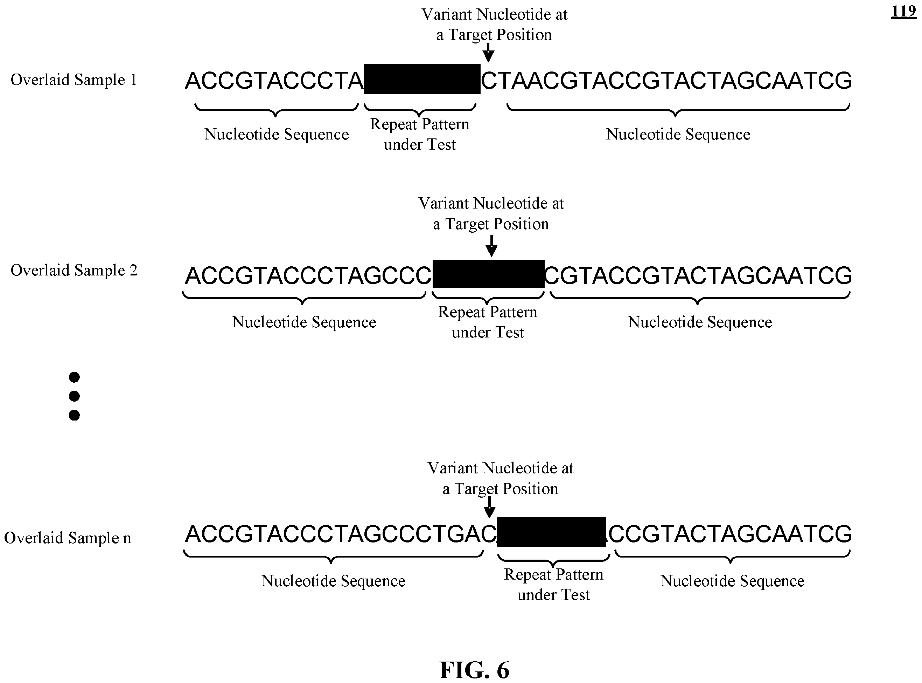

**FIG. 6** illustrates preparation of overlaid samples produced by the input preparer by overlaying the repeat patterns on nucleotide sequences. The overlaid samples are stored in the overlaid samples database **119**. The example shows an overlaid sample 1 which is generated by overlaying a homopolymer repeat pattern of 7 "A"s to left of a center nucleotide at a target position in overlaid sample. An overlaid sample 2 is created by overlaying the same repeat pattern of 7 "A"s on the nucleotide sequence to include a center nucleotide. A third overlaid sample n is generated by overlaying the repeat pattern of 7 "A"s to the right of a center nucleotide in the overlaid samples.

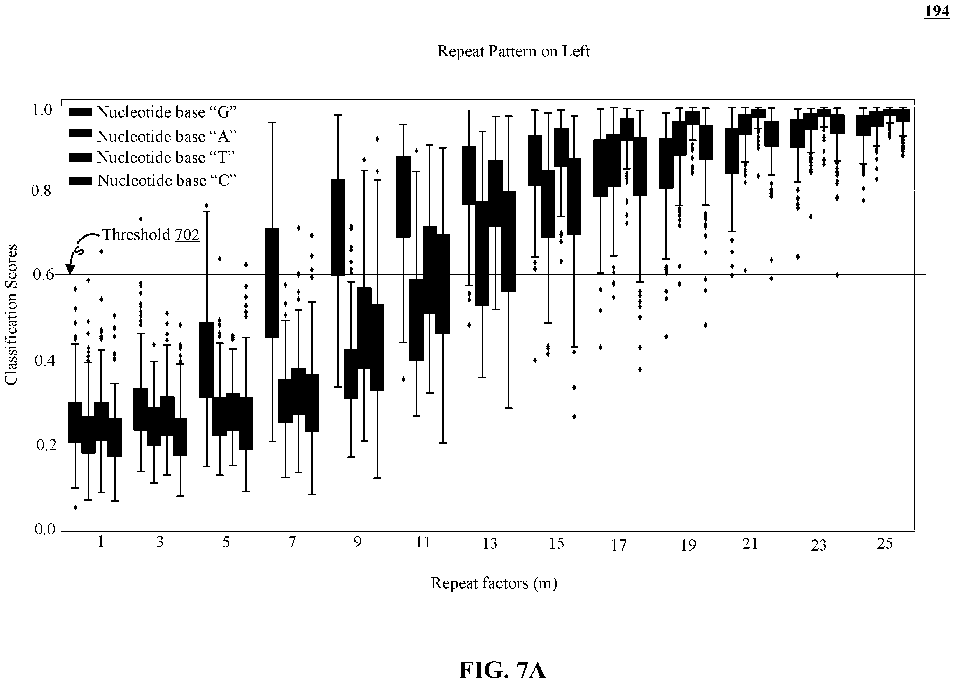

The variant filter subsystem, translates analysis by the variant filter **111** into classification scores for likelihood that the variant nucleotide in each of the overlaid samples is a true variant or a false variant. The variant filter subsystem is followed by an analysis subsystem in which the analyzer **194,** causes the display of the classification scores as a distribution for each of the repeat factors to support evaluation of sequence-specific error causation by the repeat patterns. **FIGs. 7A** to **7C** present examples of such display from the analyzer **194. FIG. 7A** using a box-and-whisker plot to identify sequence-specific error causation by repeat pattern overlaid left of a center nucleotide in the overlaid samples.

The y-axis of the graphical plot shows distribution of the classification scores outputted by the variant filter when the overlaid samples containing different repeat pattern were fed to the variant filter as input. The x-axis shows the repeat factors (m) applied to the pattern that produced the repeat pattern fed as input. The repeat patterns considered here are homopolymers generated by using repeat factors indicated on the x-axis. The example shows four box-and-whisker plots per unique repeat factor value. The four plots correspond to homopolymer repeat patterns of the four type of nucleotides (G, A, T, and C). Each repeat pattern is placed on at least 100 nucleotides sequences to generate 100 overlaid samples fed as input to the CNN of the variant filter **111**. In another implementation, at least 200 nucleotide sequences are used to generate at least 200 overlaid samples per repeat pattern. The same process is repeated to generate homopolymer repeat patterns for all repeat factors shown along the x-axis.

The graphical plot in **FIG. 7A** shows that shorter repeat patterns (length less than 10 nucleotides) of a single base "G" can introduce sequence-specific errors in variant identification. Similarly, shorter repeat patterns of a single base "C" can also introduce some errors while repeat patterns of nucleotides bases "A" and "T" are less likely to cause sequence-specific errors when repeat patterns are short. However, longer repeat patterns (length greater than 10 nucleotides) of all four types of nucleotides cause more sequence specific errors.

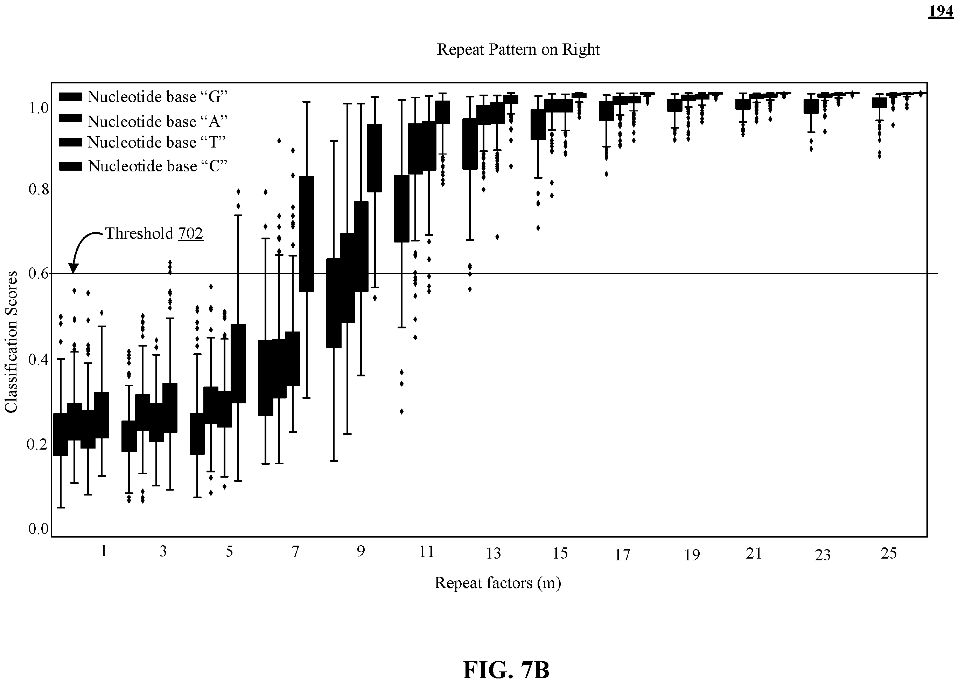

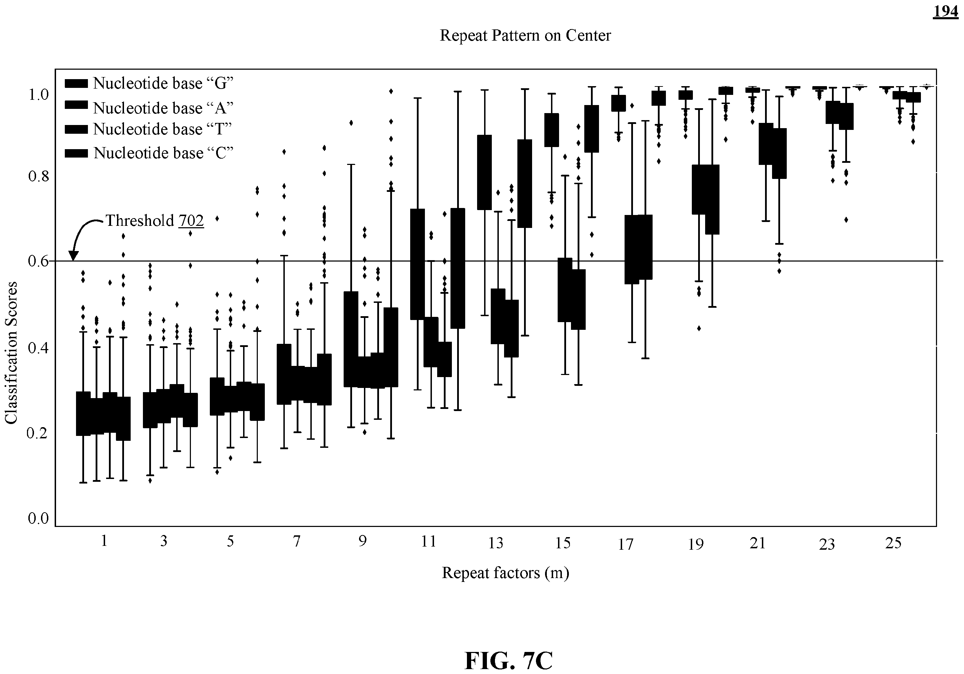

**FIG. 7B** is a box-and-whisker plot displaying classification scores as a distribution for likelihood that a variant nucleotide is true variant or a false variant when repeat patterns are overlaid on a nucleotide sequence to right of a center nucleotide in the overlaid samples. As compared to **FIG. 7A,** the shorter patterns of homoplymers of a single nucleotide "C" are more likely to cause an error in identification of a true variant. **FIG. 7C** is a box-and-whisker plot displaying classification scores as a distribution for likelihood that a variant nucleotide is a true variant or a false variant when the repeat patterns include a center nucleotide (at a target position) in the overlaid samples. As compared to **FIGs. 7A** and **7B,** the **FIG. 7C** shows that shorter repeat patterns of all four nucleotide types are less likely to cause a sequence-specific error in variant identification.

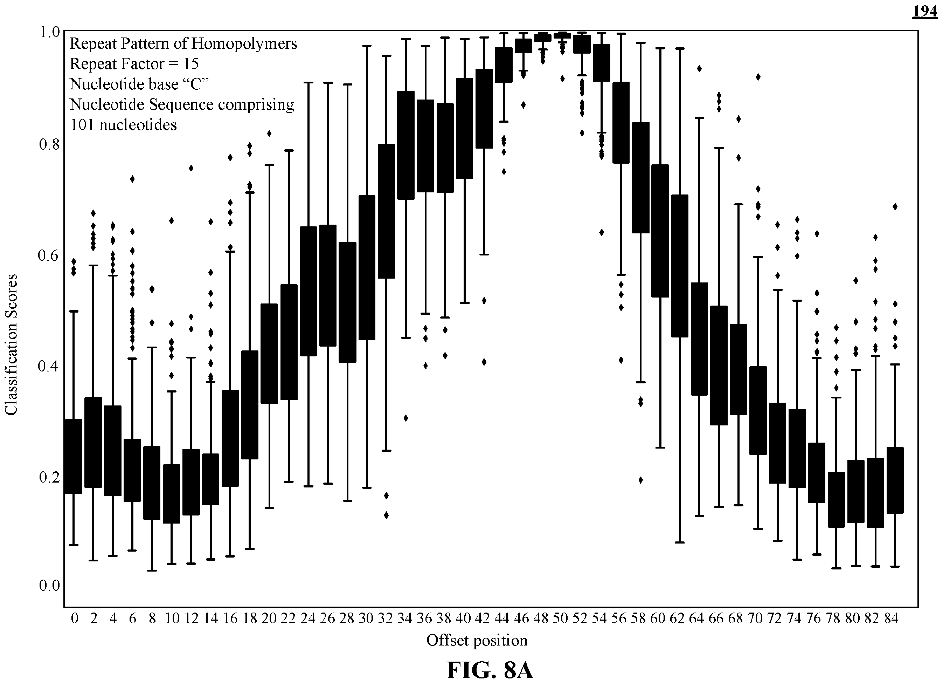

**FIGs. 8A** to **8C** present graphical plots to identify sequence specific errors causation when the homopolymers repeat patterns of a single base (A, C, G, or T) are overlaid at varying offsets on nucleotide sequences to produce overlaid samples. The varying offsets vary a position at which the repeat patterns are overlaid on the nucleotide sequences. The varying offset is measurable as an offset between an origin position of the repeat patterns and an origin position of the nucleotide sequences. In one implementation, at least ten offsets are used to produce overlaid samples. Ten is a reasonable floor to generate overlaid samples with repeat patterns at a variety of offsets to analyze the sequence specific errors causation.

**FIG. 8A** is a box-and-whisker plot to identify sequence-specific errors causation by repeat patterns of homopolymers of a single base "C" overlaid at varying offsets on nucleotide sequences. The repeat factor m=15 which means that the repeat pattern is a homopolymer of length 15 of a single base "C". This repeat pattern is overlaid on nucleotide sequences consisting of 101 nucleotides to generate overlaid samples at varying offsets. For each offset value, combinations of repeat patterns overlaid on at least 100 nucleotide sequences in at least 100 overlaid samples are fed to the CNN of the variant filter of **FIG. 1**. The **FIG. 8A** shows box-and-whisker plots for offset positions at 0, 2, 4, up to 84 when repeat pattern of 15 single bases "C" is overlaid on the nucleotide sequences. For example, when the offset is "0", the origin position of the repeat pattern coincides with the origin position of the nucleotide sequences. At offset "2", the origin position of the repeat pattern is aligned to the third base (at an index of 2) to overlay the repeat pattern on the nucleotide sequences. As the offset increases, the overlaid repeat pattern is closer to the variant nucleotide at a target position nucleotide sequence. In the example used for the illustration purposes in **FIG. 8A,** the target nucleotide is at index position of "50" which is the center of the nucleotide sequence comprising 101 nucleotides. As the offset value increases above 50, the repeat pattern moves past the variant nucleotide and is positioned on the right side of the variant nucleotide at the target position.

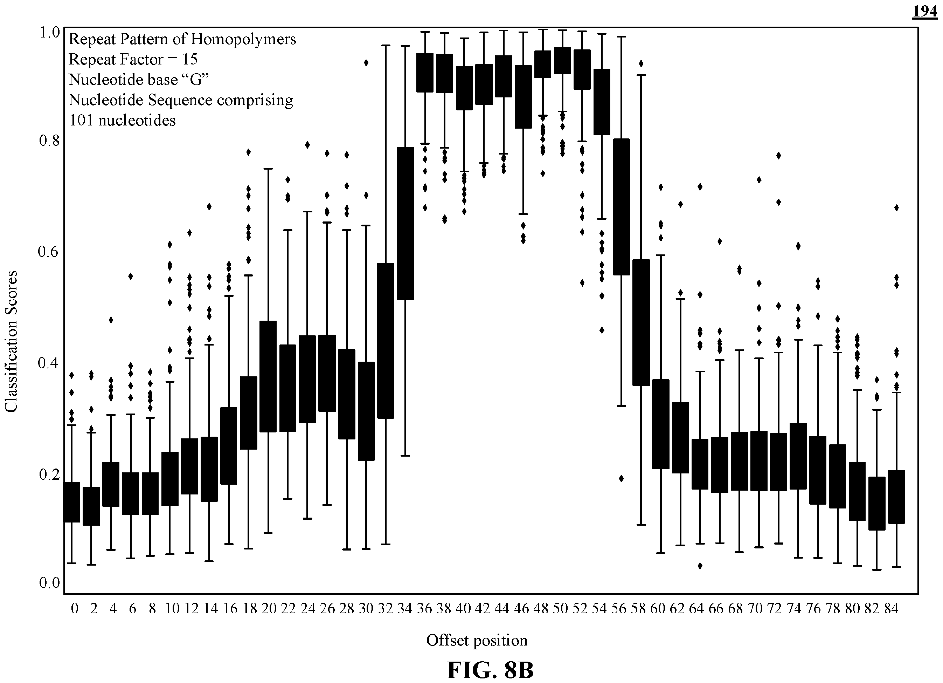

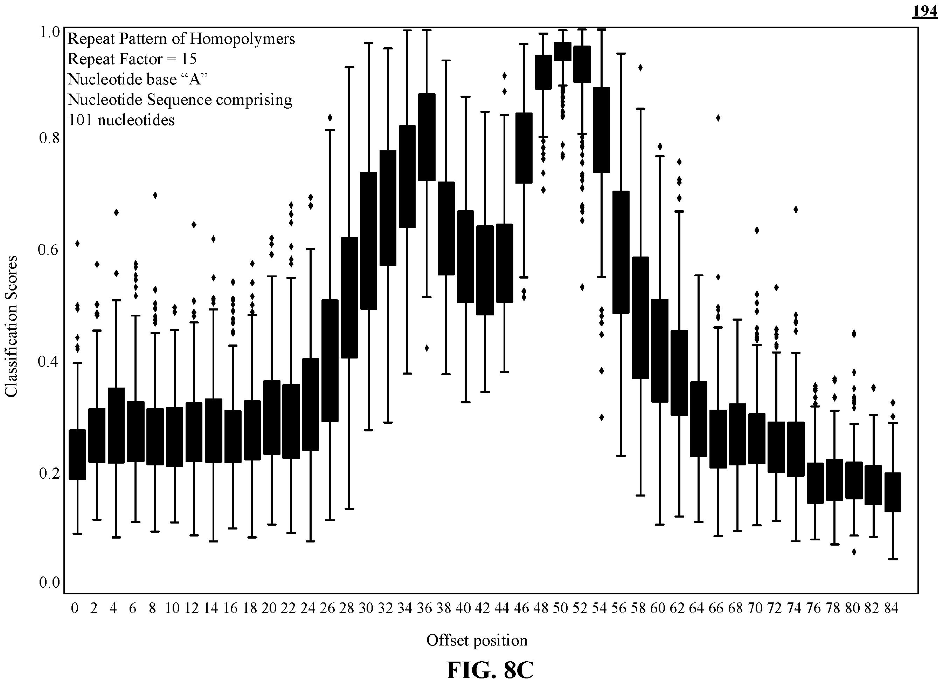

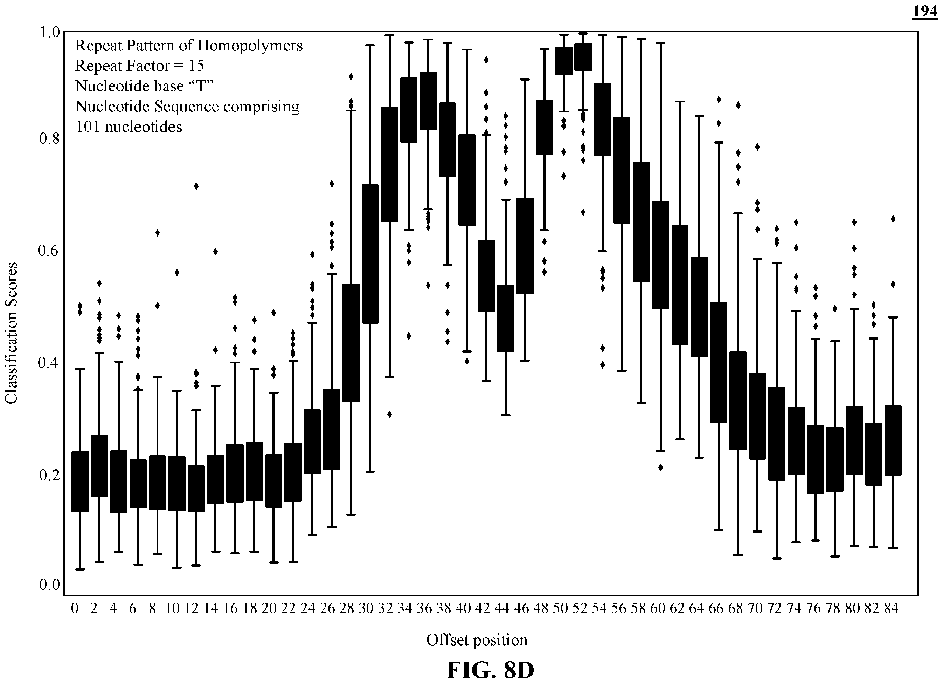

**FIGs. 8B, 8C,** and **8D** are similar box-and-whisker plots as described above to identify sequence-specific errors causation by repeat patterns of homopolymers of single bases "G", "A", and "T" respectively, overlaid at varying offsets on nucleotide sequences. The repeat factor m=15 for each of the three repeat patterns.

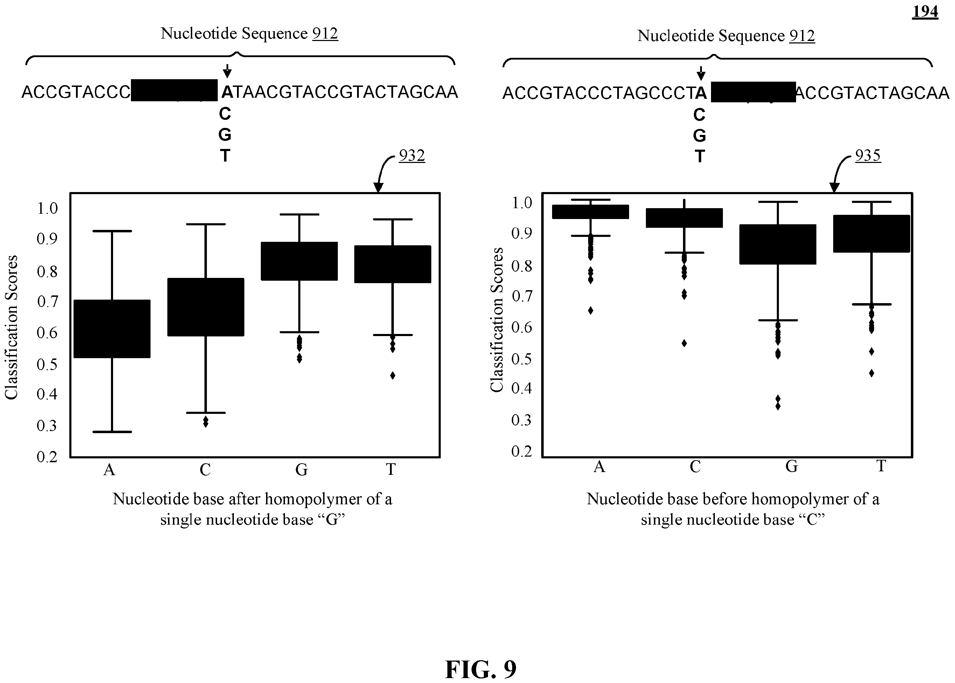

**FIG. 9** shows display of classification scores as a distribution for likelihood that a variant nucleotide is a true variant or a false variant when repeat patterns of homopolymers of a single base are overlaid "before" and "after" a variant nucleotide. The homopolymer repeat patterns are overlaid one by one before and after variant nucleotides at a target position to produce overlaid samples. A box-and-whisker plot **932** shows classification scores when a homopolymer repeat pattern of a single base "G" is overlaid to left of a center nucleotide on a nucleotide sequence. The results are generated for four types of nucleotides (A, C, G, and T) as the variant nucleotide at a target position followed by the homopolymer repeat pattern. The results show that classification scores vary by a bigger spread if the target nucleotide is of type "A" and "C".

A graphical plot **935** shows a similar visualization but for a homopolymer repeat pattern of a single base "C" overlaid to right of a center nucleotide on a nucleotide sequence **912**. The comparison of box-and-whisker plots show a larger spread of classification scores when a target nucleotide is of type "G".

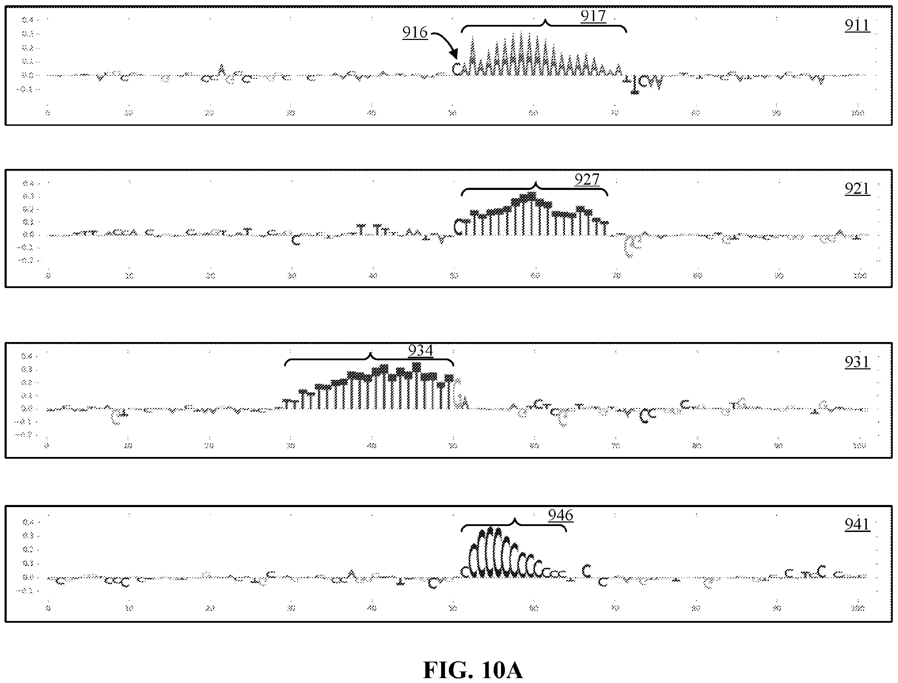

**FIGs. 10A** to **10C** present display of naturally occurring repeat patterns of copolymers in each of the sample nucleotide sequences that contribute to false variant classification. The graphical visualizations presented in **FIGs. 10A** to **10C** are generated using DeepLIFT presented by Shrikumar et. el., in their paper, "Not Just a Black Box: Learning Important Features Through Propagating Activation Differences" available at https://arxiv.org/pdf/1605.01713.pdf (reference 1). The implementation of the DeepLIFT model is presented at http://github.com/kundajelab/deeplift (reference 2) and further details for implementing DeepLIFT are presented at https://www.biorxiv.org/content/biorxiv/suppl/2017/10/05/105957.DC1/105957-o.pdf (reference 3). One or more naturally occurring repeat patterns of copolymers including a variant nucleotide at a target position are given as input to the DeepLIFT model to generate the visualizations shown in FIGs. 10A to 10C. The output of the DeepLIFT model are the arrays of contributions of input to variant classification of a variant nucleotide at the target position.

For example, consider the input sequence shown in the graphical visualization **911**. The variant nucleotide **916** is at position 50 in the sample nucleotide sequence comprising of 101 nucleotides. The variant nucleotide at the target position is flanked by at 50 nucleotides on each side at positions 0 to 49 and 51 to 100 in the sample nucleotide sequence. The variant filter 111 of **FIG. 2,** classified the variant nucleotide ("C") at the target position as a false variant. The output of the DeepLIFT is the visualization **911** showing that the naturally occurring repeat pattern **917** contributed the most to the classification of the variant nucleotide **916.** The heights of the nucleotides indicate their respective contributions to the classification of the variant nucleotide. As shown in the graphical visualization **911,** the highest contribution is from a sequence of nucleotides **917** which is a repeat pattern comprising a single base "A".

DeepLIFT contribution arrays have the same shape as the input, i.e., input sequence of nucleotides multiplied by 4 for the standard one-hot encoding (presented in **FIG. 5**). Therefore, DeepLIFT assigns scores to each sequence position by summing over contributions of input neurons associated with a fixed sequence position and associate these summed contributions with the nucleotide present at that position in the input sample nucleotide sequence. The summed contributions are referred to as "DeepLIFT interpretation scores". The following recommended best practices (as presented in reference 3 above) are followed in application of the DeepLIFT model. Contributions of input neurons to the pre-activation (activation before applying final non-linearity) of an output neuron is calculated. When an output layer uses a softmax non-linearity, the weights connecting a fixed penultimate layer neuron to the set of output neurons are mean centered. Because the sample nucleotide sequences are one-hot encoded as shown in **FIG. 5,** the method of "weight normalization for constrained inputs" is used before converting from Keras to DeepLIFT as described in reference 3 above.

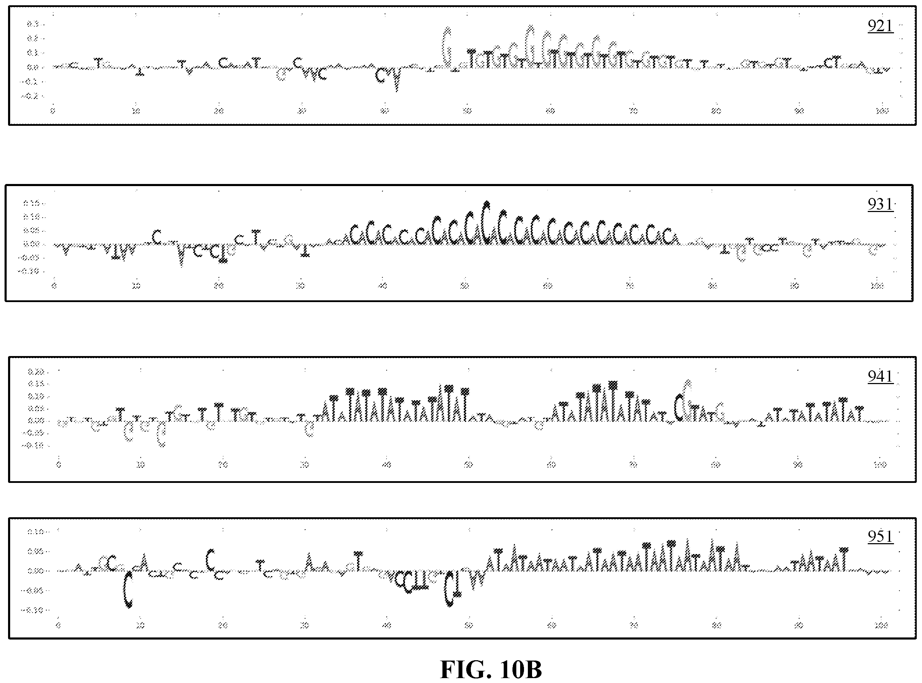

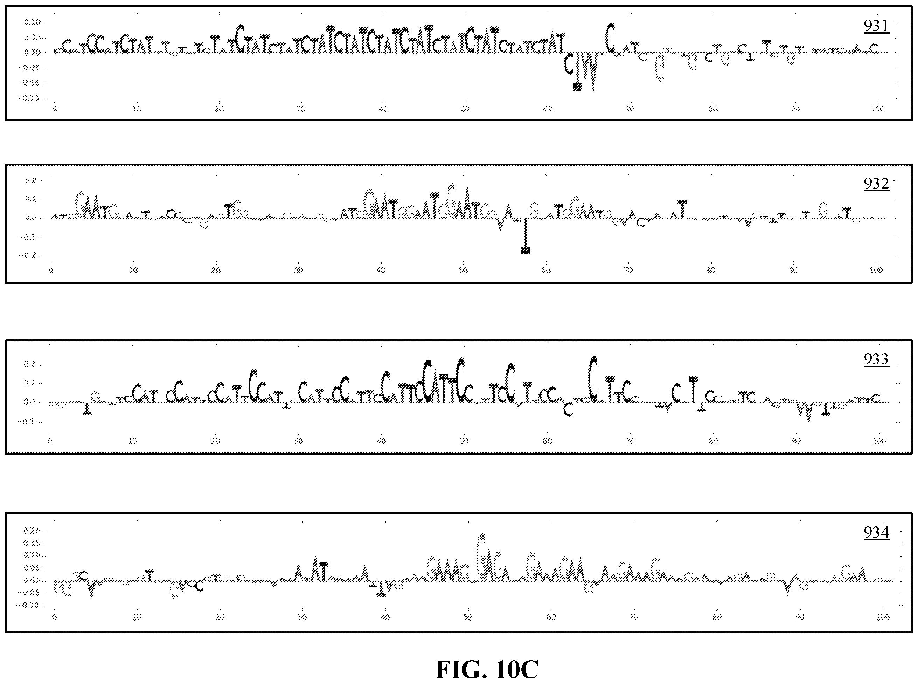

Graphical visualizations **921, 931,** and **941** show repeat patterns **927, 934,** and **946** respectively, contributing the most to the classification of the variant nucleotide in the sample nucleotide sequences. **FIG. 10B** includes graphical visualizations **921, 931, 941,** and **951.** Note that in these graphical visualizations the repeat patterns of copolymers contain patterns of two or more nucleotides. Similarly, **FIG. 10C** presents more examples of graphical visualizations **931, 932, 933,** and **934,** illustrating a variety of repeat patterns contributing to the classification of the variant nucleotide at the target position in respective input nucleotide sequences.

## Computer System

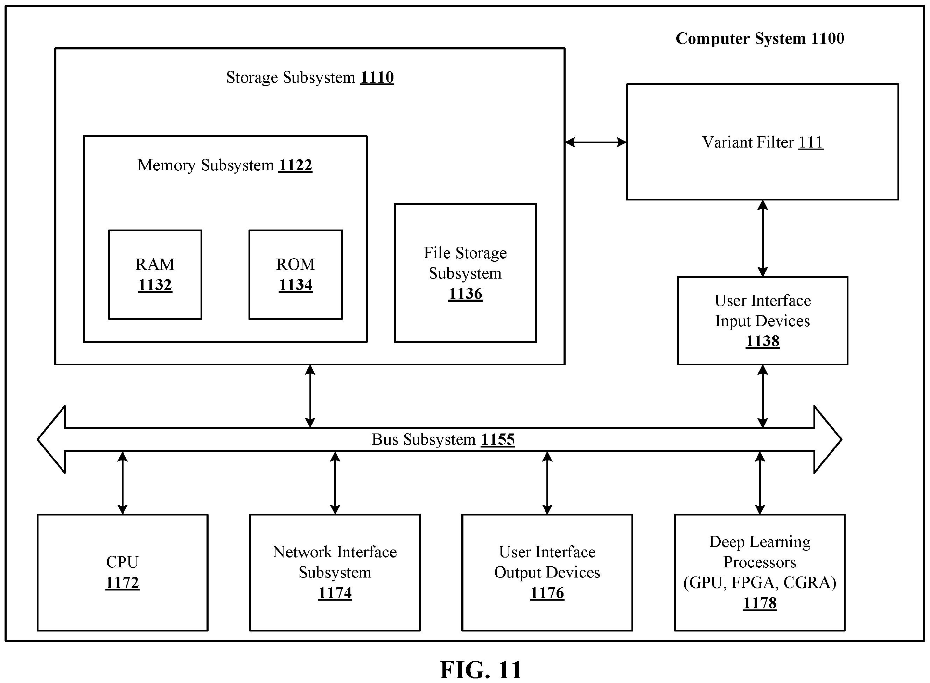

**FIG. 11** is a simplified block diagram of a computer system **1100** that can be used to implement the variant filter **111** of **FIG. 1** for identifying repeat patterns that cause sequence-specific errors. Computer system **1100** includes at least one central processing unit (CPU) **1172** that communicates with a number of peripheral devices via bus subsystem **1155.** These peripheral devices can include a storage subsystem **1110** including, for example, memory devices and a file storage subsystem **1136,** user interface input devices **1138,** user interface output devices **1176,** and a network interface subsystem **1174.** The input and output devices allow user interaction with computer system **1100.** Network interface subsystem **1174** provides an interface to outside networks, including an interface to corresponding interface devices in other computer systems.

In one implementation, the variant filter **111** of **FIG. 1** is communicably linked to the storage subsystem **1110** and the user interface input devices **1138.**

User interface input devices **1138** can include a keyboard; pointing devices such as a mouse, trackball, touchpad, or graphics tablet; a scanner; a touch screen incorporated into the display; audio input devices such as voice recognition systems and microphones; and other types of input devices. In general, use of the term "input device" is intended to include all possible types of devices and ways to input information into computer system **1100.**

User interface output devices **1176** can include a display subsystem, a printer, a fax machine, or non-visual displays such as audio output devices. The display subsystem can include an LED display, a cathode ray tube (CRT), a flat-panel device such as a liquid crystal display (LCD), a projection device, or some other mechanism for creating a visible image. The display subsystem can also provide a non-visual display such as audio output devices. In general, use of the term "output device" is intended to include all possible types of devices and ways to output information from computer system **1100** to the user or to another machine or computer system.

Storage subsystem **1110** stores programming and data constructs that provide the functionality of some or all of the modules and methods described herein. Subsystem **1178** can be graphics processing units (GPUs) or field-programmable gate arrays (FPGAs).

Memory subsystem **1122** used in the storage subsystem **1110** can include a number of memories including a main random access memory (RAM) **1132** for storage of instructions and data during program execution and a read only memory (ROM) **1134** in which fixed instructions are stored. A file storage subsystem **1136** can provide persistent storage for program and data files, and can include a hard disk drive, a floppy disk drive along with associated removable media, a CD-ROM drive, an optical drive, or removable media cartridges. The modules implementing the functionality of certain implementations can be stored by file storage subsystem **1136** in the storage subsystem **1110,** or in other machines accessible by the processor.

Bus subsystem **1155** provides a mechanism for letting the various components and subsystems of computer system **1100** communicate with each other as intended. Although bus subsystem **1155** is shown schematically as a single bus, alternative implementations of the bus subsystem can use multiple busses.

Computer system **1100** itself can be of varying types including a personal computer, a portable computer, a workstation, a computer terminal, a network computer, a television, a mainframe, a server farm, a widely-distributed set of loosely networked computers, or any other data processing system or user device. Due to the ever-changing nature of computers and networks, the description of computer system **1100** depicted in **FIG. 11** is intended only as a specific example for purposes of illustrating the preferred embodiments of the present invention. Many other configurations of computer system **1100** are possible having more or less components than the computer system depicted in **FIG. 11.**

### Sequence-Specific Error (SSE) Correlation

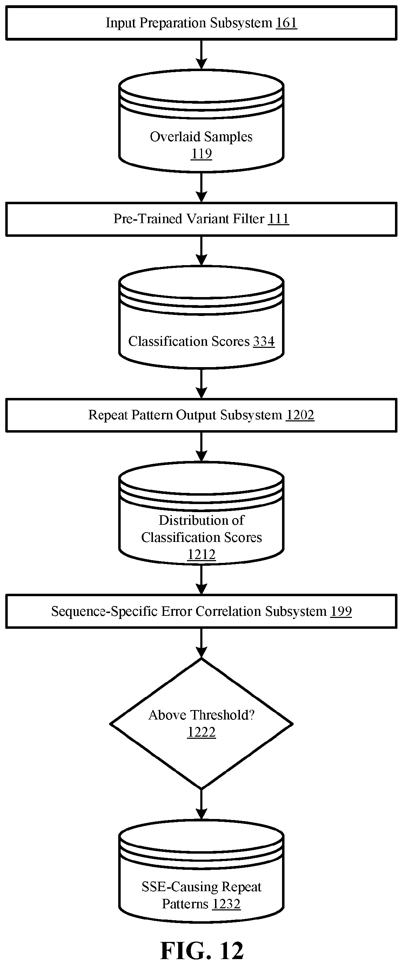

**FIG. 12** illustrates one implementation of how sequence-specific errors (SSEs) are correlated to repeat patterns based on false variant classifications.

The input preparation subsystem **161** computationally overlays the repeat patterns under test on numerous nucleotide sequences and produces the overlaid samples **119.** Each repeat pattern represents a particular nucleotide composition that has a particular length and appears in an overlaid sample at a particular offset position. Each overlaid sample has a target position considered to be a variant nucleotide. For each combination of the particular nucleotide composition, the particular length, and the particular offset position, a set of the overlaid samples is computationally generated.

The pre-trained variant filter subsystem **111** processes the overlaid samples **119** through the convolutional neural network **200** and, based on detection of nucleotide patterns in the overlaid samples **119** by convolution filters of the convolutional neural network **200,** generates classification scores **334** for likelihood that the variant nucleotide in each of the overlaid samples is a true variant or a false variant.

The repeat pattern output subsystem **1202** outputs distributions **1212** of the classification scores **334** that indicate susceptibility of the pre-trained variant filter subsystem **111** to false variant classifications resulting from presence of the repeat patterns.

The sequence-specific error correlation subsystem **199** specifies, based on a threshold **1222,** a subset of the classification scores as indicative of the false variant classifications, and classifies those repeat patterns **1232** which are associated with the subset of the classification scores that are indicative of the false variant classifications as causing the sequence-specific errors. The sequence-specific error correlation subsystem **199** classifies particular lengths and particular offset positions of the repeat patterns **1232** classified as causing the sequence-specific errors as also causing the sequence-specific errors.

**Figures 7A, 7B,** and **7C** show an example threshold **702** (e.g., 0.6) that is applied to the outputs distributions **1212** of the classification scores **334** to identify the subset of the classification scores which are above the threshold **702.** Such classification scores are indicative of the false variant classifications and repeat patterns associated with them are classified as causing the sequence-specific errors.

### Particular Implementations

The technology disclosed relates to identifying repeat patterns that cause sequence-specific errors.

The technology disclosed can be practiced as a system, method, device, product, computer readable media, or article of manufacture. One or more features of an implementation can be combined with the base implementation. Implementations that are not mutually exclusive are taught to be combinable. One or more features of an implementation can be combined with other implementations. This disclosure periodically reminds the user of these options. Omission from some implementations of recitations that repeat these options should not be taken as limiting the combinations taught in the preceding sections - these recitations are hereby incorporated forward by reference into each of the following implementations.

A first system implementation of the technology disclosed includes one or more processors coupled to memory. The memory is loaded with computer instructions to identify repeat patterns that cause sequence-specific errors. The system includes an input preparation subsystem running on numerous processors operating in parallel and coupled to memory. The input preparation subsystem overlays repeat patterns under test on nucleotide sequences to produce overlaid samples. Each of the overlaid samples has a variant nucleotide at a target position flanked by at least 20 nucleotides on each side. The repeat patterns are homopolymers of a single base (A, C, G, or T) with at least 6 repeat factors that specify a number of repetitions of the single base in the repeat patterns. The system includes a simulation subsystem that feeds each combination of the repeat patterns overlaid on at least 100 nucleotide sequences in at least 100 overlaid samples to a variant filter for analysis. The system includes a variant filter subsystem, which translates analysis by the variant filter into classification scores for likelihood that the variant nucleotide in each of the overlaid samples is a true variant or a false variant. Finally, the system includes an analysis subsystem that causes display of the classification scores as a distribution for each of the repeat factors to support evaluation of sequence-specific error causation by the repeat patterns.

This system implementation and other systems disclosed optionally include one or more of the following features. System can also include features described in connection with methods disclosed. In the interest of conciseness, alternative combinations of system features are not individually enumerated. Features applicable to systems, methods, and articles of manufacture are not repeated for each statutory class set of base features. The reader will understand how features identified in this section can readily be combined with base features in other statutory classes.

In one implementation, the repeat patterns are to right of a center nucleotide in the overlaid samples and not overlapping the center nucleotide. In another implementation, the repeat patterns are to left of a center nucleotide in the overlaid samples and not overlapping the center nucleotide. In another implementation, the repeat patterns include a center nucleotide in the overlaid samples.

The repeat factors are integers in a range of 5 to one-quarter of a count of nucleotides in the overlaid samples. The system is further configured to apply to repeat patterns that are the homopolymers of the single base for each of four bases (A, C, G, and T).

The input preparation subsystem is further configured to produce the repeat patterns and the overlaid samples for the homopolymers for each of the four bases and the analysis subsystem is further configured to cause display of the classification score distribution for each of the homopolymers in juxtaposition.

The repeat patterns are right to a center nucleotide in the overlaid samples and the juxtaposition applies to the homopolymers overlaid right to the center nucleotide. The repeat patterns are left to a center nucleotide in the overlaid samples and the juxtaposition applies to the homopolymers overlaid left to the center nucleotide. The nucleotide sequences on which the repeat patterns are overlaid are randomly generated. The nucleotide sequences on which the repeat patterns are overlaid are randomly selected from naturally occurring DNA nucleotide sequences. The analysis subsystem is further configured to cause display of the classification score distribution for each of the repeat factors using box-and-whisker plots.

The variant filter is trained on at least 500000 training examples of true variants and at least 50000 training examples of false variants. Each training example is a nucleotide sequence with a variant nucleotide at a target position flanked by at least 20 nucleotides on each side. The variant filter is a convolutional neural network (CNN) with two convolutional layers and a fully-connected layer.

Other implementations may include a non-transitory computer readable storage medium storing instructions executable by a processor to perform functions of the system described above. Yet another implementation may include a method performing the functions of the system described above.

A first computer-implemented method implementation of the technology disclosed includes identifying repeat patterns that cause sequence-specific errors. The computer-implemented method includes preparing input by overlaying repeat patterns under test on nucleotide sequences to produce overlaid samples. Each of the overlaid samples has a variant nucleotide at a target position flanked by at least 20 nucleotides on each side. The repeat patterns are homopolymers of a single base (A, C, G, or T) with at least 6 repeat factors that specify a number of repetitions of the single base in the repeat patterns. The computer-implemented method includes feeding each combination of the repeat patterns overlaid on at least 100 nucleotide sequences in at least 100 overlaid samples to a variant filter for analysis. The computer-implemented method includes translating analysis by the variant filter into classification scores for likelihood that the variant nucleotide in each of the overlaid samples is a true variant or a false variant into an output. Finally, the computer-implemented method includes causing display of the classification scores as a distribution for each of the repeat factors to support evaluation of sequence-specific error causation by the repeat patterns.

Each of the features discussed in this particular implementation section for the first system implementation apply equally to this computer-implemented method implementation. As indicated above, all the system features are not repeated here and should be considered repeated by reference.

A computer readable media (CRM) implementation includes a non-transitory computer readable storage medium storing instructions executable by a processor to perform a computer-implemented method as described above.

Another CRM implementation may include a system including memory and one or more processors operable to execute instructions, stored in the memory, to perform a computer-implemented method as described above.

Each of the features discussed in this particular implementation section for the system implementation apply equally to this CRM implementation. As indicated above, all the system features are not repeated here and should be considered repeated by reference.

A second system implementation of the technology disclosed includes one or more processors coupled to memory. The memory is loaded with computer instructions to identify repeat patterns that cause sequence-specific errors. The system includes an input preparation subsystem that overlays repeat patterns under test at varying offsets on nucleotide sequences to produce overlaid samples. Each of the overlaid samples has a variant nucleotide at a target position flanked by at least 20 nucleotides on each side. The repeat patterns are homopolymers of a single base (A, C, G, or T) with at least 6 repeat factors that specify a number of repetitions of the single base in the repeat patterns. The varying offsets vary a position at which the repeat patterns are overlaid on the nucleotide sequences. The varying offsets are measurable as an offset between an origin position of the repeat patterns and an origin position of the nucleotide sequences. In one implementation, at least ten offsets are used to produce the overlaid samples.

The system further comprises a simulation subsystem that feeds each combination of the repeat patterns overlaid on at least 100 nucleotide sequences in at least 100 overlaid samples to a variant filter for analysis. The system includes a variant filter subsystem that translates analysis by the variant filter into classification scores for likelihood that the variant nucleotide in each of the overlaid samples is a true variant or a false variant. Finally, the system includes an analysis subsystem that causes display of the classification scores as a distribution for each of the repeat factors to support evaluation of sequence-specific error causation by presence of the repeat patterns at the varying offsets.

Other implementations may include a non-transitory computer readable storage medium storing instructions executable by a processor to perform functions of the system described above. Yet another implementation may include a method performing the functions of the system described above.

A second computer-implemented method implementation of the technology disclosed includes identifying repeat patterns that cause sequence-specific errors. The method includes overlaying repeat patterns under test at varying offsets on nucleotide sequences to produce overlaid samples. Each of the overlaid samples has a variant nucleotide at a target position flanked by at least 20 nucleotides on each side. The repeat patterns are homopolymers of a single base (A, C, G, or T) with at least 6 repeat factors that specify a number of repetitions of the single base in the repeat patterns. The varying offsets vary a position at which the repeat patterns are overlaid on the nucleotide sequences. The offset is measurable as an offset between an origin position of the repeat patterns and an origin position of the nucleotide sequences. In one implementation, at least ten offsets are used to produce the overlaid samples.

The computer-implemented method includes feeding each combination of the repeat patterns overlaid on at least 100 nucleotide sequences in at least 100 overlaid samples to a variant filter for analysis. This is followed by translating analysis by the variant filter into classification scores for likelihood that the variant nucleotide in each of the overlaid samples is a true variant or a false variant. Finally, the computer-implemented method causing display of the classification scores as a distribution for each of the repeat factors to support evaluation of sequence-specific error causation by presence of the repeat patterns at the varying offsets.

A computer readable media (CRM) implementation includes a non-transitory computer readable storage medium storing instructions executable by a processor to perform a computer-implemented method as described above. Another CRM implementation may include a system including memory and one or more processors operable to execute instructions, stored in the memory, to perform a computer-implemented method as described above.

A third system implementation of the technology disclosed includes one or more processors coupled to memory. The memory is loaded with computer instructions to identify repeat patterns that cause sequence-specific errors. The system includes an input preparation subsystem, running on numerous processors operating in parallel and coupled to memory, that overlays repeat patterns under test on nucleotide sequences to produce overlaid samples. Each of the overlaid samples has a variant nucleotide at a target position flanked by at least 20 nucleotides on each side. The repeat patterns are copolymers of at least two bases from four bases (A, C, G, and T) with at least 6 repeat factors that specify a number of repetitions of the at least two bases in the repeat patterns. The system includes a simulation subsystem, running on the numerous processors operating in parallel and coupled to the memory, that feeds each combination of the repeat patterns overlaid on at least 100 nucleotide sequences in at least 100 overlaid samples to a variant filter for analysis. The system includes a variant filter subsystem, running on the numerous processors operating in parallel and coupled to the memory. The variant filter subsystem translates analysis by the variant filter into classification scores for likelihood that the variant nucleotide in each of the overlaid samples is a true variant or a false variant. Finally, the system includes an analysis subsystem, running on the numerous processors operating in parallel and coupled to the memory, that causes display of the classification scores as a distribution for each of the repeat factors to support evaluation of sequence-specific error causation by the repeat patterns.

This system implementation and other systems disclosed optionally include one or more of the following features. System can also include features described in connection with methods disclosed. In the interest of conciseness, alternative combinations of system features are not individually enumerated. Features applicable to systems, methods, and articles of manufacture are not repeated for each statutory class set of base features. The reader will understand how features identified in this section can readily be combined with base features in other statutory classes.

The repeat patterns are combinatorial enumeration of copatterns of varying repeat factors and varying pattern lengths.

Other implementations may include a non-transitory computer readable storage medium storing instructions executable by a processor to perform functions of the system described above. Yet another implementation may include a method performing the functions of the system described above.

A third computer-implemented method implementation of the technology disclosed includes identifying repeat patterns that cause sequence-specific errors. The method includes overlaying repeat patterns under test on nucleotide sequences to produce overlaid samples. Each of the overlaid samples has a variant nucleotide at a target position flanked by at least 20 nucleotides on each side. The repeat patterns are copolymers of at least two bases from four bases (A, C, G, and T) with at least 6 repeat factors that specify a number of repetitions of the at least two bases in the repeat patterns. The method includes feedings each combination of the repeat patterns overlaid on at least 100 nucleotide sequences in at least 100 overlaid samples to a variant filter for analysis. The method includes translating analysis by the variant filter into classification scores for likelihood that the variant nucleotide in each of the overlaid samples is a true variant or a false variant. Finally, the method includes causing display of the classification scores as a distribution for each of the repeat factors to support evaluation of sequence-specific error causation by the repeat pattern.

Each of the features discussed in this particular implementation section for the third system implementation apply equally to this computer-implemented method implementation. As indicated above, all the system features are not repeated here and should be considered repeated by reference.

A computer readable media (CRM) implementation includes a non-transitory computer readable storage medium storing instructions executable by a processor to perform a computer-implemented method as described above. Another CRM implementation may include a system including memory and one or more processors operable to execute instructions, stored in the memory, to perform a computer-implemented method as described above.

Each of the features discussed in this particular implementation section for the third system implementation apply equally to this CRM implementation. As indicated above, all the system features are not repeated here and should be considered repeated by reference.

A fourth system implementation of the technology disclosed includes one or more processors coupled to memory. The memory is loaded with computer instructions to identify repeat patterns that cause sequence-specific errors. The system includes an input preparation subsystem, running on numerous processors operating in parallel and coupled to memory, that overlays repeat patterns under test at varying offsets on nucleotide sequences to produce overlaid samples. Each of the overlaid samples has a variant nucleotide at a target position flanked by at least 20 nucleotides on each side. The repeat patterns are copolymers of at least two bases from four bases (A, C, G, and T) with at least 6 repeat factors that specify a number of repetitions of the at least two bases in the repeat patterns. The varying offsets vary a position at which the repeat patterns are overlaid on the nucleotide sequences. The varying offsets are measurable as an offset between an origin position of the repeat patterns and an origin position of the nucleotide sequences. In one implementation, at least ten offsets are used to produce the overlaid samples.

The system includes a simulation subsystem, running on the numerous processors operating in parallel and coupled to the memory, that feeds each combination of the repeat patterns. The repeat patterns are overlaid on at least 100 nucleotide sequences in at least 100 overlaid samples to a variant filter for analysis. The system also includes a variant filter subsystem, running on the numerous processors operating in parallel and coupled to the memory, that translates analysis by the variant filter into classification scores for likelihood that the variant nucleotide in each of the overlaid samples is a true variant or a false variant. Finally, the system includes an analysis subsystem running on the numerous processors operating in parallel and coupled to the memory. The analysis subsystem causes display of the classification scores as a distribution for each of the repeat factors to support evaluation of sequence-specific error causation by presence of the repeat patterns at the varying offsets.

Other implementations may include a non-transitory computer readable storage medium storing instructions executable by a processor to perform functions of the system described above. Yet another implementation may include a method performing the functions of the system described above.

A fourth computer-implemented method implementation of the technology disclosed includes identifying repeat patterns that cause sequence-specific errors. The computer-implemented method includes overlaying repeat patterns under test on nucleotide sequences to produce overlaid samples. Each of the overlaid samples has a variant nucleotide at a target position flanked by at least 20 nucleotides on each side. The repeat patterns are copolymers of at least two bases from four bases (A, C, G, and T) with at least 6 repeat factors. The repeat factors specify a number of repetitions of the at least two bases in the repeat patterns. The varying offsets vary a position at which the repeat patterns are overlaid on the nucleotide sequences. The repeat factors are measurable as an offset between an origin position of the repeat patterns and an origin position of the nucleotide sequences. In one implementation, at least ten offsets are used to produce the overlaid samples. The computer-implemented method includes feeding each combination of the repeat patterns overlaid on at least 100 nucleotide sequences in at least 100 overlaid samples to a variant filter for analysis. The computer-implemented method further includes translating analysis by the variant filter into classification scores for likelihood that the variant nucleotide in each of the overlaid samples is a true variant or a false variant. Finally, the computer-implemented method includes causing display of the classification scores as a distribution for each of the repeat factors to support evaluation of sequence-specific error causation by presence of the repeat patterns at the varying offsets.

A computer readable media (CRM) implementation includes a non-transitory computer readable storage medium storing instructions executable by a processor to perform a computer-implemented method as described above. Another CRM implementation may include a system including memory and one or more processors operable to execute instructions, stored in the memory, to perform a computer-implemented method as described above.

A fifth system implementation of the technology disclosed includes one or more processors coupled to memory. The memory is loaded with computer instructions to identify repeat patterns that cause sequence-specific errors. The system includes an input preparation subsystem running on numerous processors operating in parallel and coupled to memory. The input preparation subsystem selects sample nucleotide sequences from natural DNA nucleotide sequences. Each of the sample nucleotide sequences has one or more naturally occurring repeat patterns of copolymers and a variant nucleotide at a target position flanked by at least 20 nucleotides on each side. The system includes a simulation subsystem running on the numerous processors operating in parallel and coupled to the memory. The simulation subsystem feeds each of the sample nucleotide sequences to a variant filter for analysis.

The system includes a variant filter subsystem running on the numerous processors operating in parallel and coupled to the memory. The variant filter subsystem translates analysis by the variant filter into classification scores for likelihood that the variant nucleotide in each of the sample nucleotide sequences is a true variant or a false variant, and makes available activations of parameters of the variant filter responsive to the analysis. Finally, the system include an analysis subsystem running on the numerous processors operating in parallel and coupled to the memory. The analysis subsystem analyzes the activations of the parameters of the variant filter and causes display of a representation of naturally occurring repeat patterns of copolymers in each of the sample nucleotide sequences that contribute to a false variant classification.

Other implementations may include a non-transitory computer readable storage medium storing instructions executable by a processor to perform functions of the system described above. Yet another implementation may include a method performing the functions of the system described above.

A fifth computer-implemented method implementation of the technology disclosed includes identifying repeat patterns that cause sequence-specific errors. The computer-implemented method includes selecting sample nucleotide sequences from natural DNA nucleotide sequences. Each of the sample nucleotide sequences has one or more naturally occurring repeat patterns of copolymers, and a variant nucleotide at a target position flanked by at least 20 nucleotides on each side. The computer-implemented method includes feeding each of the sample nucleotide sequences to a variant filter for analysis. The method includes translating analysis by the variant filter into classification scores for likelihood that the variant nucleotide in each of the sample nucleotide sequences is a true variant or a false variant. The computer-implemented method makes available activations of parameters of the variant filter responsive to the analysis. Finally, the computer-implemented method includes analyzing the activations of the parameters of the variant filter and causing display of a representation of naturally occurring repeat patterns of copolymers in each of the sample nucleotide sequences that contribute to a false variant classification.

A computer readable media (CRM) implementation includes a non-transitory computer readable storage medium storing instructions executable by a processor to perform a computer-implemented method as described above. Another CRM implementation may include a system including memory and one or more processors operable to execute instructions, stored in the memory, to perform a computer-implemented method as described above.

The technology disclosed presents a system for identifying repeat patterns that cause sequence-specific errors.

The system comprises an input preparation subsystem that runs on numerous processors operating in parallel and coupled to memory. The input preparation subsystem overlays repeat patterns under test on nucleotide sequences to produce overlaid samples. Each of the overlaid samples has a variant nucleotide at a target position flanked by at least 20 nucleotides on each side. The repeat patterns include at least one base from four bases (A, C, G, and T) with at least 6 repeat factors.

The system comprises a simulation subsystem that runs on the numerous processors operating in parallel and coupled to the memory. The simulation subsystem feeds each combination of the repeat patterns overlaid on at least 100 nucleotide sequences in at least 100 overlaid samples to a variant filter for analysis.

The system comprises a variant filter subsystem that runs on the numerous processors operating in parallel and coupled to the memory. The variant filter subsystem translates analysis by the variant filter into classification scores for likelihood that the variant nucleotide in each of the overlaid samples is a true variant or a false variant.

The system comprises an analysis subsystem that runs on the numerous processors operating in parallel and coupled to the memory. The analysis subsystem causes display of the classification scores as a distribution for each of the repeat factors to support evaluation of sequence-specific error causation by the repeat patterns.

Each of the features discussed in this particular implementation section for the first system implementation apply equally to this system implementation. As indicated above, all the system features are not repeated here and should be considered repeated by reference.

In one implementation, the repeat patterns are homopolymers of a single base (A, C, G, or T) with the at least 6 repeat factors that specify a number of repetitions of the single base in the repeat patterns.

In another implementation, the repeat patterns are copolymers of at least two bases from four bases (A, C, G, and T) with the at least 6 repeat factors that specify a number of repetitions of the at least two bases in the repeat patterns.

In some implementations, the input preparation subsystem is further configured to overlay the repeat patterns under test at varying offsets on the nucleotide sequences to produce the overlaid samples. The varying offsets vary a position at which the repeat patterns are overlaid on the nucleotide sequences, measurable as an offset between an origin position of the repeat patterns and an origin position of the nucleotide sequences, and at least ten offsets are used to produce the overlaid samples. In such implementations, the analysis subsystem is further configured to cause display of the classification scores as a distribution for each of the repeat factors to support evaluation of sequence-specific error causation by presence of the repeat patterns at the varying offsets.

In one implementation, the repeat patterns are to right of a center nucleotide in the overlaid samples and not overlapping the center nucleotide. In another implementation, the repeat patterns are to left of a center nucleotide in the overlaid samples and not overlapping the center nucleotide. In another implementation, the repeat patterns include a center nucleotide in the overlaid samples.

The repeat factors are integers in a range of 5 to one-quarter of a count of nucleotides in the overlaid samples. The system is further configured to apply to repeat patterns that are the homopolymers of the single base for each of four bases (A, C, G, and T).

The input preparation subsystem is further configured to produce the repeat patterns and the overlaid samples for the homopolymers for each of the four bases and the analysis subsystem is further configured to cause display of the classification score distribution for each of the homopolymers in juxtaposition.

The repeat patterns are right to a center nucleotide in the overlaid samples and the juxtaposition applies to the homopolymers overlaid right to the center nucleotide. The repeat patterns are left to a center nucleotide in the overlaid samples and the juxtaposition applies to the homopolymers overlaid left to the center nucleotide. The nucleotide sequences on which the repeat patterns are overlaid are randomly generated. The nucleotide sequences on which the repeat patterns are overlaid are randomly selected from naturally occurring DNA nucleotide sequences. The analysis subsystem is further configured to cause display of the classification score distribution for each of the repeat factors using box-and-whisker plots.

The variant filter is trained on at least 500000 training examples of true variants and at least 50000 training examples of false variants. Each training example is a nucleotide sequence with a variant nucleotide at a target position flanked by at least 20 nucleotides on each side. The variant filter is a convolutional neural network (CNN) with two convolutional layers and a fully-connected layer.

The technology disclosed presents a computer-implemented method of identifying repeat patterns that cause sequence-specific errors.

The computer-implemented method includes overlaying repeat patterns under test on nucleotide sequences to produce overlaid samples.

The computer-implemented method includes feeding each combination of the repeat patterns overlaid on at least 100 nucleotide sequences in at least 100 overlaid samples to a variant filter for analysis.

The computer-implemented method includes translating analysis by the variant filter into classification scores for likelihood that the variant nucleotide in each of the overlaid samples is a true variant or a false variant.

The computer-implemented method includes causing display of the classification scores as a distribution for each of the repeat factors to support evaluation of sequence-specific error causation by the repeat patterns.

Each of the features discussed in this particular implementation section for the first system implementation apply equally to this computer-implemented method implementation. As indicated above, all the system features are not repeated here and should be considered repeated by reference.

The technology disclosed presents another system for identifying repeat patterns that cause sequence-specific errors in nucleotide sequencing data. The system comprises one or more processors and one or more storage devices storing instructions that, when executed on the one or more processors cause the one or more processors to implement an input preparation subsystem, a variant filter subsystem, and a repeat pattern output subsystem.

The input preparation subsystem is configured to overlay repeat patterns under test on nucleotide sequences to produce overlaid samples. Each of the overlaid samples has a variant nucleotide and the repeat patterns include at least one base from four bases (A, C, G, and T).

The variant filter subsystem is configured to process each combination of the repeat patterns overlaid on the nucleotide sequences in the overlaid samples to generate classification scores for likelihood that the variant nucleotide in each of the overlaid samples is a true variant or a false variant.

The repeat pattern output subsystem is configured to output particular ones of the repeat patterns that cause sequence-specific errors in the nucleotide sequencing data based on the classification scores.

Each of the features discussed in this particular implementation section for the first system implementation apply equally to this system implementation. As indicated above, all the system features are not repeated here and should be considered repeated by reference.

The system is further configured to comprise an analysis subsystem that is configured to cause display of the classification scores as a distribution for each of the repeat factors to support evaluation of sequence-specific error causation by the repeat patterns.

A computer readable media (CRM) implementation includes a non-transitory computer readable storage medium storing instructions executable by a processor to perform a computer-implemented method as described above. Another CRM implementation may include a system including memory and one or more processors operable to execute instructions, stored in the memory, to perform a computer-implemented method as described above.

The technology disclosed presents another system for identifying repeat patterns that cause sequence-specific errors in nucleotide sequencing data. The system comprises one or more processors and one or more storage devices storing instructions that, when executed on the one or more processors cause the one or more processors to implement an input preparation subsystem, a variant filter subsystem, and a repeat pattern output subsystem.

The input preparation subsystem is configured to overlay repeat patterns under test on nucleotide sequences to produce overlaid samples. Each of the overlaid samples has a variant nucleotide and the repeat patterns include at least one base from four bases (A, C, G, and T).

The variant filter subsystem is configured to process each combination of the repeat patterns overlaid on the nucleotide sequences in the overlaid samples to generate classification scores for likelihood that the variant nucleotide in each of the overlaid samples is a true variant or a false variant.

The repeat pattern output subsystem is configured to output particular ones of the repeat patterns that cause sequence-specific errors in the nucleotide sequencing data based on the classification scores.

Each of the features discussed in this particular implementation section for the first system implementation apply equally to this system implementation. As indicated above, all the system features are not repeated here and should be considered repeated by reference.

The system is further configured to comprise an analysis subsystem that is configured to cause display of the classification scores as a distribution for each of the repeat factors to support evaluation of sequence-specific error causation by the repeat patterns.

The technology disclosed presents a computer-implemented method of identifying repeat patterns that cause sequence-specific errors in nucleotide sequencing data.

The computer-implemented method includes overlaying repeat patterns under test on nucleotide sequences to produce overlaid samples. Each of the overlaid samples has a variant nucleotide and the repeat patterns include at least one base from four bases (A, C, G, and T).

The computer-implemented method includes processing each combination of the repeat patterns overlaid on the nucleotide sequences in the overlaid samples through a variant filter subsystem to generate classification scores for likelihood that the variant nucleotide in each of the overlaid samples is a true variant or a false variant.

The computer-implemented method includes translating analysis by the variant filter into classification scores for likelihood that the variant nucleotide in each of the overlaid samples is a true variant or a false variant.

The computer-implemented method includes outputting particular ones of the repeat patterns that cause sequence-specific errors in the nucleotide sequencing data based on the classification scores.

Each of the features discussed in this particular implementation section for the first system implementation apply equally to this computer-implemented method implementation. As indicated above, all the system features are not repeated here and should be considered repeated by reference.

A computer readable media (CRM) implementation includes a non-transitory computer readable storage medium storing instructions executable by a processor to perform a computer-implemented method as described above. Another CRM implementation may include a system including memory and one or more processors operable to execute instructions, stored in the memory, to perform a computer-implemented method as described above.

The technology disclosed presents another system for identifying repeat patterns that cause sequence-specific errors in nucleotide sequencing data. The system comprises one or more processors and one or more storage devices storing instructions that, when executed on the one or more processors cause the one or more processors to implement an input preparation subsystem, a variant filter subsystem, and a repeat pattern output subsystem.

The input preparation subsystem is configured to select sample nucleotide sequences from natural DNA nucleotide sequences. Each of the sample nucleotide sequences has one or more naturally occurring repeat patterns of copolymers and a variant nucleotide.

The variant filter subsystem is configured to process each of the sample nucleotide sequences to generate classification scores for likelihood that the variant nucleotide in each of the sample nucleotide sequences is a true variant or a false variant.

The repeat pattern output subsystem is configured to make available activations of parameters of the variant filter subsystem responsive to the analysis and output particular ones of the repeat patterns that cause sequence-specific errors in the nucleotide sequencing data based upon the classification scores.

Each of the features discussed in this particular implementation section for the first system implementation apply equally to this system implementation. As indicated above, all the system features are not repeated here and should be considered repeated by reference.

The system is further configured to comprise an analysis subsystem that is configured to analyze the activations of the parameters of the variant filter subsystem and cause display of a representation of naturally occurring repeat patterns of copolymers in each of the sample nucleotide sequences that contribute to a false variant classification.

The technology disclosed presents a computer-implemented method of identifying repeat patterns that cause sequence-specific errors in nucleotide sequencing data.

The computer-implemented method includes selecting sample nucleotide sequences from natural DNA nucleotide sequences. Each of the sample nucleotide sequences has one or more naturally occurring repeat patterns of copolymers and a variant nucleotide.

The computer-implemented method includes processing each of the sample nucleotide sequences through a variant filter subsystem to generate classification scores for likelihood that the variant nucleotide in each of the sample nucleotide sequences is a true variant or a false variant.

The computer-implemented method includes making available activations of parameters of the variant filter subsystem responsive to the analysis.

The computer-implemented method includes outputting particular ones of the repeat patterns that cause sequence-specific errors in the nucleotide sequencing data based upon the classification scores.

Each of the features discussed in this particular implementation section for the first system implementation apply equally to this computer-implemented method implementation. As indicated above, all the system features are not repeated here and should be considered repeated by reference.

A computer readable media (CRM) implementation includes a non-transitory computer readable storage medium storing instructions executable by a processor to perform a computer-implemented method as described above. Another CRM implementation may include a system including memory and one or more processors operable to execute instructions, stored in the memory, to perform a computer-implemented method as described above.

Any data structures and code described or referenced above are stored according to many implementations on a computer-readable storage medium, which may be any device or medium that can store code and/or data for use by a computer system. This includes, but is not limited to, volatile memory, non-volatile memory, application-specific integrated circuits (ASICs), field-programmable gate arrays (FPGAs), magnetic and optical storage devices such as disk drives, magnetic tape, CDs (compact discs), DVDs (digital versatile discs or digital video discs), or other media capable of storing computer-readable media now known or later developed.

The preceding description is presented to enable the making and use of the technology disclosed. The scope of the technology disclosed is defined by the appended claims.

**Claims**

1. A system for identifying repeat patterns that cause sequence-specific errors in nucleotide sequencing data, comprising:

   one or more processors and one or more storage devices storing instructions that, when executed on the one or more processors cause the one or more processors to implement:

   an input preparation subsystem configured to:

   computationally overlay repeat patterns under test on numerous nucleotide sequences and produce overlaid samples,
   wherein each repeat pattern represents a particular nucleotide composition that has a particular length and appears in an overlaid sample at a particular offset position,
   wherein each overlaid sample has a target position considered to be a variant nucleotide, and
   wherein for each combination of the particular nucleotide composition, the particular length, and the particular offset position, a set of the overlaid samples is computationally generated;

a pre-trained variant filter subsystem configured to:
process the overlaid samples through a convolutional neural network and, based on detection of nucleotide patterns in the overlaid samples by convolution filters of the convolutional neural network, generate classification scores for likelihood that the variant nucleotide in each of the overlaid samples is a true variant or a false variant;
a repeat pattern output subsystem configured to:
output distributions of the classification scores generated by the pre-trained variant filter subsystem for the repeat factors that specify a number of repetitions of a single base (A, C, G, and T) or at least two bases in the repeat patterns; and
a sequence-specific error correlation subsystem configured to:

specify, based on a threshold, a subset of the classification scores in the distributions as indicative of false variant classifications, and
classify those repeat patterns which are associated with the subset of the classification scores that are indicative of the false variant classifications as causing the sequence-specific errors.

2. The system of claim 1, wherein the sequence-specific error correlation subsystem is further configured to:
classify lengths and offset positions of the repeat patterns which are associated with the subset of the classification scores as causing the sequence-specific errors.

3. The system of any of claims 1-2, wherein the variant nucleotide is at the target position flanked by at least 20 nucleotides on each side.

4. The system of any of claims 1-3, wherein the pre-trained variant filter subsystem is configured to process each combination of the repeat patterns overlaid on at least 100 nucleotide sequences in at least 100 overlaid samples.

5. The system of any of claims 1-4, wherein the repeat patterns include the at least one base from four bases (A, C, G, and T) with at least 6 repeat factors.

6. The system of claim 5, wherein the repeat patterns are homopolymers of a single base (A, C, G, or T) with the at least 6 repeat factors.

7. The system of any of claims 1-6, wherein the repeat patterns are copolymers of at least two bases from four bases (A, C, G, and T) with the at least 6 repeat factors.

8. The system of any of claims 1-7, wherein the offset positions vary in terms of a position at which the repeat patterns are overlaid on the nucleotide sequences, measurable as an offset between an origin position of the repeat patterns and an origin position of the nucleotide sequences, and at least ten offsets are used to produce the overlaid samples.

9. The system of any of claims 1-8, wherein the repeat patterns are right of a center nucleotide in the overlaid samples and not overlapping the center nucleotide.

10. The system of any of claims 1-8, wherein the repeat patterns are left of a center nucleotide in the overlaid samples and not overlapping the center nucleotide.

11. The system of any of claims 1-8, wherein the repeat patterns are overlaid on a center nucleotide in the overlaid samples.

12. The system of any of claims 1-11, wherein the repeat factors are integers in a range of five to one-quarter of a count of nucleotides in the overlaid samples.

13. The system of claim 6, further configured to apply to repeat patterns that are the homopolymers of the single base for each of four bases (A, C, G, and T).

14. The system of claim 13, wherein the input preparation subsystem is further configured to produce the repeat patterns and the overlaid samples for the homopolymers for each of the four bases.

15. The system of claim 14, wherein the repeat patterns are positioned right of a center nucleotide in the overlaid samples and the classification score distributions for each of the homopolymers are displayed in juxtaposition.

16. The system of claim 14, wherein the repeat patterns are positioned left of a center nucleotide in the overlaid samples and the classification score distributions for each of the homopolymers are displayed in juxtaposition.

17. The system of any of claims 1-16, wherein the nucleotide sequences on which the repeat patterns are overlaid are randomly generated.

18. The system of any of claims 1-17, wherein the nucleotide sequences on which the repeat patterns are overlaid are randomly selected from naturally occurring DNA nucleotide sequences.

19. The system of any of claims 1-18, wherein an analysis subsystem is configured to cause display of the distributions of the classification scores for each of the repeat factors.

20. The system of any of claims 1-19, wherein the pre-trained variant filter subsystem is trained on at least 500000 training examples of true variants and at least 50000 training examples of false variants; and
wherein each training example is a nucleotide sequence with a variant nucleotide at a target position flanked by at least 20 nucleotides on each side.

21. The system of any of claims 1-20, wherein the pre-trained variant filter subsystem has convolutional layers, a fully-connected layer, and a classification layer.

22. A computer-implemented method of identifying repeat patterns that cause sequence-specific errors in nucleotide sequencing data, including:

computationally overlaying repeat patterns under test on numerous nucleotide sequences and producing overlaid samples, wherein each repeat pattern represents a particular nucleotide composition that has a particular length and appears in an overlaid sample at a particular offset position, wherein each overlaid sample has a target position considered to be a variant nucleotide, and wherein for each combination of the particular nucleotide composition, the particular length, and the particular offset position, a set of the overlaid samples is computationally generated;
processing the overlaid samples through a convolutional neural network and, based on detection of nucleotide patterns in the overlaid samples by convolution filters of the convolutional neural network, generating classification scores for likelihood that the variant nucleotide in each of the overlaid samples is a true variant or a false variant;
outputting distributions of the classification scores generated by the pre-trained variant filter subsystem for the repeat factors that specify a number of repetitions of a single base (A, C, G, and T) or at least two bases in the repeat patterns; and
specifying, based on a threshold, a subset of the classification scores in the distributions as indicative of false variant classifications and classifying those repeat patterns which are associated with the subset of the classification scores that are indicative of the false variant classifications as causing the sequence-specific errors.

23. The computer-implemented method of claim 22, further implementing any of claims 1-21, by exercising the systems claimed.

24. A non-transitory computer readable storage medium impressed with computer program instructions to identify repeat patterns that cause sequence-specific errors in nucleotide sequencing data, the instructions, when executed on a processor, implement a computer-implemented method comprising:

computationally overlaying repeat patterns under test on numerous nucleotide sequences and producing overlaid samples, wherein each repeat pattern represents a particular nucleotide composition that has a particular length and appears in an overlaid sample at a particular offset position, wherein each overlaid sample has a target position considered to be a variant nucleotide, and wherein for each combination of the particular nucleotide composition, the particular length, and the particular offset position, a set of the overlaid samples is computationally generated;
processing the overlaid samples through a convolutional neural network and, based on detection of nucleotide patterns in the overlaid samples by convolution filters of the convolutional neural network, generating classification scores for likelihood that the variant nucleotide in each of the overlaid samples is a true variant or a false variant;
outputting distributions of the classification scores generated by the pre-trained variant filter subsystem for the repeat factors that specify a number of repetitions of a single base (A, C, G, and T) or at least two bases in the repeat patterns; and

specifying, based on a threshold, a subset of the classification scores in the distributions as indicative of false variant classifications and classifying those repeat patterns which are associated with the subset of the classification scores that are indicative of the false variant classifications as causing the sequence-specific errors.

25. The non-transitory computer readable storage medium of claim 24, further including features that, when combined with hardware, implement any of claims 1-21.

**Patentansprüche**

1. System zum Identifizieren von Wiederholungsmustern, die sequenzspezifische Fehler in Nukleotidsequenzierungsdaten verursachen, Folgendes umfassend:

einen oder mehrere Prozessoren und eine oder mehrere Speichervorrichtungen, die Anweisungen speichern, die, wenn sie auf dem einen oder den mehreren Prozessoren ausgeführt werden, den einen oder die mehreren Prozessoren veranlassen, Folgendes zu implementieren:
ein Eingabevorbereitung-Subsystem, das dafür konfiguriert ist:

zu prüfende Wiederholungsmuster auf zahlreichen Nukleotidsequenzen rechnerisch zu überlagern und überlagerte Proben zu erzeugen,
wobei jedes Wiederholungsmuster eine bestimmte Nukleotidzusammensetzung darstellt, die eine bestimmte Länge aufweist und in einer überlagerten Probe an einer bestimmten Versatz-Position erscheint,
wobei jede überlagerte Probe eine Zielposition aufweist, die als eine Nukleotidvariante angesehen wird, und
wobei für jede Kombination aus der bestimmten Nukleotidzusammensetzung, der bestimmten Länge und der bestimmten Versatz-Position rechnerisch eine Menge von überlagerten Proben erzeugt wird;
ein vortrainiertes Variantenfilter-Subsystem, das dafür konfiguriert ist:

die überlagerten Proben durch ein faltendes neuronales Netz zu verarbeiten und, auf der Grundlage der Ermittlung von Nukleotidmustern in den überlagerten Proben durch Faltungsfilter des faltenden neuronalen Netzes. Klassifizierungspunktzahlen für eine Wahrscheinlichkeit zu erzeugen, dass die Nukleotidvariante in jeder der überlagerten Proben eine echte Variante oder eine falsche Variante ist;
ein Wiederholungsmusterausgabe-Subsystem, das dafür konfiguriert ist:

Verteilungen der durch das vortrainierte Variantenfilter-Subsystem erzeugten Klassifikationspunktzahlen für diejenigen Wiederholungsfaktoren auszugeben, die eine Anzahl von Wiederholungen einer einzelnen Base (A, C, G und T) oder mindestens zweier Basen in den Wiederholungsmustern angeben; und
ein Subsystem für sequenzspezifische Fehlerkorrelation, das dafür konfiguriert ist:

auf der Grundlage eines Schwellenwerts eine Teilmenge der Klassifikationspunktzahlen in den Verteilungen als bezeichnend für falsche Variantenklassifizierungen anzugeben, und
diejenigen Wiederholungsmuster, die der Teilmenge der Klassifizierungspunktzahlen zugeordnet sind, die bezeichnend für die falschen Variantenklassifizierungen sind, als Ursache der sequenzspezifischen Fehler zu klassifizieren.

2. System nach Anspruch 1, wobei das Subsystem für sequenzspezifische Fehlerkorrelation ferner dafür konfiguriert ist: Längen und Versatz-Positionen der Wiederholungsmuster, die der Teilmenge der Klassifizierungspunktzahlen zugeordnet sind, als Ursache der sequenzspezifischen Fehler zu klassifizieren.

3. System nach einem der Ansprüche 1 bis 2, wobei sich die Nukleotidvariante an der Zielposition befindet, flankiert von mindestens 20 Nukleotiden auf jeder Seite.

4. System nach einem der Ansprüche 1 bis 3, wobei das vortrainierte Variantenfilter-Subsystem dafür konfiguriert ist, jede Kombination der Wiederholungsmuster zu verarbeiten, die an mindestens 100 Nukleotidsequenzen in mindestens 100 überlagerten Proben überlagert wird.

5. System nach einem der Ansprüche 1 bis 4, wobei die Wiederholungsmuster die mindestens eine Base von vier Basen (A, C, G und T) mit mindestens 6 Wiederholungsfaktoren einschließen.

6. System nach Anspruch 5, wobei die Wiederholungsmuster Homopolymere einer einzelnen Base (A, C, G oder T) mit den mindestens 6 Wiederholungsfaktoren sind.

7. System nach einem der Ansprüche 1 bis 6, wobei die Wiederholungsmuster Copolymere von mindestens zwei Basen von vier Basen (A, C, G und T) mit den mindestens 6 Wiederholungsfaktoren sind.

8. System nach einem der Ansprüche 1 bis 7, wobei die Versatz-Positionen in Bezug auf eine Position variieren, an der die Wiederholungsmuster an den Nukleotidsequenzen überlagert werden, messbar als ein Versatz zwischen einer Ursprungsposition der Wiederholungsmuster und einer Ursprungsposition der Nukleotidsequenzen, und mindestens zehn Versätze verwendet werden, um die überlagerten Proben zu erzeugen.

9. System nach einem der Ansprüche 1 bis 8, wobei die Wiederholungsmuster rechts von einem zentralen Nukleotid in den überlagerten Proben liegen und das zentrale Nukleotid nicht überlappen.

10. System nach einem der Ansprüche 1 bis 8, wobei die Wiederholungsmuster links von einem zentralen Nukleotid in den überlagerten Proben liegen und das zentrale Nukleotid nicht überlappen.

11. System nach einem der Ansprüche 1 bis 8, wobei die Wiederholungsmuster an einem zentralen Nukleotid in den überlagerten Proben überlagert sind.

12. System nach einem der Ansprüche 1 bis 11, wobei die Wiederholungsfaktoren ganze Zahlen in einem Bereich von fünf bis zu einem Viertel einer Nukleotidanzahl in den überlagerten Proben sind.

13. System nach Anspruch 6, ferner dafür konfiguriert, auf Wiederholungsmuster angewendet zu werden, die Homopolymere der einzelnen Base für jede von vier Basen (A, C, G und T) sind.

14. System nach Anspruch 13, wobei das Eingabevorbereitung-Subsystem ferner dafür konfiguriert ist, die Wiederholungsmuster und die überlagerten Proben für die Homopolymere für jede der vier Basen zu erzeugen.

15. System nach Anspruch 14, wobei die Wiederholungsmuster in den überlagerten Proben rechts von einem zentralen Nukleotid positioniert sind und die Verteilungen der Klassifikationspunktzahlen für jedes der Homopolymere nebeneinander angezeigt werden.

16. System nach Anspruch 14, wobei die Wiederholungsmuster in den überlagerten Proben links von einem zentralen Nukleotid positioniert sind und die Verteilungen der Klassifizierungspunktzahlen für jedes der Homopolymere nebeneinander angezeigt werden.

17. System nach einem der Ansprüche 1 bis 16, wobei die Nukleotidsequenzen, an denen die Wiederholungsmuster überlagert sind, zufällig erzeugt werden.

18. System nach einem der Ansprüche 1 bis 17, wobei die Nukleotidsequenzen, an denen die Wiederholungsmuster überlagert sind, zufällig aus natürlich vorkommenden DNA-Nukleotidsequenzen ausgewählt werden.

19. System nach einem der Ansprüche 1 bis 18, wobei ein Analyse-Subsystem dafür konfiguriert ist, eine Anzeige der Verteilungen der Klassifizierungspunktzahlen für jeden der Wiederholungsfaktoren zu veranlassen.

20. System nach einem der Ansprüche 1 bis 19, wobei das vortrainierte Variantenfilter-Subsystem auf mindestens 500 000 Trainingsbeispielen von echten Varianten und auf mindestens 50 000 Trainingsbeispielen von falschen Varianten trainiert ist; und
wobei jedes Trainingsbeispiel eine Nukleotidsequenz mit einer Nukleotidvariante an einer Zielposition ist, die von mindestens 20 Nukleotiden auf jeder Seite flankiert wird.

21. System nach einem der Ansprüche 1 bis 20, wobei das vortrainierte Variantenfilter-Subsystem Faltungsschichten, eine vollständig verbundene Schicht und eine Klassifizierungsschicht aufweist.

22. Computerimplementiertes Verfahren zum Identifizieren von Wiederholungsmustern, die sequenzspezifische Fehler in Nukleotidsequenzierungsdaten verursachen, Folgendes einschließend:

rechnerisches Überlagern von zu prüfenden Wiederholungsmustern auf zahlreichen Nukleotidsequenzen und Erzeugen von überlagerten Proben, wobei jedes Wiederholungsmuster eine bestimmte Nukleotidzusammensetzung darstellt, die eine bestimmte Länge aufweist und in einer überlagerten Probe an einer bestimmten Versatz-Position erscheint, wobei jede überlagerte Probe eine Zielposition aufweist, die als eine Nukleotidvariante angesehen wird, und wobei für jede Kombination aus der bestimmten Nukleotidzusammensetzung, der bestimmten Länge und der bestimmten Versatz-Position rechnerisch eine Menge von überlagerten Proben erzeugt wird;

Verarbeiten der überlagerten Proben durch ein faltendes neuronales Netz und auf der Ermittlung von Nukleotidmustern in den überlagerten Proben durch Faltungsfilter des faltenden neuronalen Netzes basierendes Erzeugen von Klassifizierungspunktzahlen für eine Wahrscheinlichkeit, dass die Nukleotidvariante in jeder der überlagerten Proben eine echte Variante oder eine falsche Variante ist;

Ausgeben von Verteilungen der durch das vortrainierte Variantenfilter-Subsystem erzeugten Klassifizierungspunktzahlen für diejenigen Wiederholungsfaktoren, die eine Anzahl von Wiederholungen einer einzelnen Base (A, C, G und T) oder mindestens zweier Basen in den Wiederholungsmustern angeben; und

auf einem Schwellenwert basierendes Angeben einer Teilmenge der Klassifizierungspunktzahlen in den Verteilungen als bezeichnend für falsche Variantenklassifizierungen und Klassifizieren derjenigen Wiederholungsmuster, die der Teilmenge der Klassifizierungspunktzahlen zugeordnet sind, die bezeichnend für die falschen Variantenklassifizierungen als Ursache der sequenzspezifischen Fehler ist.

23. Computerimplementiertes Verfahren nach Anspruch 22, das ferner einen der Ansprüche 1 bis 21 implementiert.

24. Nichtflüchtiges computerlesbares Speichermedium, dem Computerprogrammanweisungen aufgeprägt sind, um Wiederholungsmuster zu identifizieren, die sequenzspezifische Fehler in Nukleotidsequenzierungsdaten verursachen, wobei die Anweisungen, wenn sie auf einem Prozessor ausgeführt werden, ein computerimplementiertes Verfahren implementieren, welches Folgendes umfasst:

rechnerisches Überlagern von zu prüfenden Wiederholungsmustern auf zahlreichen Nukleotidsequenzen und Erzeugen von überlagerten Proben, wobei jedes Wiederholungsmuster eine bestimmte Nukleotidzusammensetzung darstellt, die eine bestimmte Länge aufweist und in einer überlagerten Probe an einer bestimmten Versatz-Position erscheint, wobei jede überlagerte Probe eine Zielposition aufweist, die als eine Nukleotidvariante angesehen wird, und wobei für jede Kombination der bestimmten Nukleotidzusammensetzung, der bestimmten Länge und der bestimmten Versatz-Position rechnerisch eine Menge von überlagerten Proben erzeugt wird;

Verarbeiten der überlagerten Proben durch ein faltendes neuronales Netz und auf der Ermittlung von Nukleotidmustern in den überlagerten Proben durch Faltungsfilter des faltenden neuronalen Netzes basierendes Erzeugen von Klassifizierungspunktzahlen für eine Wahrscheinlichkeit, dass die Nukleotidvariante in jeder der überlagerten Proben eine echte Variante oder eine falsche Variante ist;

Ausgeben von Verteilungen der durch das vortrainierte Variantenfilter-Subsystem erzeugten Klassifizierungspunktzahlen für diejenigen Wiederholungsfaktoren, die eine Anzahl von Wiederholungen einer einzelnen Base (A, C, G und T) oder mindestens zweier Basen in den Wiederholungsmustern angeben; und

auf einem Schwellenwert basierendes Angeben einer Teilmenge der Klassifizierungspunktzahlen in den Verteilungen als bezeichnend für falsche Variantenklassifizierungen und Klassifizieren derjenigen Wiederholungsmuster, die der Teilmenge der Klassifizierungspunktzahlen zugeordnet sind, die bezeichnend für die falschen Variantenklassifizierungen ist, als Ursache der sequenzspezifischen Fehler.

25. Nichtflüchtiges, computerlesbares Speichermedium nach Anspruch 24, das ferner Merkmale einschließt, die, wenn sie mit Hardware kombiniert werden, einen der Ansprüche 1 bis 21 implementieren.

**Revendications**

1. Système pour identifier des motifs de répétition qui causent des erreurs spécifiques à la séquence dans des données de séquençage de nucléotides, comprenant :
un ou plusieurs processeurs et un ou plusieurs dispositifs de stockage stockant des instructions qui, lorsqu'elles sont exécutées sur l'un ou les plusieurs processeurs, amènent l'un ou les plusieurs processeurs à mettre en oeuvre :
un sous-système de préparation d'entrée configuré pour :

superposer par informatique des motifs de répétition sous test sur de nombreuses séquences nucléotidiques et produire des échantillons superposés,

dans lequel chaque motif de répétition représente une composition nucléotidique particulière qui présente une longueur particulière et qui apparaît dans un échantillon superposé au niveau d'une position décalée particulière,

dans lequel chaque échantillon superposé présente une position cible considérée comme étant un nucléotide variant, et

dans lequel, pour chaque combinaison de la composition nucléotidique particulière, de la longueur particulière et de la position décalée particulière, un ensemble des échantillons superposés est généré par informatique ; un sous-système de filtres de variants pré-soumis à apprentissage configuré pour :

traiter les échantillons superposés au travers d'un réseau neural à convolution et, sur la base de la détection de motifs nucléotidiques dans les échantillons superposés par des filtres de convolution du réseau neural à convolution, générer des scores de classification pour une probabilité que le nucléotide variant dans chacun des échantillons superposés soit un variant vrai ou un variant faux ;

un sous-système d'émission en sortie de motifs de répétition configuré pour :

émettre en sortie des distributions des scores de classification générés par le sous-système de filtres de variants pré-soumis à apprentissage pour les facteurs de répétition qui spécifient un nombre de répétitions d'une unique base (A, C, G et T) ou d'au moins deux bases dans les motifs de répétition ; et un sous-système de corrélation d'erreurs spécifiques à la séquence configuré pour :

spécifier, sur la base d'un seuil, un sous-ensemble des scores de classification dans les distributions comme étant indicatifs de classifications de variant faux, et

classifier ces motifs de répétition qui sont associés au sous-ensemble des scores de classification qui sont indicatifs des classifications de variant faux comme causant les erreurs spécifiques à la séquence.

2. Système selon la revendication 1, dans lequel le sous-système de corrélation d'erreurs spécifiques à la séquence est en outre configuré pour :
classifier des longueurs et des positions décalées des motifs de répétition qui sont associés au sous-ensemble des scores de classification comme causant les erreurs spécifiques à la séquence.

3. Système selon l'une quelconque des revendications 1 à 2, dans lequel le nucléotide variant est au niveau de la position cible flanqué par au moins 20 nucléotides sur chaque côté.

4. Système selon l'une quelconque des revendications 1 à 3, dans lequel le sous-système de filtres de variants pré-soumis à apprentissage est configuré pour traiter chaque combinaison des motifs de répétition superposés sur au moins 100 séquences nucléotidiques dans au moins 100 échantillons superposés.

5. Système selon l'une quelconque des revendications 1 à 4, dans lequel les motifs de répétition incluent l'au moins une base parmi quatre bases (A, C, G et T) avec au moins 6 facteurs de répétition.

6. Système selon la revendication 5, dans lequel les motifs de répétition sont des homopolymères d'une unique base (A, C, G ou T) avec les au moins 6 facteurs de répétition.

7. Système selon l'une quelconque des revendications 1 à 6, dans lequel les motifs de répétition sont des copolymères d'au moins deux bases parmi quatre bases (A, C, G et T) avec les au moins 6 facteurs de répétition.

8. Système selon l'une quelconque des revendications 1 à 7, dans lequel les positions décalées varient en termes d'une position au niveau de laquelle les motifs de répétition sont superposés sur les séquences nucléotidiques, mesurable en tant que décalage entre une position d'origine des motifs de répétition et une position d'origine des séquences nucléotidiques, et au moins dix décalages sont utilisés pour produire les échantillons superposés.

9. Système selon l'une quelconque des revendications 1 à 8, dans lequel les motifs de répétition sont à droite d'un nucléotide central dans les échantillons superposés et ne chevauchent pas le nucléotide central.

10. Système selon l'une quelconque des revendications 1 à 8, dans lequel les motifs de répétition sont à gauche d'un nucléotide central dans les échantillons superposés et ne chevauchent pas le nucléotide central.

**11.** Système selon l'une quelconque des revendications 1 à 8, dans lequel les motifs de répétition sont superposés sur un nucléotide central dans les échantillons superposés.

**12.** Système selon l'une quelconque des revendications 1 à 11, dans lequel les facteurs de répétition sont des nombres entiers dans une plage de cinq à un quart d'un comptage de nucléotides dans les échantillons superposés.

**13.** Système selon la revendication 6, configuré en outre pour être appliqué à des motifs de répétition qui sont les homopolymères de l'unique base pour chacune de quatre bases (A, C, G et T).

**14.** Système selon la revendication 13, dans lequel le sous-système de préparation d'entrée est en outre configuré pour produire les motifs de répétition et les échantillons superposés pour les homopolymères pour chacune des quatre bases.

**15.** Système selon la revendication 14, dans lequel les motifs de répétition sont positionnés à droite d'un nucléotide central dans les échantillons superposés et les distributions de scores de classification pour chacun des homopolymères sont affichées en juxtaposition.

**16.** Système selon la revendication 14, dans lequel les motifs de répétition sont positionnés à gauche d'un nucléotide central dans les échantillons superposés et les distributions de scores de classification pour chacun des homopolymères sont affichées en juxtaposition.

**17.** Système selon l'une quelconque des revendications 1 à 16, dans lequel les séquences nucléotidiques sur lesquelles les motifs de répétition sont superposés sont générées de façon aléatoire.

**18.** Système selon l'une quelconque des revendications 1 à 17, dans lequel les séquences nucléotidiques sur lesquelles les motifs de répétition sont superposés sont sélectionnées de façon aléatoire à partir de séquences nucléotidiques d'ADN survenant naturellement.

**19.** Système selon l'une quelconque des revendications 1 à 18, dans lequel un sous-système d'analyse est configuré pour provoquer l'affichage des distributions des scores de classification pour chacun des facteurs de répétition.

**20.** Système selon l'une quelconque des revendications 1 à 19, dans lequel le sous-système de filtres de variants présoumis à apprentissage est soumis à apprentissage sur au moins 500 000 exemples d'apprentissage de variants vrais et au moins 50 000 exemples d'apprentissage de variants faux ; et
dans lequel chaque exemple d'apprentissage est une séquence nucléotidique avec un nucléotide variant en une position cible flanqué par au moins 20 nucléotides sur chaque côté.

**21.** Système selon l'une quelconque des revendications 1 à 20, dans lequel le sous-système de filtres de variants présoumis à apprentissage comporte des couches de convolution, une couche intégralement connectée et une couche de classification.

**22.** Procédé mis en oeuvre par ordinateur d'identification de motifs de répétition qui causent des erreurs spécifiques à la séquence dans des données de séquençage de nucléotides, incluant :

la superposition par informatique de motifs de répétition sous test sur de nombreuses séquences nucléotidiques et la production d'échantillons superposés, dans lequel chaque motif de répétition représente une composition nucléotidique particulière qui présente une longueur particulière et qui apparaît dans un échantillon superposé au niveau d'une position décalée particulière, dans lequel chaque échantillon superposé présente une position cible considérée comme étant un nucléotide variant, et dans lequel, pour chaque combinaison de la composition nucléotidique particulière, de la longueur particulière et de la position décalée particulière, un ensemble des échantillons superposés est généré par informatique ;
le traitement des échantillons superposés au travers d'un réseau neural à convolution et, sur la base de la détection de motifs nucléotidiques dans les échantillons superposés par des filtres de convolution du réseau neural à convolution, la génération de scores de classification pour une probabilité que le nucléotide variant dans chacun des échantillons superposés soit un variant vrai ou un variant faux ;
l'émission en sortie de distributions des scores de classification générés par le sous-système de filtres de variants pré-soumis à apprentissage pour les facteurs de répétition qui spécifient un nombre de répétitions d'une unique base (A, C, G et T) ou d'au moins deux bases dans les motifs de répétition ; et

la spécification, sur la base d'un seuil, d'un sous-ensemble des scores de classification dans les distributions comme étant indicatifs de classifications de variant faux et la classification de ces motifs de répétition qui sont associés au sous-ensemble des scores de classification qui sont indicatifs des classifications de variant faux comme causant les erreurs spécifiques à la séquence.

**23.** Procédé mis en oeuvre par ordinateur selon la revendication 22, mettant en outre en oeuvre l'une quelconque des revendications 1 à 21.

**24.** Support de stockage lisible par ordinateur non transitoire sur lequel sont apposées des instructions de programme informatique pour identifier des motifs de répétition qui causent des erreurs spécifiques à la séquence dans des données de séquençage de nucléotides, les instructions, lorsqu'elles sont exécutées sur un processeur, mettent en oeuvre un procédé mis en oeuvre par ordinateur comprenant :

la superposition par informatique de motifs de répétition sous test sur de nombreuses séquences nucléotidiques et la production d'échantillons superposés, dans lequel chaque motif de répétition représente une composition nucléotidique particulière qui présente une longueur particulière et qui apparaît dans un échantillon superposé au niveau d'une position décalée particulière, dans lequel chaque échantillon superposé présente une position cible considérée comme étant un nucléotide variant, et dans lequel, pour chaque combinaison de la composition nucléotidique particulière, de la longueur particulière et de la position décalée particulière, un ensemble des échantillons superposés est généré par informatique ;

le traitement des échantillons superposés au travers d'un réseau neural à convolution et, sur la base de la détection de motifs nucléotidiques dans les échantillons superposés par des filtres de convolution du réseau neural à convolution, la génération de scores de classification pour une probabilité que le nucléotide variant dans chacun des échantillons superposés soit un variant vrai ou un variant faux ;

l'émission en sortie de distributions des scores de classification générés par le sous-système de filtres de variants pré-soumis à apprentissage pour les facteurs de répétition qui spécifient un nombre de répétitions d'une unique base (A, C, G et T) ou d'au moins deux bases dans les motifs de répétition ; et

la spécification, sur la base d'un seuil, d'un sous-ensemble des scores de classification dans les distributions comme étant indicatifs de classifications de variant faux et la classification de ces motifs de répétition qui sont associés au sous-ensemble des scores de classification qui sont indicatifs des classifications de variant faux comme causant les erreurs spécifiques à la séquence.

**25.** Support de stockage lisible par ordinateur non transitoire selon la revendication 24, incluant en outre des caractéristiques qui, lorsqu'elles sont combinées avec du matériel informatique, mettent en oeuvre l'une quelconque des revendications 1 à 21.

**FIG. 1**

**FIG. 2**

EP 3 619 712 B1

300

```
┌─────────────────────────────────────┐
│   Overlaid Sample (101, 5) 302       │
└─────────────────────────────────────┘
                 │
                 ▼
┌─────────────────────────────────────┐
│  Convolution Layer 1 (64, 3, 1) 304  │
└─────────────────────────────────────┘
                 │
                 ▼
┌─────────────────────────────────────┐
│   Batch Normalization & ReLU 306     │
└─────────────────────────────────────┘
                 │
                 ▼
┌─────────────────────────────────────┐
│      Output (101, 64) 308            │
└─────────────────────────────────────┘
                 │
                 ▼
┌─────────────────────────────────────┐
│  Convolution Layer 2 (128, 5, 1) 310 │
└─────────────────────────────────────┘
                 │
                 ▼
┌─────────────────────────────────────┐
│   Batch Normalization & ReLU 312     │
└─────────────────────────────────────┘
                 │
                 ▼
┌─────────────────────────────────────┐
│      Output (101, 128) 314           │
└─────────────────────────────────────┘
                 │
                 ▼
┌─────────────────────────────────────┐
│          Droput 316                  │
└─────────────────────────────────────┘
                 │
                 ▼
┌─────────────────────────────────────┐
│  Flatten (101*128 = 12928) 318       │
└─────────────────────────────────────┘
                 │
                 ▼
┌─────────────────────────────────────┐
│  Fully-Connected Layer 1 (128) 320   │
└─────────────────────────────────────┘
                 │
                 ▼
┌─────────────────────────────────────┐
│   Batch Normalization & ReLU 322     │
└─────────────────────────────────────┘
                 │
                 ▼
┌─────────────────────────────────────┐
│          Droput 326                  │
└─────────────────────────────────────┘
                 │
                 ▼
┌─────────────────────────────────────┐
│  Fully-Connected Layer 2 (2) 328     │
└─────────────────────────────────────┘
                 │
                 ▼
┌─────────────────────────────────────┐
│    Batch Normalization 330           │
└─────────────────────────────────────┘
                 │
                 ▼
┌─────────────────────────────────────┐
│   Classification Layer (2) 332       │
└─────────────────────────────────────┘
                 │
                 ▼
┌─────────────────────────────────────┐
│   Classification Scores (2) 334      │
│ (true variant call, false variant   │
│              call)                   │
└─────────────────────────────────────┘
```

# FIG. 3

FIG. 4A

Variant Nucleotide is a False Variant

Chr11 70845504

P(X is False) = 0.974398

Prediction of the Variant Filter 111

Reference genome 498

**FIG. 4B**

Variant Nucleotide is a True Variant                                    _412_

Chr21    45731238

_410_

Father

Mother

_485_

NA12881                                _495_

_496_    P (X is True) = 0.304499

**FIG. 4C**    Prediction of the Variant Filter _111_

EP 3 619 712 B1

Nucleotide Sequence 514

Reference genome   ....ACCGTACCCTAGCCCTGACTAACGTACCGTACTAGCAATCG....

50 nucleotides    50 nucleotides

Variant Nucleotide
at a target position

50 nucleotides

Variant Nucleotide
at a target position

50 nucleotides

| N | T | G | C | A |
|---|---|---|---|---|
| 0 | 1 | 0 | 0 | 0 |
| 0 | 0 | 0 | 0 | 1 |
| 0 | 0 | 1 | 0 | 0 |
| 0 | 0 | 0 | 1 | 0 |
| 0 | 0 | 0 | 1 | 0 |
| 0 | 0 | 0 | 1 | 0 |
| 0 | 1 | 0 | 0 | 0 |
| 0 | 0 | 1 | 0 | 0 |
| 0 | 0 | 0 | 0 | 1 |
| 0 | 0 | 0 | 1 | 0 |
| 0 | 1 | 0 | 0 | 0 |
| 0 | 0 | 0 | 0 | 1 |
| 0 | 0 | 0 | 0 | 1 |
| 0 | 0 | 0 | 1 | 0 |
| 0 | 0 | 1 | 0 | 0 |

101 nucleotides

TAGCCCTGACTAACG

EP 3 619 712 B1

**FIG. 5**

FIG. 6

Repeat Pattern on Left

**FIG. 7A**

EP 3 619 712 B1

Repeat Pattern on Right

**FIG. 7B**

EP 3 619 712 B1

Repeat Pattern on Center

**FIG. 7C**

EP 3 619 712 B1

Repeat Pattern of Homopolymers
Repeat Factor = 15
Nucleotide base "C"
Nucleotide Sequence comprising
101 nucleotides

Classification Scores

Offset position

**FIG. 8A**

Repeat Pattern of Homopolymers
Repeat Factor = 15
Nucleotide base "G"
Nucleotide Sequence comprising
101 nucleotides

Classification Scores

Offset position

**FIG. 8B**

EP 3 619 712 B1

FIG. 8C

Repeat Pattern of Homopolymers
Repeat Factor = 15
Nucleotide base "T"
Nucleotide Sequence comprising
101 nucleotides

Classification Scores

1.0
0.8
0.6
0.4
0.2
0.0

0 2 4 6 8 10 12 14 16 18 20 22 24 26 28 30 32 34 36 38 40 42 44 46 48 50 52 54 56 58 60 62 64 66 68 70 72 74 76 78 80 82 84

Offset position

**FIG. 8D**

EP 3 619 712 B1

FIG. 9

**FIG. 10A**

**FIG. 10B**

**FIG. 10C**

**Computer System 1100**

Storage Subsystem **1110**

Memory Subsystem **1122**

RAM
**1132**

ROM
**1134**

File Storage
Subsystem
**1136**

Variant Filter <u>111</u>

User Interface
Input Devices
**1138**

Bus Subsystem <u>1155</u>

CPU
**1172**

Network Interface
Subsystem
**1174**

User Interface
Output Devices
**1176**

Deep Learning
Processors
(GPU, FPGA, CGRA)
**1178**

**FIG. 11**

EP 3 619 712 B1

Input Preparation Subsystem 161

Overlaid Samples
119

Pre-Trained Variant Filter 111

Classification Scores 334

Repeat Pattern Output Subsystem 1202

Distribution of
Classification Scores
1212

Sequence-Specific Error Correlation Subsystem 199

Above Threshold?
1222

SSE-Causing Repeat
Patterns 1232

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 1761554 W
- US 62447076
- US 62422841
- WO 07010252 A
- GB 2007003798 W
- US 20090088327 A
- US 20160085910
- US 20130296175
- WO 04018497 A
- US 7057026 B
- WO 9106678 A
- WO 07123744 A
- US 7329492 B
- US 7211414 B
- US 7315019 B
- US 7405281 B

- US 20080108082
- US 5641658 A
- US 20020055100
- US 7115400 B
- US 20040096853
- US 20040002090
- US 20070128624
- US 20080009420
- US 20070099208 A1
- US 20070166705 A1
- US 20080280773 A1
- US 018255
- WO 004018497 A
- US 2013030867 W
- WO 2014142831 A

**Non-patent literature cited in the description**

- **T SAUNDERS ; CHRISTOPHER ; WONG, WENDY ; SWAMY, SAJANI ; BECQ, JENNIFER ; J MURRAY ; LISA ; CHEETHAM, KEIRA.** Strelka: Accurate somatic small-variant calling from sequenced tumor-normal sample pairs. *Bioinformatics (Oxford, England),* 2012, vol. 28, 1811-7
- **A. VAN DEN OORD ; S. DIELEMAN ; H. ZEN ; K. SIMONYAN ; O. VINYALS ; A. GRAVES ; N. KALCHBRENNER ; A. SENIOR ; K. KAVUKCUOGLU.** WAVENET: A GENERATIVE MODEL FOR RAW AUDIO. *arXiv:1609.03499,* 2016
- **S. Ö. ARIK ; M. CHRZANOWSKI ; A. COATES ; G. DIAMOS ; A. GIBIANSKY ; Y. KANG ; X. LI ; J. MILLER ; A. NG ; J. RAIMAN.** DEEP VOICE: REAL-TIME NEURAL TEXT-TO-SPEECH. *arXiv:1702.07825,* 2017
- **F. YU ; V. KOLTUN.** MULTI-SCALE CONTEXT AGGREGATION BY DILATED CONVOLUTIONS. *arXiv:1511.07122,* 2016
- **K. HE ; X. ZHANG ; S. REN ; J. SUN.** DEEP RESIDUAL LEARNING FOR IMAGE RECOGNITION. *arXiv:1512.03385,* 2015
- **R.K. SRIVASTAVA ; K. GREFF ; J. SCHMIDHUBER.** HIGHWAY NETWORKS. *arXiv: 1505.00387,* 2015
- **G. HUANG ; Z. LIU ; L. VAN DER MAATEN ; K. Q. WEINBERGER.** DENSELY CONNECTED CONVOLUTIONAL NETWORKS. *arXiv:1608.06993,* 2017

- **C. SZEGEDY ; W. LIU ; Y. JIA ; P. SERMANET ; S. REED ; D. ANGUELOV ; D. ERHAN ; V. VANHOUCKE ; A. RABINOVICH.** GOING DEEPER WITH CONVOLUTIONS. *arXiv: 1409.4842,* 2014
- **S. IOFFE ; C. SZEGEDY.** BATCH NORMALIZATION: ACCELERATING DEEP NETWORK TRAINING BY REDUCING INTERNAL COVARIATE SHIFT. *arXiv: 1502.03167,* 2015
- **SRIVASTAVA, NITISH ; HINTON, GEOFFREY ; KRIZHEVSKY, ALEX ; SUTSKEVER, ILYA ; SALAKHUTDINOV, RUSLAN.** DROPOUT: A SIMPLE WAY TO PREVENT NEURAL NETWORKS FROM OVERFITTING. *The Journal of Machine Learning Research,* 2014, vol. 15 (1), 1929-1958
- **L. C. PIQUERAS.** AUTOREGRESSIVE MODEL BASED ON A DEEP CONVOLUTIONAL NEURAL NETWORK FOR AUDIO GENERATION. Tampere University of Technology, 2016
- **J. WU.** Introduction to Convolutional Neural Networks. Nanjing University, 2017
- CONVOLUTIONAL NETWORKS. **I. J. GOODFELLOW ; D. WARDE-FARLEY ; M. MIRZA ; A. COURVILLE ; Y. BENGIO.** Deep Learning. MIT Press, 2016

- **J. GU ; Z. WANG ; J. KUEN ; L. MA ; A. SHAHROUDY ; B. SHUAI ; T. LIU ; X. WANG ; G. WANG.** RECENT ADVANCES IN CONVOLUTIONAL NEURAL NETWORKS. *arXiv:1512.07108,* 2017
- **M. LIN ; Q. CHEN ; S. YAN.** Network in Network. *Proc. of ICLR,* 2014
- **L. SIFRE.** Rigid-motion Scattering for Image Classification. *Ph.D. thesis,* 2014
- **L. SIFRE ; S. MALLAT.** Rotation, Scaling and Deformation Invariant Scattering for Texture Discrimination. *Proc. of CVPR,* 2013
- **F. CHOLLET.** Xception: Deep Learning with Depthwise Separable Convolutions. *Proc. of CVPR,* 2017
- **X. ZHANG ; X. ZHOU ; M. LIN ; J. SUN.** ShuffleNet: An Extremely Efficient Convolutional Neural Network for Mobile Devices. *arXiv: 1707.01083,* 2017
- **K. HE ; X. ZHANG ; S. REN ; J. SUN.** Deep Residual Learning for Image Recognition. *Proc. of CVPR,* 2016
- **S. XIE ; R. GIRSHICK ; P. DOLLÁR ; Z. TU ; K. HE.** Aggregated Residual Transformations for Deep Neural Networks. *Proc. of CVPR,* 2017
- **A. G. HOWARD ; M. ZHU ; B. CHEN ; D. KALENICHENKO ; W. WANG ; T. WEYAND ; M. ANDREETTO ; H. ADAM.** Mobilenets: Efficient Convolutional Neural Networks for Mobile Vision Applications. *arXiv:1704.04861,* 2017
- **M. SANDLER ; A. HOWARD ; M. ZHU ; A. ZHMOGINOV ; L. CHEN.** MobileNetV2: Inverted Residuals and Linear Bottlenecks. *arxiv:1801.04381v3,* 2018
- **Z. QIN ; Z. ZHANG ; X. CHEN ; Y. PENG.** FD-MobileNet: Improved MobileNet with a Fast Downsampling Strategy. *arXiv: 1802.03750,* 2018
- Convolutional Neural Networks for Regulatory Genomics. **N. TEN DIJKE.** Master's Thesis. Universiteit Leiden Opleiding Informatica, 17 June 2017
- **BRENDAN D. O'FALLON et al.** A support vector machine for identification of single-nucleotide polymorphisms from next-generation sequencing data. *Bioinformatics,* 24 April 2013
- **BENTLEY et al.** *Nature,* 2008, vol. 456, 53-59
- **LIZARDI et al.** *Nat. Genet.,* 1998, vol. 19, 225-232
- **DUNN, TAMSEN ; BERRY, GWENN ; EMIG-AGIUS, DOROTHEA ; JIANG, YU ; IYER, ANITA ; UDAR, NITIN ; STRÖMBERG, MICHAEL.** *Pisces: An Accurate and Versatile Single Sample Somatic and Germline Variant Caller,* 2017, 595-595
- **T SAUNDERS ; CHRISTOPHER ; WONG, WENDY ; SWAMY, SAJANI ; BECQ, JENNIFER ; J MURRAY ; LISA ; CHEETHAM, KEIRA.** Strelka: Accurate somatic small-variant calling from sequenced tumor-normal sample pairs. *Bioinformatics (Oxford, England),* 2012, vol. 28, 1811-7
- **STROMBERG, MICHAEL ; ROY, RAJAT ; LAJUGIE, JULIEN ; JIANG, YU ; LI, HAOCHEN ; MARGULIES, ELLIOTT.** *Nirvana: Clinical Grade Variant Annotator,* 2017, 596-596
- **J. WU.** INTRODUCTION TO CONVOLUTIONAL NEURAL NETWORKS. Nanjing University, 2017